# EUROPEAN PATENT SPECIFICATION

(11) **EP 0 945 464 B1**
(45) Date of publication and mention of the grant of the patent: **07.02.2001**
(21) Application number: 99102166.8
(22) Date of filing: 07.10.1996
(51) Int. Cl.: C07K 16/22

(54) **Specific binding members for human transforming growth factor beta; materials and methods**
Spezifische Bindungspartner für menschliches TGF-beta, Materialien und Verfahren
Membres de liaison spécifiques contre TGF-beta humaine, matériaux et méthodes

(30) Priority: 06.10.1995 GB 9520486; 19.01.1996 GB 9601081
(43) Date of publication of application: 29.09.1999
(62) Divisional of application: 96932730.3
(73) Proprietor: Cambridge Antibody Technology Limited, Royston, Cambridgeshire SG8 6JJ (GB)
(72) Inventor: Thompson, Julia Elizabeth, Melbourne, Royston Herts SG8 6UH (GB); Williams, Andrew James, Forest Gate, London E7 8DA (GB); Vaughan, Tristan John, Impington, Cambridge CB4 4NZ (GB); Green, Jonathan Alexander, Linton, Cambridgeshire CB1 6LD (GB); Johnson, Kevin Stuart, Caldecote Highfields, Cambs CB3 7NY (GB); Tempest, Philip Ronald, West Wratting, Cambridge CB1 5CU (GB); Wilton, Alison Jane, Cambridge CB3 0HH (GB)
(74) Representative: Walton, Seán Malcolm

(56) References cited:
- WO-A-91/04748
- WO-A-92/17206
- WO-A-92/22653
- LUCAS C. ET AL.,: "The autocrine production of transforming growth factor-beta1 during lymphocyte activation" J. IMMUNOLOGY, vol. 145, - 1 September 1990 pages 1415-1422, XP002025314
- DASCH J.R. ET AL.,: 'Monoclonal antibodies recognizing transforming growth factor- beta' J. IMMUNOLOGY vol. 142, no. 5, 05 March 1989, pages 1536 - 1541, XP002025315
- SHAH M. ET AL.,: "Neutralisation of TGF-beta1 and TGF-beta2 or exogenous addition of TGF-beta3 to cutaneous rat wounds reduces scarring" J. CELL SCIENCE, - March 1995 pages 985-1002, XP002025316
- TEMPEST P R ET AL: "Human antibodies specific for human TGF-beta derived from phage display libraries" IMMUNOTECHNOLOGY, vol. 2, no. 4, 1 November 1996, page 306 XP004063262

## Description

This invention relates co specific binding members for human transforming growth factor beta (TGFβ) and materials and methods relating thereto. In particular, it relates to specific binding members comprising human antibody binding domains. Human antibodies against human TGFβ may be isolated and utilised in the treatment of disease, particularly fibrotic disease and also immune/inflammatory diseases. The isolation of antiself antibodies from antibody segment repertoires displayed on phage has been described (A.D.Griffiths et al. EMBO J. 12, 725-734, 1993; A. Nissim et al. EMBO J. 13, 692-698, 1994; A.D. Griffiths et al. 13, 3245-3260, 1994; C.Barbas et al. Proc. Natl. Acad. Sci. USA 90, 10003-10007 1993; WO93/11236). However, the present invention provides specific antibodies against a particular isoforms of TGFβ, which antibodies have unexpected and advantageous properties.

TGFβ is a cytokine known to be involved in many cellular processes such as cell proliferation and differentiation, embryonic development, extracellular matrix formation, bone development, wound healing, hematopoiesis and immune and inflammatory responses(A.B. Roberts & M. Sporn 1990 pp419-472 in Handbook of Experimental Pharmacology eds M.B. Sporn & A.B. Roberts, Springer Heidelberg; J.Massague et al.Annual Rev. Cell Biol. 6, 597-646, 1990).

The accumulation of excessive extra-cellular matrix is associated with various fibrotic diseases. Thus there is a need to control agents such as TGFβ1 and TGFβ2 to prevent their deleterious effects in such diseases and this is one application of human antibodies to human TGFβ.

The modulation of immune and inflammatory responses by TGFbetas includes (i) inhibition of proliferation of all T-cell subsets (ii) inhibitory effects on proliferation and function of B lymphocytes (iii) down-regulation of natural-killer cell activity and the T-cell response (iv) regulation of cytokine production by immune cells (v) regulation of macrophage function and (vi) leucocyte recruitment and activation.

A further application of antibodies to TGFβ may be in the treatment of immune/inflammatory diseases such as rheumatoid arthritis, where these functions need to be controlled.

It is a demanding task to isolate an antibody fragment specific for TGFβ of the same species. Animals do not normally produce antibodies to self antigens, a phenomenon called tolerance (G.J. Nossal Science 245, 147-153, 1989). In general, vaccination with a self antigen does not result in production of circulating antibodies. It is therefore difficult to raise human antibodies to human self antigens. There are also in addition, ethical problems in vaccinating humans. In relation to the raising of non-human antibodies specific for TGFβ, there are a number of problems. TGFβ is an immunosuppressive molecule and further, there is strong conservation of sequence between human and mouse TGFβ molecules. Mouse and human TGFβ1 only differ by one amino acid residue, an alanine (human) to serine (mouse) change at a buried residue (R.Derynck et al. J.Biol. Chem. 261, 4377-4379, 1986). Mouse and human TGFβ2 only differ at three residues; residue 59 (T mouse, S human); residue 60 (K mouse, R human) and residue 94 (N mouse; K human). This makes it difficult to raise antibodies in mice against human TGFβ. Further, any antibodies raised may only be directed against a restricted set of epitopes.

Polyclonal antibodies binding to human TGFβ1 and human TGFβ2 against both neutralising and non-neutralising epitopes have been raised in rabbit (Danielpour et al. Growth Factors 2 61-71, 1989; A. Roberts et al. Growth Factors 3, 277-286, 1990), chicken (R&D Systems, Minneapolis) and turkey (Danielpour et al. J. Cell Physiol. 138, 79-86, 1989). Peptides representing partial TGFβ sequences have also been used as immunogens to raise neutralising polyclonal antisera in rabbits (W.A Border et al. Nature 346, 371-374, 1990; K.C. Flanders Biochemistry 27, 739-746, 1988; K.C. Flanders *et al*, Growth Factors 3, 45-52, 1990). In addition there have been limited reports of isolation of mouse monoclonals against TGFβ. Following immunisation with bovine TGFβ2 (identical to human TGFβ2), three non-neutralising monoclonal antibodies were isolated that are specific for TGFβ2 and one neutralising antibody that is specific for TGFβ1 and TGFβ2 (J.R. Dasch et al. J. Immunol. 142, 1536-1541, 1989). In another report, following immunisation with human TGFβ1, neutralising antibodies were isolated which were either specific for TGFβ1 or cross-reacted with TGFβ1, TGFβ2 and TGFβ3 (C. Lucas et al. J.Immunol. 145, 1415-1422, 1990). A neutralising mouse monoclonal antibody which binds both TGFβ2 and TGFβ3 isoforms is available commercially from Genzyme Diagnostics.

The present text discloses the first isolation of human antibodies directed against human TGFβ1 and against human TGFβ2. A mouse monoclonal antibody directed against human TGFβ1 is available from R&D Systems. This antibody only weakly neutralises TGFβ1 in a neutralisation assay. Neutralising mouse monoclonal antibodies have also been generated from mice immunised with human TGFβ1 peptides comprising amino acid positions 48 to 60 (antibody reactive with TGFβ1, TGfβ2 and TGFβ3) and amino acid positions 86-101 (antibody specific for TGFβ1; M. Hoefer & F.A. Anderer Cancer Immunol. Immunother. 41, 302-308, 1995).

Phage antibody technology (WO92/01047; PCT/GB92/00883; PCT/GB92/01755; WO93/11236) offers the ability to isolate directly human antibodies against human TGFβ. In application WO93/11236 the isolation of antiself antibodies from phage display libraries was disclosed and it was suggested that antibodies specific for TGFβ could be isolated from phage display libraries.

The present application shows that antibodies of differing specificities for TGFβ molecules may be isolated. TGFβ1, TGFβ2 and TGFβ3 are a closely related group of cytokines. They are dimers consisting of two 112 amino acid monomers joined by an interchain disulphide bridge. TGFβ1 differs from TGFβ2 by 27 mainly conservative changes and from TGFβ3 by 22 mainly conservative changes. These differences have been related to the 3D structure (M.Schlunegger & M.G.Grutter Nature 358, 430-434, 1992). The present applicants have isolated antibodies which are essentially specific for TGFβ1 (very low cross-reactivity with TGFβ2); antibodies which are essentially specific for TGFβ2 (very low cross-reactivity TGFβ1); and antibodies which bind both TGFβ1 and TGFβ2. Hence, these three different types of antibodies, each type with distinctive binding specificities must recognise different epitopes on the TGFβ molecules. These antibodies have low cross-reactivity with TGFβ3 as assessed by binding studies using biosensor assays (e.g.BIACore™), ELISA and radioreceptor assays. The most extensively studied antibody, 6B1 IgG4, shows 9% cross-reactivity with TGFβ3 as compared with TGFβ2, as determined by their relative dissociation constants, determined using a biosensor.

TGFβ isoforms are initially exported from cells as inactive, latent forms (R. Pircher *et al*, Biochem. Biophys. Res. Commun. 136, 30-37, 1986; L.M. Wakefield *et al., Growth Factors* 1*,* 203-218, 1989). These inactive forms are activated by proteases in plasma to generate the active form of TGFβ. It is this active form of TGFβ2 which binds to receptors promoting the deposition of extracellular matrix and the other biological effects of TGFβ. The active form of TGFβ represents a relatively low proportion of TGFβ that is in the plasma. Therefore, for a neutralising antibody against TGFβ to be most effective at preventing fibrosis the antibody should recognise the active but not the latent form. In Example 6, it is demonstrated that an antibody ("6B1 IgG4") recognises the active but not the latent form of TGFβ2.

The epitope of 6B1 IgG4 has been identified using a combination of peptide display libraries and inhibition studies using peptides from the region of TGFβ2 identified from phage selected from the peptide phage display library. This is described in Examples 11 and 14. The sequence identified from the peptide library is RVLSL and represents amino acids 60 to 64 of TGFβ2 (Example 11). The antibody 6B1 IgG4 has also been shown to bind to a peptide corresponding to amino acids 56 to 69 of TGFβ2 (TQHSRVLSLYNTIN) with a three amino acid (CGG) extension at the N-terminus. RVLSL is the minimum epitope, 6B1 IgG4 is likely to bind to further adjacent amino acids. Indeed, if the epitope is three dimensional there may be other non-contiguous sequences to which the antibody will bind. 6B1 IgG4 shows much weaker binding to the peptide corresponding to amino acids 56 to 69 of TGFβ1 (CGG-TQYSKVLSLYNQHN).

The results of Example 14 support the assignment of the epitope of 6B1 IgG4 on TGFβ2 to the aminoacids in the region of residues 60 to 64. The peptide used in this example, residues 56 to 69, corresponds to the amino acids of alpha helix H3 (M.P. Schlunegger & M.G. Grutter Nature 358 430-434, 1992; also known as the a3 helix (S. Daopin et al Proteins: Structure, Function and Genetics 17 176-192, 1993). TGFβ2 forms a head-to-tail dimer with the alpha helix H3 (also referred to as the heel) of one subunit forming an interface with finger regions (including residues 24 to 37 and residues in the region of amino acids 91 to 95; also referred to as fingers 1 and 2) from the other subunit (S. Daopin et al supra). It has been proposed that the primary structural features which interact with the TGFβ2 receptor consist of amino acids at the C-terminal end of the alpha helix H3 from one chain together with residues of fingers 1 and 2 of the other chain (D.L. Griffith et al Proc. Natl. Acad. Sci. USA 93 878-883,, 1996). The identification of an epitope for 6B1 IgG4 within the alpha helix H3 of TGFβ2 is consistent with 6B1 IgG4 preventing receptor binding and neutralising the biological activity of TGFβ2.

As noted above if the epitope for 6B1 IgG4 is three dimensional there may be other non-contiguous amino acids to which the antibody may bind.

There is earlier advice that antibodies directed against this region of TGFβ2 may be specific for TGFβ2 and neutralise its activity. Flanders et al (Development 113 183-191, 1991) showed that polyclonal antisera could be raised in rabbits against residues 50 to 75 of mature TGFβ2 and that these antibodies recognised TGFβ2 but the TGFβ1 in Western blots. In an earlier paper, K.C. Flanders et al (Biochemistry 27 739-746, 1988) showed that polyclonal antisera raised in rabbits against amino acids 50 to 75 of TGFβ1 could neutralise the biological activity of TGFβ1. The antibody isolated in this application 6B1 IgG4 is a human antibody directed against the amino acids in this region which neutralises the biological activity of human TGFβ2. It is surprising that such a neutralising antibody against TGFβ2 can be isolated in humans (where immunisation with a peptide cannot be used for ethical reasons) directly from a phage display antibody repertoire.

The knowledge that the residues of the alpha helix H3 form a neutralising epitope for TGFβ2 means that phage displaying neutralising antibodies are obtainable by selection from phage antibody repertoires by binding to a peptide from this region coupled to a carrier protein such as bovine serum albumin or keyhole limpet haemocyanin. This approach may be applied to select antibodies which are capable of neutralising the biological activity of TGFβ1 by selecting on the peptide TQYSKVLSLYNQHN coupled to a carrier protein. It is possible that such an approach may be extended to peptides from receptor binding regions of TGFβ isoforms, other than the H3 alpha helix.

It has further been demonstrated by the present inventors that antibodies specific for TGFβ are obtainable by isolation from libraries derived from different sources of immunoglobulin genes: from repertoires of natural immunoglobulin variable domains, e.g. from immunised or non-immunised hosts; and synthetic repertoires derived from germline V genes combined with synthetic CDR3s. The properties of these antibodies in single chain Fv and whole IgG4 format are described.

As noted above WO93/11236 suggested that human antibodies directed against human TGFβ could be isolated from phage display libraries. Herein it is shown that the phage display libraries from which antiself antibodies were isolated in WO93/11236 may be utilised as a source of human antibodies specific for particular human TGFβ isoforms. For instance, in example 1 of the present application, the antibody 1A-E5 specific for TGFβ1 and the antibodies 2A-H11 and 2A-A9 specific for TGFβ2 were isolated from the "synthetic library" described in examples 5 to 7 of WO93/11236 and in Nissim et al. (1994; supra). Also, the phage display library derived from peripheral blood lymphocytes (PBLs) of an unimmunised human (examples 1 to 3 of WO93/11236) was the source for the antibody 1B2 specific for TGFβ1. Phage display libraries made subsequently utilising antibody genes derived from human tonsils and bone marrow, have also provided sources of antibodies specific for human TGFβ. Thus human TGFβ is an example of a human self antigen to which antibodies may be isolated from "large universal libraries". Human antibodies against human TGFβ with improved properties can be obtained by chain shuffling for instance combining the VH domains of antibodies derived from one library with the VL domains of another library thus expanding the pool of VL partners tested for each VH domain. For instance, the antibodies 6B1, 6A5 and 6H1 specific for TGFβ2 utilise the 2A-H11 VH domain isolated from the "synthetic library" combined with a light chain from the PBL library.

Thus the VH and VL domains of antibodies specific for TGFβ can be contributed from phage display libraries derived from rearranged V genes such as those in PBLs, tonsil and bone marrow and from V domains derived from cloned germline V segments combined with synthetic CDRs. There are also shown to be a diverse range of antibodies which are specific for TGFβ1 or TGFβ2. The antibodies which have been isolated both against TGFβ1 and TGFβ2 have mainly utilised V genes derived from VH germlines of the VH3 family. A wider variety of light chain variable regions have been used, of both the lambda and kappa types.

Individual antibodies which have been isolated have unexpectedly advantageous properties. For example, the antibodies directed against TGFβ2 (6H1, 6A5 and 6B1) have been shown to bind to TGFβ2 with slow off-rates (off-rate constants k_{off} of the order of 10⁻³ s⁻¹ and dissociation constants of less than 10⁻⁸M) to neutralise TGFβ2 activity in in vitro assays and to be potent in in vivo applications. The antibody 6B1 IgG4 has been shown to bind specifically to TGFβ2 in immunohistochemistry in mammalian tissues and not to cross-react with other antigens in human tissues. The properties of these antibodies may make them particularly suitable for therapeutic applications. The fact that these antibodies share the same heavy chain, shows that VH domains can be effective with a number of different light chains, although there may be differences in potency or subtle changes of epitope with different light chains. As shown in Examples 3 and 4 and Tables 4 and 5, 6B1 IgG4 is the most potent antibody in neutralising TGFβ2 activity in the radioreceptor assay and the TF1 proliferation assay. Its properties may however be expected to be qualitatively similar to the antibodies 6A5 and 6H1 with which it shares a common VH domain. Thus the reduction in neural scarring observed on treatment with 6A5 single chain Fv and 6H1 IgG4 shown in Example 5 would be expected to be reproduced with 6B1. The antibodies directed against TGFβ1 (particularly 1B2 and its derivatives) also have unexpectedly advantageous properties. Antibody 27C1/10A6 derived from 1B2 by chain shuffling, spiking and conversion into whole antibody IgG4 , has been shown to be potent in an *in vitro* scarring model. The VH domain of this antibody was derived by site directed "spiking" mutagenesis from the parent antibody 7A3. A large number of spiked clones were obtained which show similar properties in in vitro assays. There can be a number of changes in CDR3 of the VH compared to 27C1, for instance, 28A-H11 differs in 7 of the 14 positions, 2 of which are non-conservative changes. Thus there may be up to 50% of the residues in the VH CDR3 changed without affecting binding properties.

Antibodies specific for human TGFβ1 and human TGFβ2 have been shown to be effective in animal models for the treatment of fibrotic diseases and other diseases such as rheumatoid arthritis where TGFβ is overexpressed. Antibodies against TGFβ have been shown to be effective in the treatment of glomerulonephritis (W.A Border et al. Nature 346, 371-374, 1990); neural scarring (A. Logan et al. Eur. J. Neurosci. 6, 355-363, 1994); dermal scarring (M. Shah et al. Lancet 339, 213-214 1992; M.Shah et al. J.Cell Science 107, 1137-1157, 1994; M. Shah et al. 108, 985-1002, 1995); lung fibrosis (S.N. Giri et al. Thorax 48, 959-966, 1993); arterial injury (Y.G. Wolf, L.M. Rasmussen & E. Ruoslahti J. Clin. Invest. 93, 1172-1178, 1994) and rheumatoid arthritis (Wahl et al J. Exp. Medicine 177, 225-230, 1993). It has been suggested that TGFβ3 acts antagonistically to TGFβ1 and TGFβ2 in dermal scarring (M.Shah et al. 1995 supra.). Therefore, antibodies to TGFβ1 or TGFβ2 with apparent low cross-reactivity to TGFβ3, as assessed by binding studies using a biosensor assay (e.g BIACore™), ELISA or a radioreceptor assay, as disclosed in this application, that is to say antibodies which bind preferentially to TGFβ1 or TGFβ2 compared with TGFβ3, should be advantageous in this and other conditions such as fibrotic conditions in which it is desirable to counteract the fibrosis promoting effects of TGFβ1 and TGFβ2. An antibody which cross-reacts strongly with TGFβ3 has however had an effect in an animal model of rheumatoid arthritis (Wahl *et al.*, 1993, *supra).*

There are likely to be applications further to the above mentioned conditions, as there are several other *in vitro* models of disease where antibodies against TGFβ have shown promise of therapeutic efficacy. Of particular importance may be the use of antibodies against TGFβ for the treatment of eye diseases involving ocular fibrosis, including proliferative retinopathy (R.A. Pena *et al.* (ref. below) , retinal detachment and post glaucoma (P.T. Khaw *et al., Eye* **8** 188-195, 1994) drainage surgery. Connor *et al. (J. Clin. Invest* **83** 1661-1666, 1989) showed that much higher levels of TGFβ2 were present in vitreous aspirates from patients with intraocular fibrosis associated with proliferative retinopathy compared with patients with uncomplicated retinal detachment without ocular firbrosis and that the biological activity of this TGFβ2 could be neutralised with antibodies directed against TGFβ2. Moreover, Pena *et al. (Invest. Ophthalmology. Vis. Sci.* **35**: 2804-2808, 1994) showed that antibodies against TGFβ2 inhibit collagen contraction stimulated by TGFβ2. Contraction of the vitreous gel by fibroblasts and other cell types plays a critical role in the proliferative retinopathy disease process, a process thought to be mediated by TGFβ2.

There is other evidence pointing to TGFβ2 being the most important TGFβ isoform promoting intraocular fibrosis. TGFβ2 has been shown to be the predominant isoform of TGFβ in the neural retina, retinal pigment epithelium-choroid and vitreous of the human eye (Pfeffer *et al. Exp. Eye Res.* **59**: 323-333, 1994) and found in human aqueous humour in specimens from eyes undergoing cataract extraction with intraocular lens implantation (Jampel *et al. Current Eye Research* **9**: 963-969, 1990). Non-transformed human retinal pigment epithelial cells predominantly secrete TGFβ2 (Kvanta *Opthalmic Res.* **26**: 361-367, 1994).

Other diseases which have potential for treatment with antibodies against TGFβ include adult respiratory distress syndrome, cirrhosis of the liver, post myocardial infarction, post angioplasty restenosis, keloid scars and scleroderma. The increased level of expression of TGFβ2 in osteoporosis (Erlenbacher *et al. J. Cell Biol.* **132**: 195-210, 1996) means that this is a disease potentially treatable by antibodies directed against TGFβ2.

The use of antibodies against TGFβ for the treatment of diseases has been the subject of patent applications for fibrotic disease (WO91/04748); dermal scarring (WO92/17206); macrophage deficiency diseases (PCT/US93/00998); macrophage pathogen infections (PCT/US93/02017); neural scarring (PCT/US93/03068); vascular disorders (PCT/US93/03795); prevention of cataract (WO95/13827). The human antibodies against human TGFβ disclosed in this application should be valuable in these conditions.

It is shown herein that the human antibodies both against human TGFβ1 and against human TGFβ2 can be effective in the treatment of fibrosis in animal models of neural scarring and glomerulonephritis in either single chain Fv and whole antibody format. This is the first disclosure of the effectiveness of antibodies directed only against TGFβ2 as sole treatment in these indications, although some effectiveness of antibodies against TGFβ2 only has been observed in a lung fibrosis model (Giri et al. Thorax 48, 959-966, 1993 supra). The effectiveness of the human antibodies against human TGFβ in treatment of fibrotic disease has been determined by measuring a decrease in the accumulation of components of the extracellular matrix, including fibronectin and laminin in animal models.

The evidence of efficacy of the antibodies against TGFβ2 and TGFβ1 describe herein in prevention of neural scarring in the animal model experiment means that these antibodies are likely to be effective in other disease states mediated by TGFβ. For comparison, antisera isolated from turkeys directed against TGFβ isoforms by Danielpour *et al. (Cell Physiol.* **138**: 79-86, 1989) have been shown to be effective in the prevention of dermal scarring (Shah *et al. J. Cell Science* **108**: 985-1002, 1995), neural scarring (Logan *et al., supra)* and in *in vitro* experiments relating to proliferative retinopathy (Connor *et al., supra) .*

### TERMINOLOGY

### Specific binding member

This describes a member of a pair of molecules which have binding specificity for one another. The members of a specific binding pair may be naturally derived or wholly or partially synthetically produced. One member of the pair of molecules has an area on its surface, or a cavity, which specifically binds to and is therefore complementary to a particular spatial and polar organisation of the other member of the pair of molecules. Thus the members of the pair have the property of binding specifically to each other. Examples of types of specific binding pairs are antigen-antibody, biotin-avidin, hormone-hormone receptor, receptor-ligand, enzyme-substrate. This application is concerned with antigen-antibody type reactions.

### Antibody

This describes an immunoglobulin whether natural or partly or wholly synthetically produced. The term also covers any polypeptide or protein having a binding domain which is, or is homologous to, an antibody binding domain. These can be derived from natural sources, or they may be partly or wholly synthetically produced. Examples of antibodies are the immunoglobulin isotypes and their isotypic subclasses; fragments which comprise an antigen binding domain such as Fab, scFv, Fv, dAb, Fd; and diabodies.

It is possible to take monoclonal and other antibodies and use techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP-A-184187, GB 2188638A or EP-A-239400. A hybridoma or other cell producing an antibody may be subject to genetic mutation or other changes, which may or may not alter the binding specificity of antibodies produced.

As antibodies can be modified in a number of ways, the term "antibody" should be construed as covering any specific binding member or substance having a binding domain with the required specificity. Thus, this term covers antibody fragments, derivatives, functional equivalents and homologues of antibodies, including any polypeptide comprising an immunoglobulin binding domain, whether natural or wholly or partially synthetic. Chimeric molecules comprising an immunoglobulin binding domain, or equivalent, fused to another polypeptide are therefore included. Cloning and expression of chimeric antibodies are described in EP-A-0120694 and EP-A-0125023.

It has been shown that fragments of a whole antibody can perform the function of binding antigens. Examples of binding fragments are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment (Ward, E.S. et al., Nature 341, 544-546 (1989)) which consists of a VH domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al, Science, **242**, 423-426, 1988; Huston et al, PNAS USA, 85, 5879-5883, 1988); (viii) bispecific single chain Fv dimers (PCT/US92/09965) and (ix) "diabodies", multivalent or multispecific fragments constructed by gene fusion (WO94/13804; P. Holliger et al Proc. Natl. Acad. Sci. USA **90** 6444-6448, 1993).

Diabodies are multimers of polypeptides, each polypeptide comprising a first domain comprising a binding region of an immunoglobulin light chain and a second domain comprising a binding region of an immunoglobulin heavy chain, the two domains being linked (e.g. by a peptide linker) but unable to associate with each other to form an antigen binding site: antigen binding sites are formed by the association of the first domain of one polypeptide within the multimer with the second domain of another polypeptide within the multimer (WO94/13804).

Where bispecific antibodies are to be used, these may be conventional bispecific antibodies, which can be manufactured in a variety of ways (Holliger, P. and Winter G. Current Opinion Biotechnol. 4, 446-449 (1993)), eg prepared chemically or from hybrid hybridomas, or may be any of the bispecific antibody fragments mentioned above. It may be preferable to use scFv dimers or diabodies rather than whole antibodies. Diabodies and scFv can be constructed without an Fc region, using only variable domains, potentially reducing the effects of anti-idiotypic reaction. Other forms of bispecific antibodies include the single chain "Janusins" described in Traunecker et al, Embo Journal, 10, 3655-3659, (1991).

Bispecific diabodies, as opposed to bispecific whole antibodies, may also be particularly useful because they can be readily constructed and expressed in *E.coli.* Diabodies (and many other polypeptides such as antibody fragments) of appropriate binding specificities can be readily selected using phage display (WO94/13804) from libraries. If one arm of the diabody is to be kept constant, for instance, with a specificity directed against antigen X, then a library can be made where the other arm is varied and an antibody of appropriate specificity selected.

### Antigen binding domain

This describes the part of an antibody which comprises the area which specifically binds to and is complementary to part or all of an antigen. Where an antigen is large, an antibody may only bind to a particular part of the antigen, which part is termed an epitope. An antigen binding domain may be provided by one or more antibody variable domains. Preferably, an antigen binding domain comprises an antibody light chain variable region (VL) and an antibody heavy chain variable region (VH).

### Specific

This may be used to refer to the situation in which one member of a specific binding pair will not show any significant binding to molecules other than its specific binding partner(s). The term is also applicable where e.g. an antigen binding domain is specific for a particular epitope which is carried by a number of antigens, in which case the specific binding member carrying the antigen binding domain will be able to bind to the various antigens carrying the epitope.

### Neutralisation

This refers to the situation in which the binding of a molecule to another molecule results in the abrogation or inhibition of the biological effector function of the another molecule.

A variant, derivative or mutant of an antibody may be obtained by modification of the parent molecule by the addition, deletion, substitution or insertion of one or more amino acids, or by the linkage of another molecule. These changes may be made at the nucleotide or protein level. For example, the encoded polypeptide may be a Fab fragment which is then linked to an Fc tail from another source. Alternatively, a marker such as an enzyme, flourescein, etc, may be linked.

### Comprise

This is generally used in the sense of include, that is to say permitting the presence of one or more features or components.

The present invention generally provides a specific binding member comprising a human antibody antigen binding domain. More particularly it provides a specific binding member for human TGFβ1.

The present invention provides a specific binding member which comprises a human antibody antigen binding domain specific for TGFβ1 and which has low cross reactivity with TGFβ3. The cross-reactivity may be as assessed using any or all of the following assays: biosensor (e.g. BIACore™), ELISA and radioreceptor. The present invention provides specific binding member which comprises a human antibody antigen binding domain specific for human TGFβ1 which binds preferentially to these isoforms compared with TGFβ3.

The specific binding member may be in the form of an antibody fragment such as single chain Fv (scFv). Other types of antibody fragments may also be utilised such as Fab, Fab', F(ab')₂ , Fabc, Facb or a diabody (G.Winter & C.Milstein Nature 349, 293-299, 1991; WO94/13804). The specific binding member may be in the form of a whole antibody. The whole antibody may be in any of the forms of the antibody isotypes eg IgG, IgA, IgE, and IgM and any of the forms of the isotype subclasses eg IgG1 or IgG4.

The specific binding member may also be in the form of an engineered antibody eg bispecific antibody molecules (or fragments such as F(ab')₂) which have one antigen binding arm (ie specific binding domain) against TGFβ and another arm against a different specificity. Indeed the specific binding members directed against TGFβ1 and/or TGFβ2 described herein may be combined in a bispecific diabody format. For example the antibodies 31G9 directed against TGFβ1 and 6H1 directed against TGFβ2 may be combined to give a single dimeric molecule with both specificities.

The binding domain may comprise part or all of a VH domain encoded by a germ line gene segment or a re-arranged gene segment. The binding domain may comprise part or all of either a VL kappa domain or a VL lambda domain.

The binding domain may be encoded by an altered or variant form of a germ line gene with one or more nucleotide alterations (addition, deletion, substitution and/or insertion), e.g. about or less than about 25, 20, 15, 10 or 5 alterations, 4, 3, 2 or 1, which may be in one or more frameworks and/or CDR's.

The binding domain may comprise a VH3 gene sequence of one of the following germ lines; the DP49 germ line; the DP53 germ line; the DP50 germ line; the DP46 germ line; or a re-arranged form thereof.

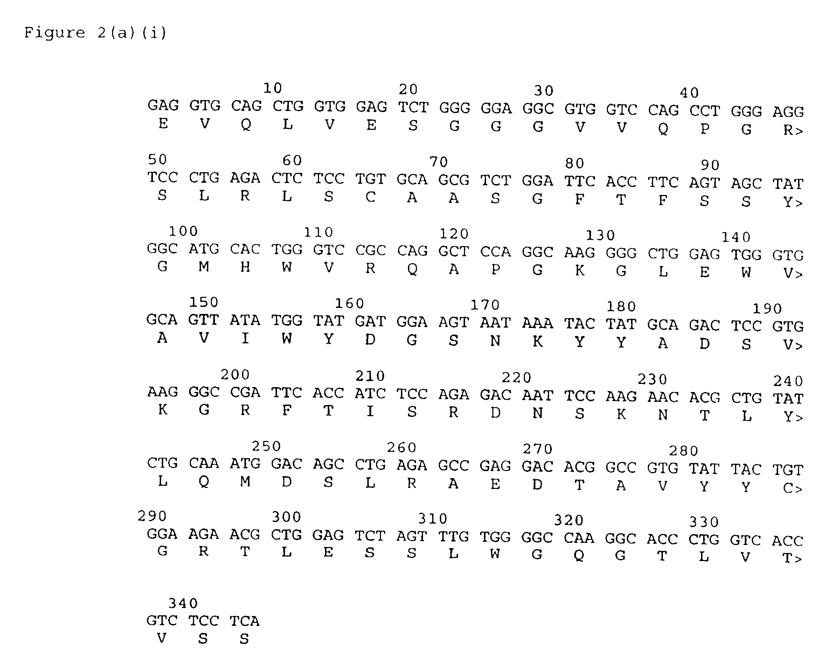

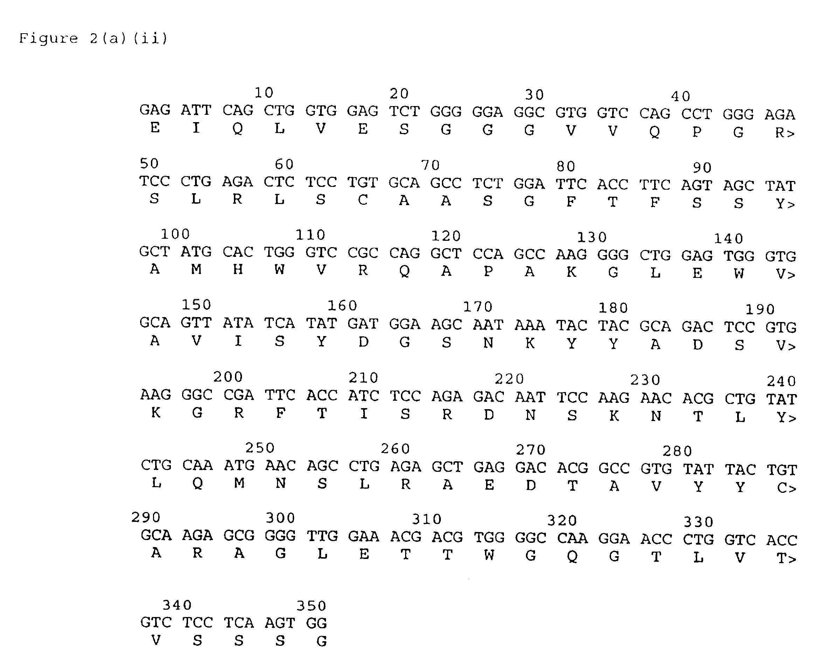

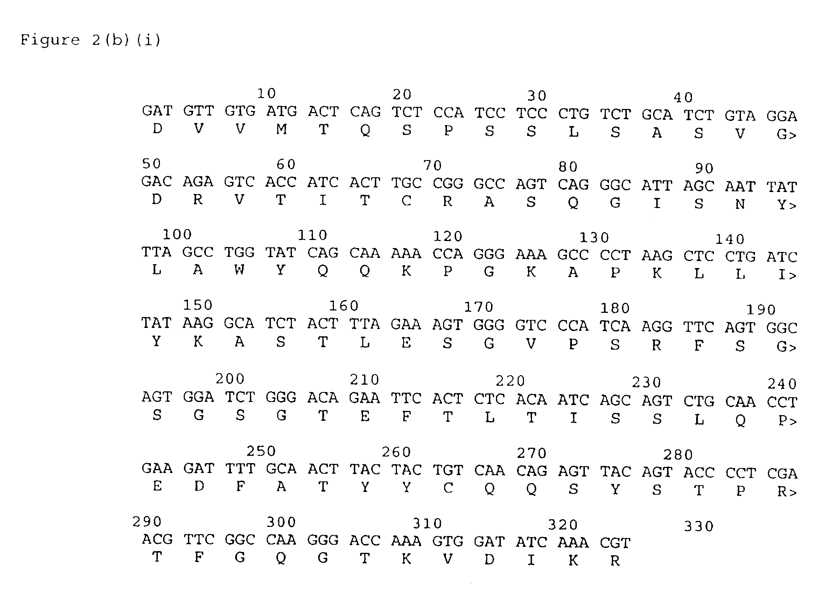

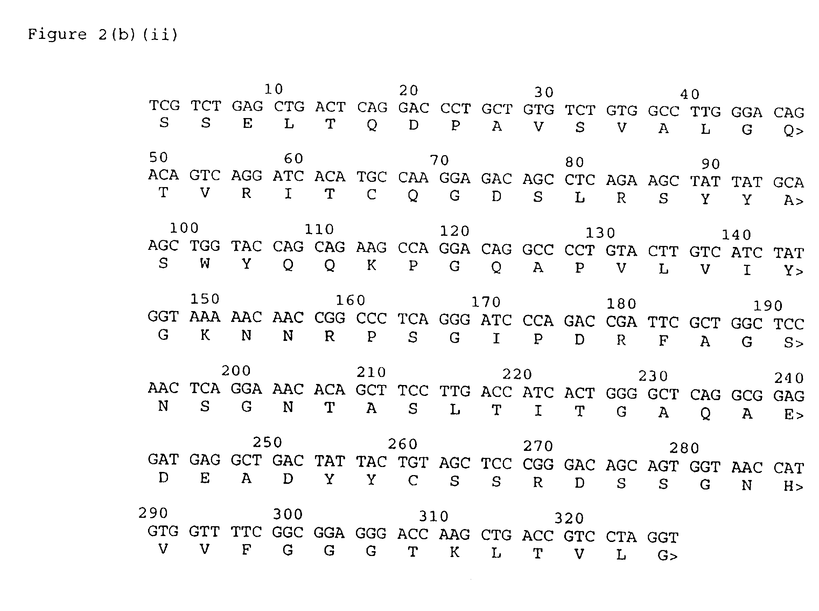

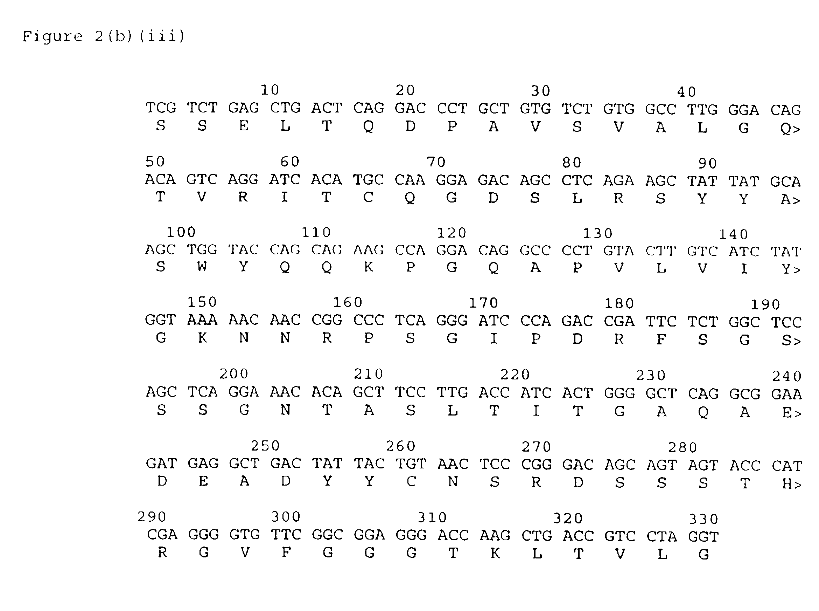

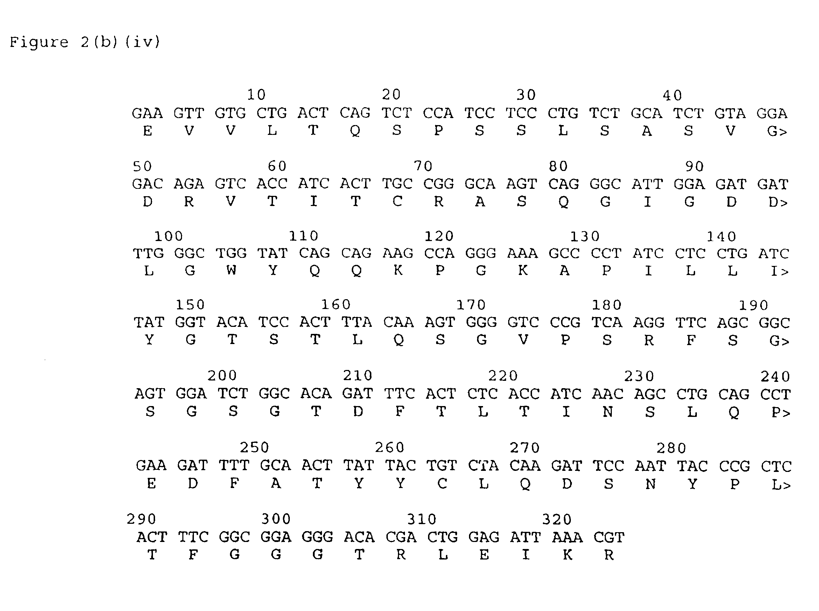

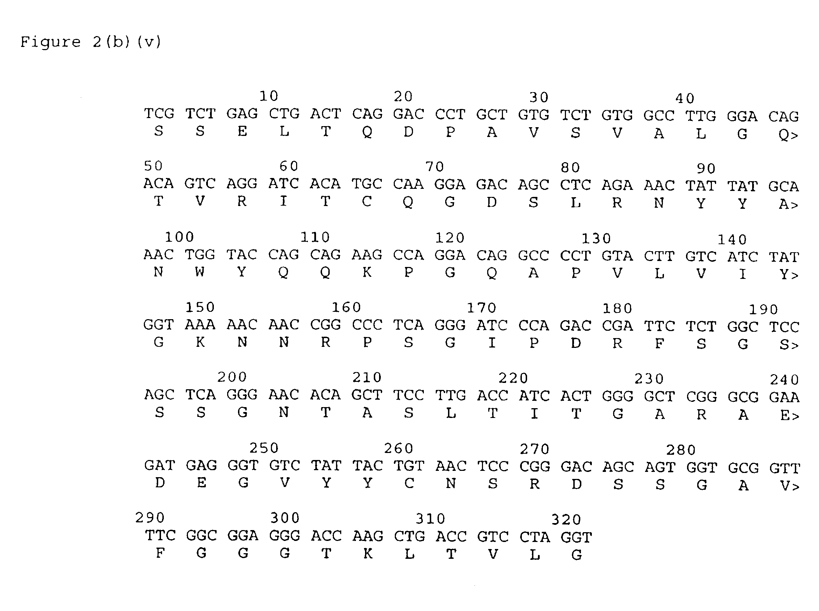

A preferred VH domain for anti-TGFβ2 specific binding members according to the present invention is that of 6H1 VH, whose sequence is shown in Figure 2(a) (i). 6H1 may be paired with a variety of VL domains, as exemplified herein. Amino acid sequence variants of 6H1 VH may be employed.

The specific binding member neutralises the *in vitro* and/or *in vivo* effect of TGFβ1.

The specific binding member may be a high affinity antibody. Preferred affinities are discussed elsewhere herein.

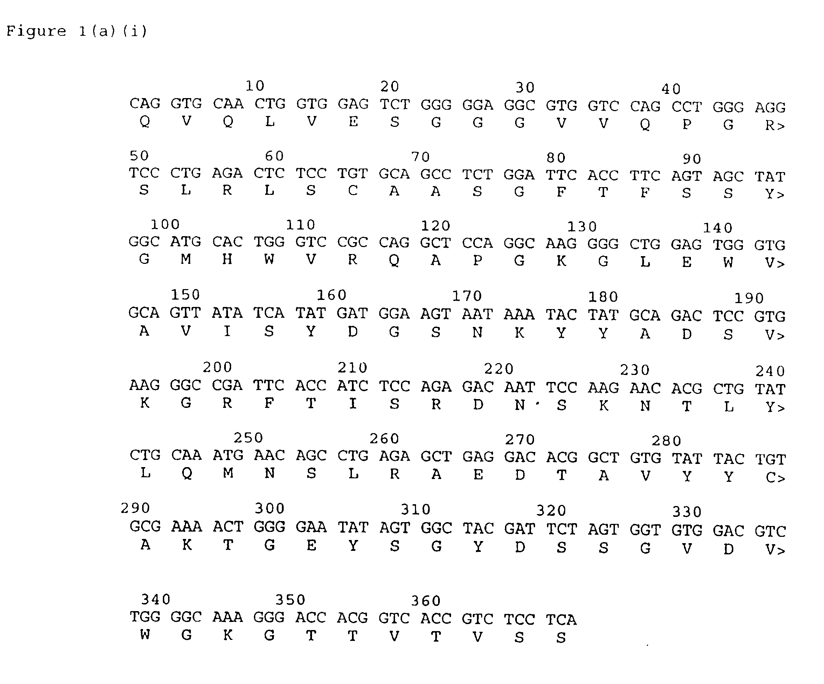

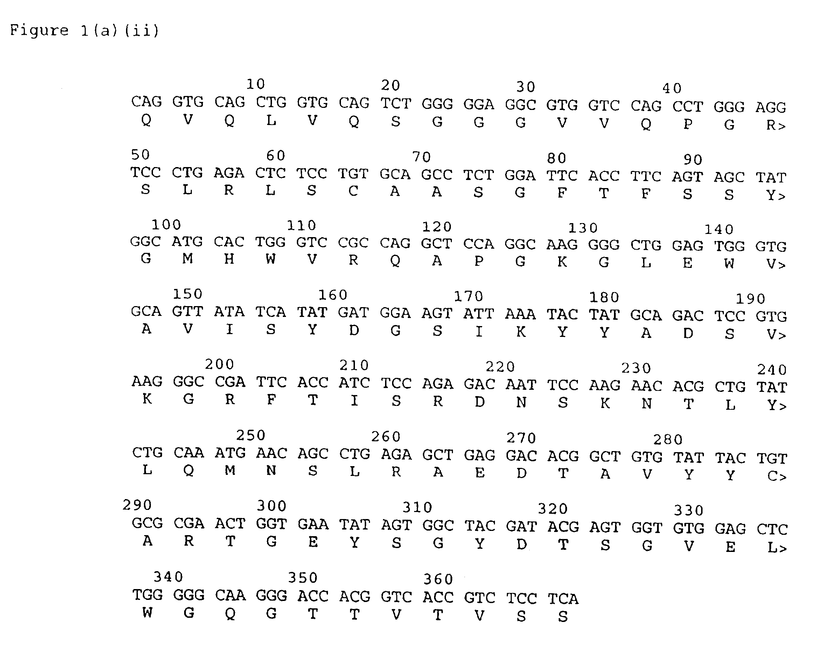

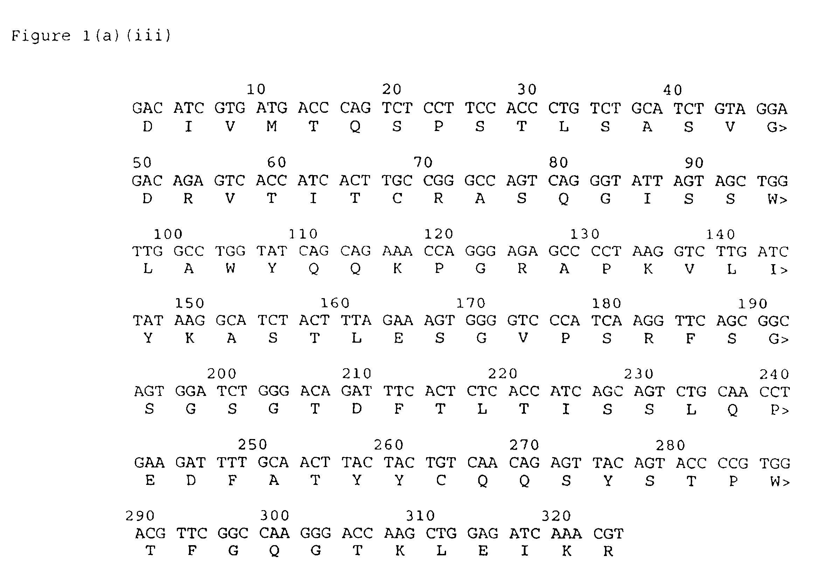

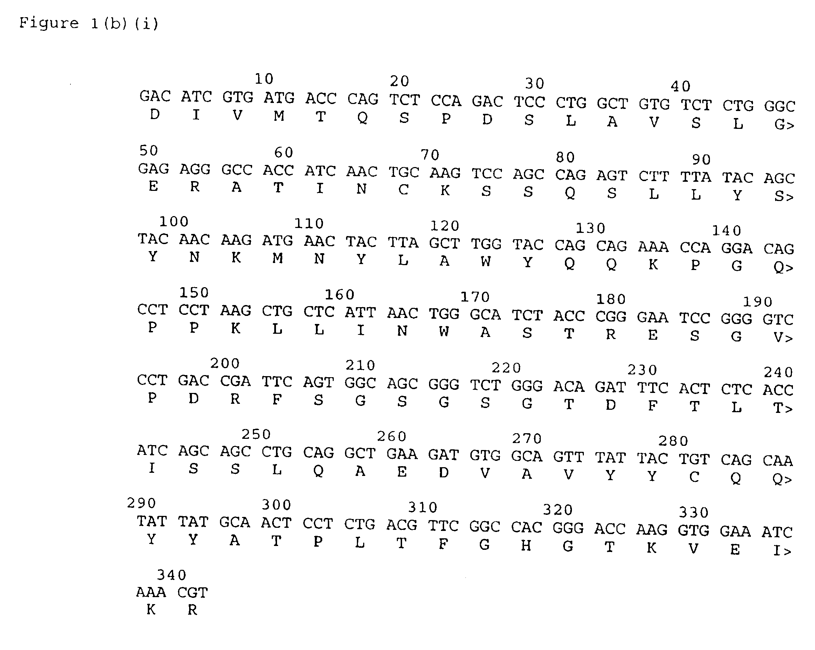

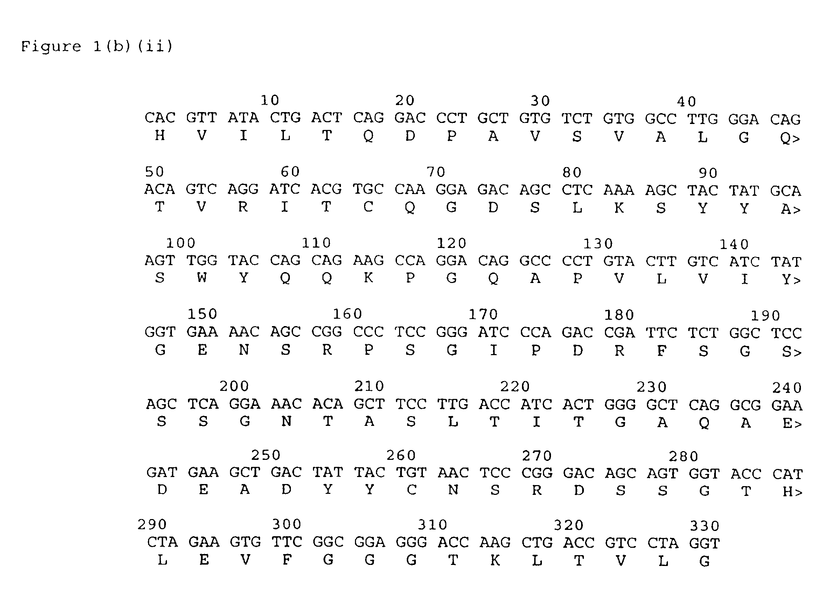

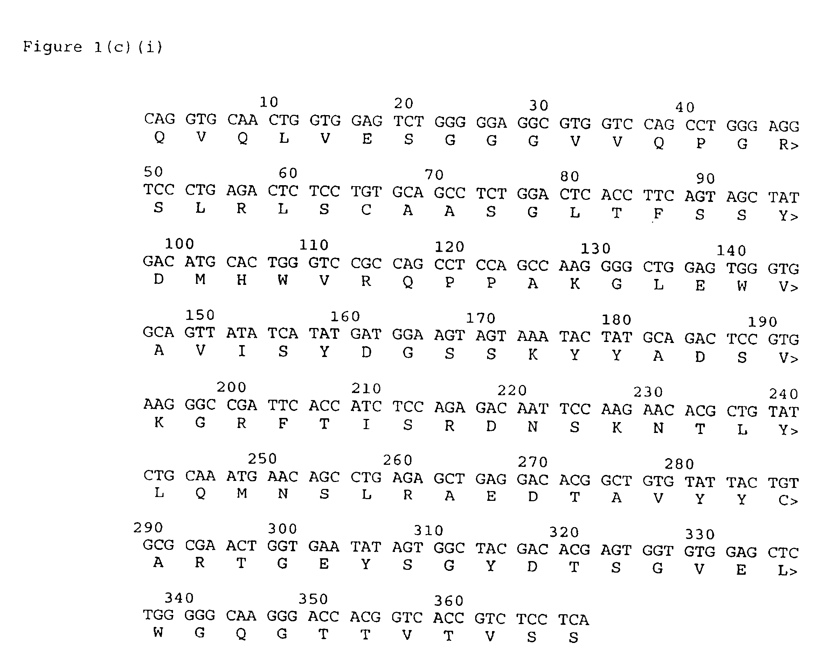

The binding domain may comprise a VH domain having an amino acid sequence as shown in Fig 1(a)(i) or (ii) or Fig 1(c)(i).

The binding domain may comprise a VH domain encoded by a nucleotide sequence as shown in Fig 1(a)(i) or (ii) or Fig 1(c)(i).

The binding domain may comprise a VL domain having either an amino acid sequence as shown in Fig 1(a)(iii) or Fig 1(b).

The binding domain may comprise a VL domain encoded by a nucleotide sequence as shown in Fig 1(a) (iii) or Fig 1(b).

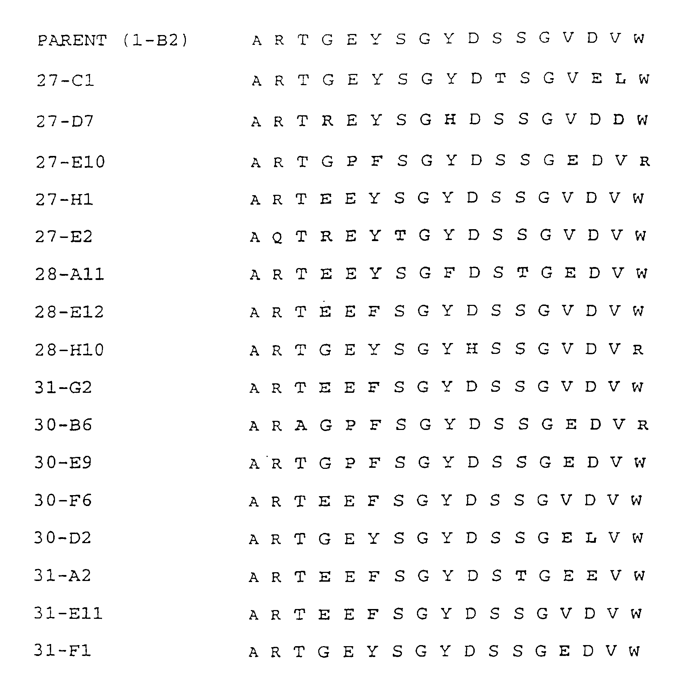

The binding domain may comprise part or all of a VH domain having a variant form of the Fig 1(a)(i) amino acid, the variant form being one of those as provided by Fig 3.

The specific binding member may be in the form of scFv.

A human antibody antigen, the binding domain may comprise one or more CDR (complementarity determining region) with an amino acid sequence shown in any of the figures. In a preferred embodiment, the binding domain comprises one or more of the CDRs, CDR1, CDR2 and/or CDR3 shown in the Figures. In a preferred embodiment, the binding domain comprises a VH CDR3 sequence as shown. The specific binding member may comprise all or part of the framework regions shown flanking and between the CDRs in the Figures, or different framework regions including modified versions of those shown.

So-called "CDR-grafting" in which one or more CDR sequences of a first antibody is placed within a framework of sequences not of that antibody, e.g. of another antibody is disclosed in EP-B-0239400.

A specific binding member comprising a human antibody antigen binding domain according to the invention may be one which competes for binding to human TGFβ1 with any specific binding member according to any of claims 1 to 3. Competition between binding members may be assayed easily *in vitro,* for example by tagging a specific reporter molecule to one binding member which can be detected in the presence of other untagged binding member(s), to enable identification of specific binding members which bind the same epitope or an overlapping epitope.

Preferred specific binding members for TGFβ1 compete for binding to TGFβ1 with the antibody CS37, discussed in more details elsewhere herein.

Specific binding members for TGFβ2 compete for binding to TGFβ2 with the antibody 6B1 discussed in more detail elsewhere herein. They may bind the epitope RVLSL or a peptide comprising the amino acid sequence RVLSL, particularly such a peptide which adopts an α-helical conformation. They may bind the peptide TQHSRVLSLYNTIN. In testing for this, a peptide with this sequence plus CGG at the N-terminus may be used. Specific binding members may be such that their binding for TGFβ2 is inhibited by a peptide comprising RVLSL, such as a peptide with the sequence TQHSRVLSLYNTIN. In testing for this, a peptide with this sequence plus CGG at the N-terminus may be used.

TQHSRVLSLYNTIN corresponds to the alpha helix H3 (residues 56-69) of TGFβ2, as discussed elsewhere herein. The equivalent region in TGFβ1 has the sequence TQYSKVLSLYNQHN. Anti-TGFβ1 antibodies which bind this region are of particular interest in the present invention, and are obtainable for example by panning a peptide with this sequence (or with CGG at the N-terminus) against a phage display library. Specific binding members which bind the peptide may be selected by means of their binding, and may be neutralising for TGFβ1 activity. Binding of such specific binding members to TGFβ1 may be inhibited by the peptide TQYSKVLSLYNQHN (optionally with CGG at the N-terminus).

A specific binding member which is specific for TGFβ2 may show no or substantially no binding for the latent form of TGFβ2, i.e. be specific for the active form of TGFβ2. 6B1 is shown in Example 6 to have this property.

6B1 is particularly suitable for therapeutic use in the treatment of fibrotic disorders because it has the following advantageous properties. 6B1 binds to TGFβ2 with a dissociation constant of 2.3nM in the single chain form and 0.89nM for the whole antibody form, 6B1 IgG4 (Example 13). The antibody 6B1 IgG4 neutralises the biological activity of TGFβ2 in an antiproliferation assay (IC₅₀ 2nM; examples 7 and 10) and in a radioreceptor assay (IC₅₀ less than 1nM; Table 6). The antibody binds to the peptide TQHSRVLSLYNTIN (TGFβ2₅₆₋₆₉) from the alpha helix H3 of TGFβ2 and recognises the corresponding peptide from TGFβ1 more weakly. 6B1 recognises the active but not the latent form of TGFβ2 (Example 6), recognises TGFβ2 in mammalian tissues by ICC and does not bind non-specifically to other human tissues (Example 12). The antibody preferentially binds to TGFβ2 as compared to TGFβ3, the cross-reactivity with TGFβ3 being 9% as determined by the ratio of the dissociation constants.

The other antibodies described in this application which contain the 6H1 VH domain, 6H1 and 6A5 have similar properties. The dissociation constants of were determined to be 2nM for 6B1 IgG4 (Example 2) and 0.7nM for 6A5 single chain Fv (Table 1). 6H1 IgG4 neutralises the biological activity of TGFβ2 with IC₅₀ values of 12 to 15nM (Examples 7 and 10). 6A5 and 6H1 inhibit receptor binding of TGFβ2 in a radioreceptor assay with IC₅₀ values of about 1nM in the single chain Fv format and 10nM or below in the whole antibody, IgG4 format. Both 6H1 IgG4 and 6A5 scFv were shown to be effective in the prevention of neural scarring (Example 5).

For antibodies against TGFβ1, the properties desired for an antibody to be effective in treatment of fibrotic disease are similar. Such antibodies neutralise TGFβ1 and have a dissociation constant for TGFβ1 of less than about 100nM, more preferably below about 10nM, more preferably below about 5nM. The antibodies preferentially bind to TGFβ1 as compared to TGFβ3, preferably have less than about 20% cross-reactivity with TGFβ3 (as measured by the ratio of the dissociation constants) and more preferably have less than about 10% cross-reactivity. The antibody preferably recognises the active but not the latent form of TGFβ1. The antibody 31G9 has a dissociation constant of 12nM (Table 5). The antibodies CS37 scFv and 27C1/10A6 IgG4 show IC₅₀ values in a radioreceptor assay of 8nM and 9nM, respectively, indicating a dissociation constant in the low nanomolar range. 27C1/10A6 IgG4 was shown to be effective in a neural scarring model. Cross-reactivity of antibodies of the 1B2 lineage with TGFβ3 is very low (Example 9).

In addition to an antibody sequence, the specific binding member may comprise other amino acids, e.g. forming a peptide or polypeptide, or to impart to the molecule another functional characteristic in addition to ability to bind antigen. For example, the specific binding member may comprise a label, an enzyme or a fragment thereof and so on.

The present invention also provides a polynucleotide which codes for a specific binding member according to any of claims 1 to 11.

The present invention also provides constructs in the form of plasmids, vectors, transcription or expression cassettes which comprise least one polynucleotide as above.

The present invention also provides a recombinant host cell which comprises one or more constructs as above.

A specific binding member according to the present invention may be made by expression from encoding nucleic acid. Nucleic acid encoding any specific binding member as provided itself forms an aspect of the present invention, as does a method of production of the specific binding member which method comprises expression from encoding nucleic acid therefor. Expression may conveniently be achieved by culturing under appropriate conditions recombinant host cells containing the nucleic acid. Following production by expression a specific binding member may be isolated and/or purified using any suitable technique, then used as appropriate.

Specific binding members and encoding nucleic acid molecules and vectors according to the present invention may be provided isolated and/or purified, e.g. from their natural environment, in substantially pure or homogeneous form, or, in the case of nucleic acid, free or substantially free of nucleic acid or genes origin other than the sequence encoding a polypeptide with the required function. Nucleic acid according to the present invention may comprise DNA or RNA and may be wholly or partially synthetic. The term "isolate" encompasses all these possibilities.

The nucleic acid may encode any of the amino acid sequences shown in any of the Figures, or any functionally equivalent form. The nucleotide sequences employed may be any of those shown in any of the Figures, or may be a variant, allele or derivative thereof. Changes may be made at the nucleotide level by addition, substitution, deletion or insertion of one or more nucleotides, which changes may or may not be reflected at the amino acid level, dependent on the degeneracy of the genetic code.

Systems for cloning and expression of a polypeptide in a variety of different host cells are well known. Suitable host cells include bacteria, mammalian cells, yeast and baculovirus systems. Mammalian cell lines available in the art for expression of a heterologous polypeptide include Chinese hamster ovary cells, HeLa cells, baby hamster kidney cells and many others. A common, preferred bacterial host is *E. coli.*

The expression of antibodies and antibody fragments in prokaryotic cells such as *E*. *coli* is well established in the art. For a review, see for example Plückthun, A. Bio/Technology **9**: 545-551 (1991). Expression in eukaryotic cells in culture is also available to those skilled in the art as an option for production of a specific binding member, see for recent reviews, for example Reff, M.E. (1993) Curr. Opinion Biotech. **4**: 573-576; Trill J.J. et al. (1995) Curr. Opinion Biotech 6: 553-560.

Suitable vectors can be chosen or constructed, containing appropriate regulatory sequences, including promoter sequences, terminator sequences, polyadenylation sequences, enhancer sequences, marker genes and other sequences as appropriate. Vectors may be plasmids, viral e.g. 'phage, or phagemid, as appropriate. For further details see, for example, *Molecular Cloning: a Laboratory Manual:* 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in *Short Protocols in Molecular Biology,* Second Edition, Ausubel et al. eds., John Wiley & Sons, 1992. The disclosures of Sambrook et al. and Ausubel et al. are incorporated herein by reference.

Thus, a further aspect of the present invention provides a host cell containing nucleic acid as disclosed herein. A still further aspect provides a method comprising introducing such nucleic acid into a host cell. The introduction may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. For bacterial cells, suitable techniques may include calcium chloride transformation, electroporation and transfection using bacteriophage.

The introduction may be followed by causing or allowing expression from the nucleic acid, e.g. by culturing host cells under conditions for expression of the gene.

In one embodiment, the nucleic acid of the invention is integrated into the genome (e.g. chromosome) of the host cell. Integration may be promoted by inclusion of sequences which promote recombination with the genome, in accordance with standard techniques.

The present invention also provides a method which comprises using a construct as stated above in an expression system in order to express a specific binding member or polypeptide as above.

Following production of a specific binding member it may be used for example in any of the manners disclosed herein, such as in the formulation of a composition, pharmaceutical or a diagnostic product, such as a kit comprising in addition to the specific binding member one or more reagents for determining binding of the member to cells, as discussed. A composition may comprise at least one component in addition to the specific binding member.

The present invention also provides pharmaceuticals which comprise a specific binding member as above, optionally with one or more excipients.

The present invention also provides the use of a specific binding member as above in the preparation of a medicament to treat a condition in which it is advantageous to counteract the fibrosis promoting effects of TGFβ. The condition may be a fibrotic condition characterized by an accumulation in a tissue of components of the extracellular matrix. The components of the extracellular matrix may be fibronectin or laminin.

The condition may be selected from the group consisting of: glomerulonephritis, neural scarring, dermal scarring, ocular scarring, lung fibrosis, arterial injury, proliferative retinopathy, retinal detachment, adult respiratory distress syndrome, liver cirrhosis, post myocardial infarction, post angioplasty restenosis, keloid scarring, scleroderma, vascular disorders, cataract, glaucoma, proliferative retinopathy.

The condition may be neural scarring or glomerulonephritis.

The present invention also provides the use of a specific binding member as above, in the preparation of a medicament to treat an immune/inflammatory disease condition in which it is advantageous to counteract the effects of TGFβ. Illustrative conditions are rheumatoid arthritis, macrophage deficiency disease and macrophage pathogen infection.

The present invention also provides a method which comprises administering to a patient a therapeutically effective amount of a specific binding member as above in order to treat a condition in which it is advantageous to counteract the fibrosis promoting effects of TGFβ. Fibrotic conditions are listed above.

The present invention also provides a method which comprises administering to a patient a prophylactically effective amount of a specific binding member as above in order to prevent a condition in which it is advantageous to prevent the fibrosis promoting effects of TGFβ. Fibrotic conditions are listed above.

The present invention also provides methods which comprise administering to patients prophylactically and/or therapeutically effective amounts of a specific binding member as above in order to prevent or treat an immune/inflammatory disease condition in which it is advantageous to counteract the effects of TGFβ. Illustrative conditions are stated above.

Thus, various aspects of the invention provide methods of treatment comprising administration of a specific binding member as provided, pharmaceutical compositions comprising such a specific binding member, and use of such a specific binding member in the manufacture of a medicament for administration, for example in a method of making a medicament or pharmaceutical composition comprising formulating the specific binding member with a pharmaceutically acceptable excipient.

In accordance with the present invention, compositions provided may be administered to individuals, which may be any mammal, particularly rodent, e.g. mouse, horse, pig, sheep, goat, cattle, dog, cat or human. Administration is preferably in a "therapeutically effective amount", this being sufficient to show benefit to a patient. Such benefit may be at least amelioration of at least one symptom. The actual amount administered, and rate and time-course of administration, will depend on the nature and severity of what is being treated. Prescription of treatment, eg decisions on dosage etc, is within the responsibility of general practioners and other medical doctors. Appropriate doses of antibody are well known in the art; see Ledermann J.A. et al. (1991) Int J. Cancer 47: 659-664; Bagshawe K.D. et al. (1991) Antibody, Immunoconjugates and Radiopharmaceuticals 4: 915-922.

A composition may be administered alone or in combination with other treatments, either simultaneously or sequentially dependent upon the condition to be treated.

Pharmaceutical compositions according to the present invention, and for use in accordance with the present invention, may comprise, in addition to active ingredient, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. intravenous.

Pharmaceutical compositions for oral administration may be in tablet, capsule, powder or liquid form. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

Further aspects of the invention and embodiments will be apparent to those skilled in the art. In order that the present invention is fully understood, the following examples are provided by way of exemplification only and not by way of limitation.

Reference is made to the following figures.

Figure 1 shows the DNA and protein sequences of antibodies specific for TGFβ1. Figure 1(a) shows the amino acid and encoding nucleic acid sequences of antibody variable domains of antibodes to TGFβ1 isolated directly from repertoires: Figure 1(a)(i)-1B2 VH (also known as 7A3 VH); Figure 1(a) (ii) - 31G9 VH; Figure 1(a) (iii) - 31G9 VL. Figure 1 (b) shows the amino acid and encoding nucleic acid sequences of antibody light chain variable domains of antibodies to TGFβ1 isolated by chain shuffling: Figure 1(b) (i) - 7A3 VL; Figure 1(b) (ii) - 10A6 VL. Figure 1(c) (i) shows the amino acid and encoding nucleic acid sequences for 27C1 VH, from an antibody to TGFβ1 isolated from a CDR3 spiking experiment.

Figure 2 shows the DNA and protein sequences of antibodies specific for TGFβ2. Figure 2(a) shows amino acid and encoding nucleic acid sequences for variable domains of antibodies to TGFβ2 isolated directly from repertoires: Figure 2(a) (i) - 2A-H11 VH (also known as 6H1 VH); Figure 2(a) (ii) - 2A-A9 VH (also known as 11E6 VH). Figure 2(b) shows amino acid and encoding nucleic acid sequences of antibody variable domains of antibodies specific for TGFβ2 isolated following chain shuffling: Figure 2(b) (i) - 6H1 VL; Figure 2(b) (ii) - 6A5 VL; Figure 2(b) (iii) - 6B1 VL; Figure 2(b) (iv) 11E6 VL; (v) Figure 2(b) (v) - 14F12 VL.

Figure 3 shows the protein sequences of VH CDR3 of clones derived from 1B2 by 'spiking' mutagenesis. Differences from 1B2 VH CDR3 are in bold.

Figure 4 shows the DNA and protein sequence of the VH and VL domains of VT37, cross-reactive between TGFβ1 and TGFβ2.

Figure 5 shows the DNA sequence and encoded amino acid sequence in the region of the heavy chain VH leader from the vector vhcassette2. Restriction enzymes HindIII, SfiI, PstI, BstEII, BamHI and EcoRI cut at the points indicated.

Figure 6 shows a map of the vector pG4D100 (not to scale). Multiple cloning site (MCS): 5'-HindIII-PacI-BamHI-(XanI)-(PmlI)-(NheI)-AscI-(BssHII)-XhoI-PmeI-BsiWI-3'. Restriction sites shown in brackets are not unique.

Figure 7 shows the DNA sequence, including intron, and encoded amino acid sequence in the region of the light chain VL leader for the vector vlcassettel (vlcassette CAT1). Restriction enzymes HindIII, ApaLI, SacI, XhoI and BamHI cut at the sites indicated (ApaLI within the leader).

Figure 8 shows a map of the vector pLN10 (not to scale). Multiple cloning site (MCS): 5'-HindIII-(SphI)-(PstI)-SalI-XbaI-BamHI-3' (1224-1259. Restriction sites shown in brackets are not unique.

Figure 9 shows a map of the vector pKN100 (not to scale). Multiple cloning site (MCS): 5'-MluI-(AvaI)-HindIII-(SphI)-(PstI)-SalI-XbaI-BamHI-3'. Restriction sites shown in brackets are not unique.

Figure 10 shows the % neutralisation of TGFβ2 activity by single chain Fv antibodies in an assay using proliferation of the erythroleukaemia cell line TF1 at different nM concentrations of scFv.

Figure 11 shows the neutralisation of TGFβ2 activity by whole IgG4 antibodies in an assay using proliferation of the erythroleukaemia cell line TF1 at different nM concentrations of antibody.

Figure 12 shows the effect of treatment of animals with antibodies on neural scarring as measured by the deposition of (Figure 12(a)) fibronectin and (Figure 12(b)) laminin detected using integrated fluorescence intensity. The graphs show scatter plots of individual animal data points. The bar graph shows the mean integrated fluorescence intensity of the group.

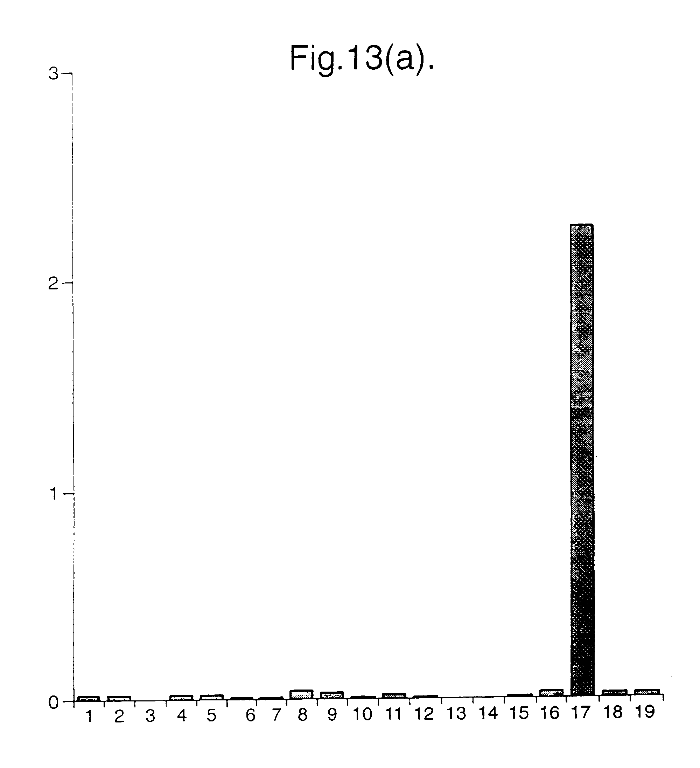

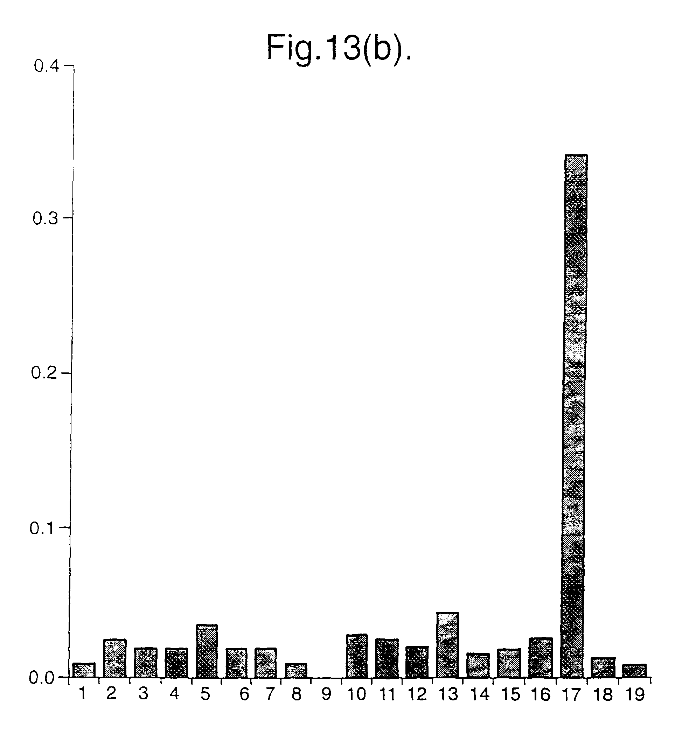

Figure 13 shows the results of an ELISA to measure the cross-reactivity of the antibodies 6B1 IgG4 and 6A5 IgG4 with TGFβ isoforms and non-specific antigens. Figure 13(a) shows cross-reactivty of 6B1 IgG4 to a panel of non-specific antigens and TGFβ's, plotting OD405nm for each antigen: 1 - interleukin 1; 2 - human lymphotoxin (TNFβ); 3 - human insulin; 4 - human serum albumin; 5 - ssDNA; 6 - oxazolone-bovine serum albumin; 7 - keyhole limpet haemocyanin; 8 - chicken egg white trypsin inhibitor; 9 - chymotrypsinogen; 10 - cytochrome C; 11 - GADPH; 12 - ovalbumin; 13 - hen egg lysozyme; 14 - bovine serum albumin; 15 - TNFα; 16 - TGFβ1; 17 - TGFβ2; 18 - TGFβ3; 19 - PBS only. Figure 13(b) shows the OD405nm for the antibody 6A5 IgG4 against the same panel of antigens. For both Figure 13(a) and Figure 13(b), antigens 1 to 15 were used for coating the plate at a concentration of 10µg/ml in PBS. The TGFbetas were coated at 0.2µg/ml in PBS. Coating was performed at 4°C overnight. 100µg of each antigen was used per well and duplicates of each antigen for each IgG to be tested. IgG samples were incubated with the coated antigens at 37°C for 2 hours after blocking with 2% marvel-PBS. The labelled second antibody was a mouse anti-human Fc1 alkaline phosphatase conjugated and the substrate used to detect bound second antibody was PNPP at 1mg/ml with the absorbance read at 405nm.

Figure 14 shows the amino acid and encoding nucleic acid sequence for the VL domain of the TGFβ1-specific antibody CS37.

Figure 15 shows data from an ELISA detecting binding of 6B1 IgG4 to BSA conjugated with either peptide TGFβ2₅₆₋₆₉ or peptide TGFβ1₅₆₋₆₉ coated on to an ELISA plate. 6B1 IgG4 was incubated at various concentrations in µg/ml and the absorbance at 405nm measured after addition of the detection agents. OD405nm results are plotted at the various concentrations for BSA-TGFβ2₅₆₋₆₉ ("Beta2 peptide" - diamonds) and BSA-TGFβ1₅₆₋₆₉ ("Betal peptide" - squares).

Figure 16 shows % neutralisation of TGF-β2 antiproliferative effect on TF1 cells by whole antibodies, 6H1 IgG4, 6B1 IgG4 and the mouse monoclonal from Genzyme, at various concentrations (nM IgG).

Figure 17 shows % neutralisation of TGF-β1 antiproliferative effect on TF1 cells by whole antibodies,6H1 IgG4, 6B1 IgG4 and the mouse monoclonal from Genzyme, at various concentrations (nM IgG).

Figure 18 shows % neutralisation of TGF-β3 antiproliferative effect on TF1 cells by whole antibodies, 6H1 IgG4, 6B1 IgG4 and the mouse monoclonal from Genzyme, at various concentrations (nM IgG).

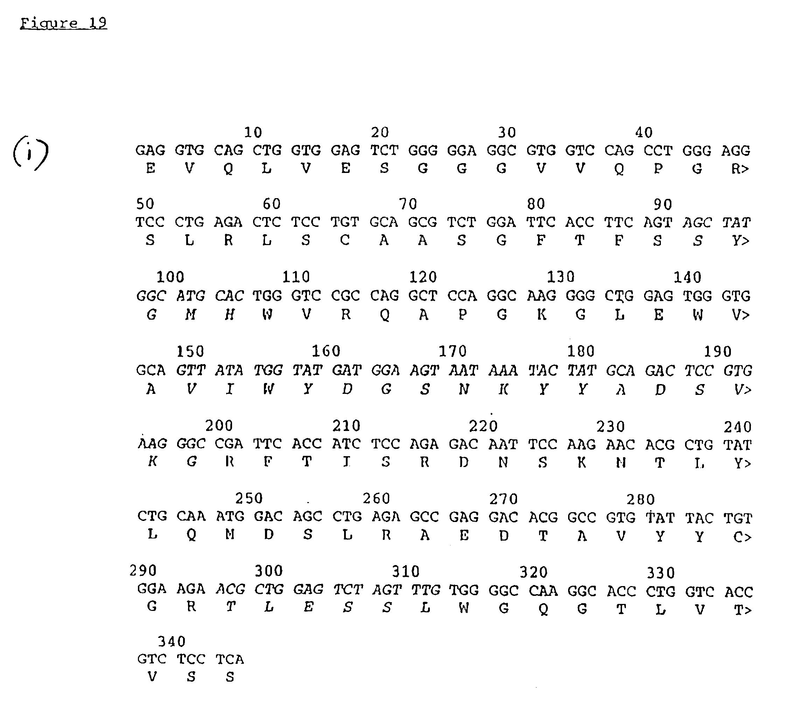

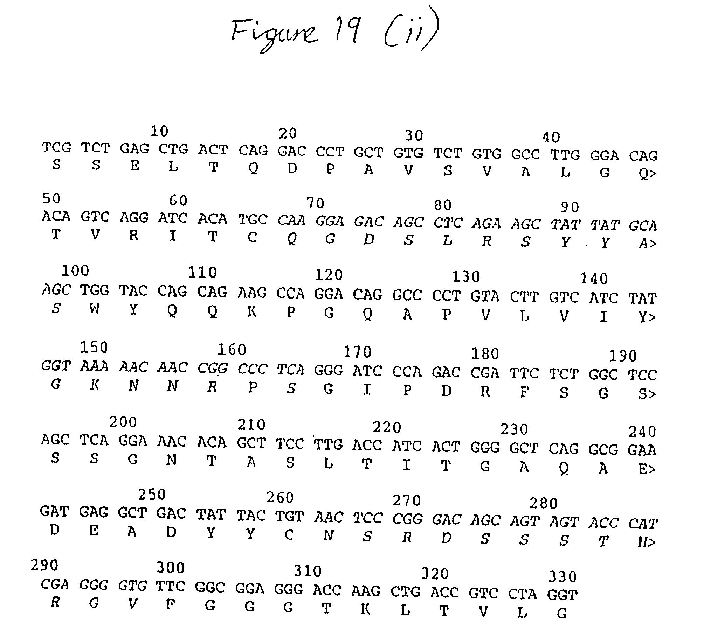

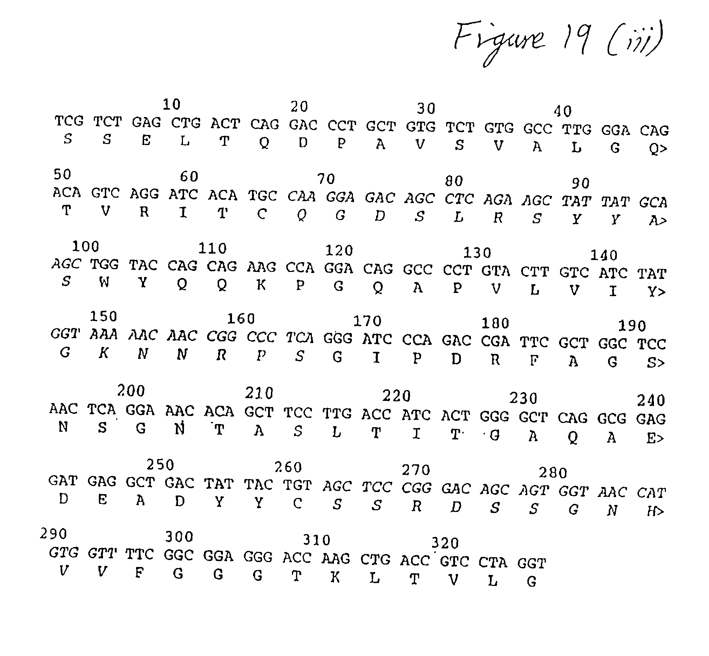

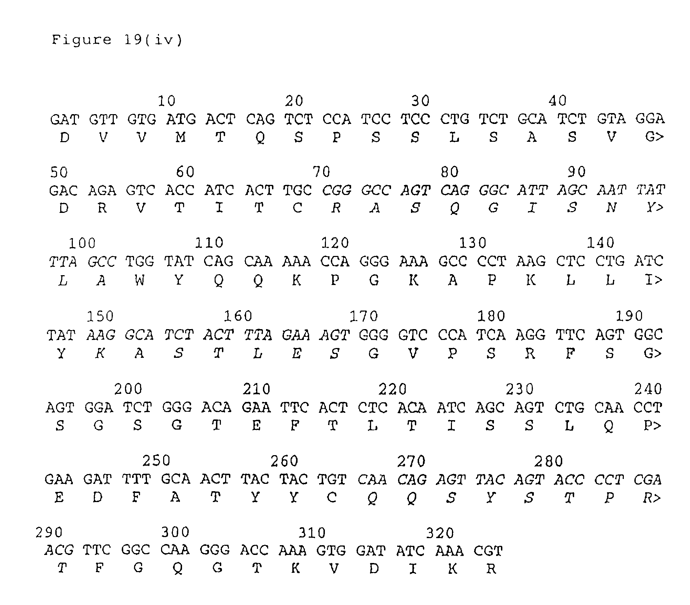

Figure 19 shows amino acid and encoding DNA sequences of regions of antibodies directed against TGFβ2 showing CDR sequences in italics: Figure 19(i) 2A-H11 VH (also known as 6H1 VH); Figure 19(ii) 6B1 VL; Figure 19(iii) 6A5 VL and Figure 19(iv) 6H1 VL.

Figure 20 shows the vector p6Hl VH-gamma4 (7263 bp). The gene encoding 6H1 VH is inserted as a HindIII-ApaI restriction fragment.

Figure 21 shows the vector p6Bl lambda (10151 bp). The gene encoding 6B1 VL is inserted as an EcoRI-BstBI restriction fragment.

Figure 22 shows the vector p6Bl gamma4gs (14176 bp). The genes encoding the heavy and light chains of 6BI IgG4 are combined in a single vector.

Figure 23 shows the results of competition ELISA experiments described in Example 6. Following overnight incubation with TGFβ2, plates were treated with the following solutions 1-4 (number corresponding to those in Figure): 1 - 400µl Hams F12/DMEM (reagent blank), 2 - 400µl Hams F12/DMEM plus 4µg 6B1 IgG4 antibody (positive control), 3 - 400µl PC3 untreated conditioned media plus 4µg 6B1 IgG4 antibody (latent TGFβ₂ sample), 4 - 400µl PC3 acid activated conditioned media plus 4µg 6B1 IgG4 antibody (active TGFβ₂ sample).

All documents mentioned herein are incorporated by reference.

### List of Examples

Example 1 - Isolation of antibodies specific for TGFβ1, antibodies specific for TGFβ2 and antibodies specific for TGFβ1 and TGFβ2.
Example 2 - Construction of cell lines expressing whole antibodies.
Example 3 - Neutralisation of TGFβ activity by antibodies assessed using in vitro assays.
Example 4 - Inhibition by antibodies of TGFβ binding to receptors.
Example 5 - Prevention of neural scarring using antibodies against TGFβ.
Example 6 - Determination of Binding of 6B1 IgG4 to Active or Latent Form of TGFβ₂.
Example 7 - Neutralisation by antibodies directed against TGFβ2 of the inhibitory effect of TGFβ isoforms on cells proliferation.
Example 8 - Inhibition by antibodies directed against TGFβ2 of binding of other TGFβ isoforms to receptors measured in a radioreceptor assay.
Example 9 - Assessment of TGFβ1 antibodies for potential therapeutic use.
Example 10 - Construction of a high expressing cell line for 6B1 IgG4 using the glutamine synthase selection system and assessment in a neutralisation assay.
Example 11 - Determination of the epitope on TGFβ2 for the antibody 6B1 using a peptide phage display library.
Example 12 - Determination of the binding of 6B1 IgG4 to tissues by immunocytochemistry (ICC).
Example 13 - Determination of the kinetic parameters of 6B1 IgG4 and single chain Fv for binding to TGFβ2.
Example 14 - Binding of a Peptide Corresponding to Residues 56 to 69 of TGFβ2 to 6B1 IgG4.

### EXAMPLE 1

### Isolation and Characterisation of Antibodies Binding to TGFβ1 and TGFβ2

### 1 Identification and Characterisation of Antibodies to Human TGFb-1 by Selection of Naive and Synthetic Phage Antibody Repertoires

### Antibody repertoires

The following antibody repertoires were used:
1. Peripheral blood lymphocyte (PBL) library derived from unimmunized human (Marks, J. D., Hoogenboom, H. R. Bonnert, T. P., McCafferty, J., Griffiths, A. D. & Winter, G. (1991) J. Mol. Biol. 222, 581-597)
2. Synthetic library (Nissim, A., Hoogenboom, H. R., Tomlinson, I. M., Flynn, G., Midgley, C., Lane, D. and Winter, G. (1994) EMBO J. 13, 692-698) derived from cloned human germline VH genes and synthetic CDR3s with a fixed light chain
3. Tonsil library derived from the tonsils of unimmunised humans. Tonsil B cells were isolated from freshly removed (processed within 2 hours) whole tonsils provided by Addenbrookes Hospital, Hills Road, Cambridge, U.K. Each tonsil was processed as follows. Tonsils were placed in a petri dish containing 5ml of PBS and macerated with a scalpel blade to release the cells. The suspension was transferred to a fresh tube and large debris allowed to sediment under gravity for 5 minutes. The cell suspension was then overlaid onto 10mls of Lymphoprep in a 50 ml polypropylene tube (Falcon) and centrifuged at 1000xg 20 minutes at room temperature (no brake) and cells at the interface harvested with a glass pipette. These were diluted to a final volume of 50 ml in RPMI medium at 37° C and centrifuged at 500xg for 15 minutes at room temperature. The supernatant was aspirated and the the cells washed another two times with RPMI.
Polyadenylated RNA was prepared from pelleted cells using the "QuickprepTM mRNA Kit" (Pharmacia Biotech, Milton Keynes, U.K.). The entire output of cells from one tonsil (ca. 1x10⁶ cells) was processed using one Oligo(dT)-Cellulose Spun column and processed exactly as described in the accompanying protocol. MRNA was ethanol precipitated as described and resuspended in 40ml RNase free water.
The cDNA synthesis reaction was set up using the "First-Strand cDNA Synthesis Kit (Pharmacia Biotech, Milton Keynes, U.K.) as follows:

| | |
|---|---|
| RNA | 20µl (heated to 67°C 10 minutes before use) |
| 1st strand buffer | 11µl |
| DTT solution | 1µl |
| pd(N)₆ primer | 1µl |

After gentle mixing, the reaction was incubated at 37°C for 1 hour.
Human VH genes were amplified from tonsil cDNA using the nine family-based back primers (VH 1b/7a -6a back *Sfi* , which introduce a *Sfi* I site at the 5'-end, Table 1) together with an equimolar mixture of the four JH forward primers (JH 1-2, 3, 4-5, 6, for; Marks et al., 1991 supra). Thus, nine primary PCR amplifications were performed. Each reaction mixture (50 µl) comprised 2 µl cDNA template, 25 pmol back primer, 25 pmol forward primers, 250 µM dNTPs, 1.5 mM MgCl₂, 50 mM KCl, 10 mM Tris-HCL pH 8.3 and 2.5 u of *Taq* polymerase (Boehringer). The reaction mixture was overlaid with mineral (paraffin) oil and was cycled 30 times (94 °C for 1 min, 55 °C for 1 min, 72 °C for 1 min) using a Techne thermal cycler. The products were purified on a 1% (w/v) agarose gel, isolated from the gel using "Geneclean" (Bio 101 Inc.) and resuspended in 15 µl of water. The amplified VH genes were recombined with human VL genes derived from PBLs (Marks et al., 1991 supra) together with the (Gly₄, Ser)₃ linker (Huston, J.S., et al. 1988 Proc Natl Acad Sci U S A. 85: 5879-83) by PCR assembly (Marks et al, 1991 supra). The VH-linker-VL antibody constructs were cloned into the SfiI and NotI sites of the phagemid vector, pCANTAB6 ( McCafferty, J., et al. 1994 Appl. Biochem. Biotech. 47: 157 - 173) to give a library of 6 x 10⁷ clones.
4. Large single chain Fv library derived from lymphoid tissues including tonsil, bone marrow and peripheral blood lymphocytes.
Polyadenylated RNA was prepared from the B-cells of various lymphoid tissues of 43 non-immunised donors using the "Quickprep mRNA Kit" (Pharmacia).
First-strand cDNA was synthesized from mRNA using a "First-strand cDNA synthesis" kit (Pharmacia) using random hexamers to prime synthesis. V-genes were amplified using family-specific primers for VH, Vκ and Vλ genes as previously described (Marks et al., supra) and subsequently recombined together with the (Gly₄, Ser)₃ scFv linker by PCR assembly. The VH-linker-VL antibody constructs were cloned into the *Sfi* I and *Not* I sites of the phagemid vector, pCANTAB 6. Ligation, electroporation and plating out of the cells was as described previously (Marks et al, 1991 supra). The library was made ca. 1000x larger than that described previously by bulking up the amounts of vector and insert used and by performing multiple electroporations. This generated a scFv repertoire that was calculated to have ca. 1.3 x 10¹⁰ individual recombinants which by *Bst* NI fingerprinting were shown to be extremely diverse.

### a. Induction of phage antibody libraries

The four different phage antibody repertoires above were selected for antibodies to TGFβ-1. The VH synthetic (Nissim et al., 1994 supra), tonsil, 'large' scFv and PBL (Marks et al., 1991 supra) repertoires were each treated as follows in order to rescue phagemid particles. 500 ml prewarmed (37 °C) 2YTAG (2YT media supplemented with 100 µg/ml ampicillin and 2 % glucose) in a 2 l conical flask was inoculated with approximately 3 x 10¹⁰ cells from a glycerol stock (-70 °C) culture of the appropriate library. The culture was grown at 37 °C with good aeration until the OD₆₀₀ₙₘ reached 0.7 (approximately 2 hours). M13K07 helper phage (Stratagene) was added to the culture to a multiplicity of infection (moi) of approximately 10 (assuming that an OD₆₀₀ₙₘ of 1 is equivalent to 5 x 10⁸ cells per ml of culture). The culture was incubated stationary at 37 °C for 15 minutes followed by 45 minutes with light aeration (200 rpm) at the same temperature. The culture was centrifuged and the supernatant drained from the cell pellet. The cells were resuspended in 500 ml 2YTAK (2YT media supplemented with 100 µg/ml ampicillin and 50 µg/ml kanamycin), and the culture incubated overnight at 30 °C with good aeration (300 rpm). Phage particles were purified and concentrated by three polyethylene glycol (PEG) precipitations (Sambrook, J., Fritsch, E.F., & Maniatis, T. (1990). Molecular Cloning - A Laboratory Manual. Cold Spring Harbour, New York) and resuspended in PBS to 10¹² transducing units (tu)/ml (ampicillin resistant clones).

### b. Panning of phage antibody library on TGFβ-1

Phage induced from the four repertoires were each separately panned on TGFβ-1. A 75mm x 12mm immuno tube (Nunc; Maxisorp) was coated with 2 ml of recombinant human TGFβ-1 (0.5ug/ml, Genzyme) in PBS overnight at 4 °C. After washing 3 times with PBS, the tube was filled with 3%MPBS (3 % 'Marvel' skimmed milk powder, lx PBS) and incubated for 2 hours at 37 °C for blocking. The wash was repeated, phagemid particles (10¹³ tu) in 2 ml of 3% MPBS were added and the tube incubated stationary at 37 °C for 1 hour. The tube was washed 20 times with PBST(0.1%), then 20 times with PBS. Bound phage particles were eluted from the tube by adding 2 ml of 100mM-triethylamine, and incubating the tube stationary at room temperature for 10 minutes. The eluted material was immediately neutralised by pipetting into a tube containing 1 ml 1M-Tris.HCl (pH7.4). Phage were stored at 4 °C. 1.5 ml of the eluted phage were used to infect 20 ml of logarithmically growing E. coli TG1 (Gibson, T.J. (1984). PhD thesis. University of Cambridge, UK.). Infected cells were grown for 1 hour at 37 °C with light aeration in 2YT broth, and then plated on 2YTAG medium in 243mm x 243mm dishes (Nunc). Plates were incubated overnight at 30 °C. Colonies were scraped off the plates into 10 ml of 2YT broth and 15 % (v/v) glycerol added for storage at -70 °C.

Glycerol stock cultures from the first round of panning of each of the four repertoires on TGFβ-1 were each rescued using helper phage to derive phagemid particles for the second round of panning. 250 µl of glycerol stock was used to inoculate 50 ml 2YTAG broth, and incubated in a 250 mL conical flask at 37 °C with good aeration until the OD₆₀₀ₘₙ reached 0.7 (approximately 2 hours). M13K07 helper phage (moi=10) was added to the culture which was then incubated stationary at 37 °C for 15 minutes followed by 45 minutes with light aeration (200 rpm) at the same temperature. The culture was centrifuged and the supernatant drained from the cell pellet. The cells were resuspended in 50 ml prewarmed 2YTAK, and the culture incubated overnight at 30 °C with good aeration. Phage particles were purified and concentrated by PEG precipitation (Sambrook et al., 1990 supra) and resuspended in PBS to 1013 tu/ml.

Phage induced from the first round of panning of each of the three repertoires, was selected a second time essentially as described above except that the panning tube was coated with only 1 ml of TGFβ-1 (0.5ug/ml, Genzyme), and the volume of phage added to the tube similarly reduced. After extensive washing, bound phage were eluted from the tube using 1 ml of 100 mM-triethylamine, and neutralised by the addition of 0.5 ml 1M-Tris.HCl (pH7.4) as earlier described. The process of phage growth and panning was repeated over a third and a fourth round of selection.

### c. Growth of single selected clones for immunoassay

Individual colonies from the third and fourth round selections were used to inoculate 100 µl 2YTAG into individual wells of 96 well tissue culture plates (Corning). Plates were incubated at 30 °C overnight with moderate shaking (200 rpm). Glycerol to 15 % was added to each well and these master plates stored at -70 °C until ready for analysis.

### d. ELISA to identify anti-TGFβ-1 scFv

Clones specific for TGFβ-1 were identified by ELISA, using scFv displayed on phage or soluble scFv.

### i. Phage ELISA

Cells from the master plates were used to inoculate fresh 96 well tissue culture plates containing 100 µl 2YTAG per well. These plates were incubated at 37 °C for 6-8 hours or until the cells in the wells were growing logarithmically (OD600 0.2-1.0). M13K07 was added to each well to an moi of 10 and incubated stationary for 15 min then 45 min with gentle shaking (100 rpm), both at 37 °C. The plates were centrifuged at 2000 rpm for 10 min and the supernatant eluted. Each cell pellet was resuspended in 100 µl 2YTAK and incubated at 30 °C overnight.

Each plate was centrifuged at 2000 rpm and the 100 µl supernatant from each well recovered and blocked in 20 µl 18%M6PBS (18 % skimmed milk powder, 6 x PBS), stationary at room temperature for 1 hour. Meanwhile, flexible microtitre plates which had been blocked overnight stationary at 4 °C with either 50 µl 0.2 µg/ml TGFβ-1 in PBS or 50 µl PBS alone (giving an uncoated control plate), were washed 3 times in PBS and blocked for 2 h stationary at 37 °C in 3MPBS. These plates were then washed three times with PBS and 50 µl preblocked phage added to each well of both the TGFβ-1-coated or uncoated plate. The plates were incubated stationary at 37 °C for 1 h after which the phage were poured off. The plates were washed by incubating for 2 min in PBST three times followed by incubating for 2min in PBS three times, all at room temperature.

To each well of both the TGFβ-1-coated and the uncoated plate, 50 µl of a 1 in 10,000 dilution of sheep anti-fd antibody (Pharmacia) in 3MPBS was added and the plates incubated at 37 °C stationary for 1 h. Each plate was washed as described above and 50 µl of a 1 in 5,000 dilution donkey anti-sheep alkaline phosphatase conjugate (Sigma) in 3MPBS added and incubated stationary at 37 °C for 1 h. Plates were washed as described as above followed by two rinses in 0.9% NaCl. Alkaline phosphatase activity was visualised using either the chromagenic substrate pNPP (Sigma) or the Ampak system (Dako). The absorbance signal generated by each clone was assessed by measuring the optical density at either 405 nm (pNPP) or 492 nm (Ampak) using a microtitre plate reader. Clones were chosen for further analysis if the ELISA signal generated on the TGFβ-1-coated plate was at least double that on the uncoated plate.

### ii. Soluble ELISA

Cells from the master plates were used to inoculate fresh 96 well tissue culture plates containing 100 µl 2YTAG per well. These plates were incubated at 30 °C for 8 hours then centrifuged at 2000 rpm for 10 min and the supernatant eluted. Each cell pellet was resuspended in 100 µl 2YTA ( 2YT media supplemented with 100ug/ml ampicillin) containing 10 mM IPTG ( isopropyl-B-D-thiogalactopyranoside) and incubated at 30 °C overnight.

Each plate was centrifuged at 2000 rpm and the 100 ul supernatant from each well recovered and blocked in 20 µl 18%M6PBS stationary at room temperature for 1 hour. Meanwhile, flexible microtitre plates which had been blocked overnight stationary at 4 °C with either 50 µl 0.2 µg/ml TGFβ-1 in PBS or 50 µl PBS alone, were washed 3 times in PBS and blocked for 2 h stationary at 37 °C in 3%MPBS. These plates were then washed three times with PBS and 50 µl preblocked soluble scFv added to each well of both the TGFβ-1-coated or uncoated plate. The plates were incubated stationary at 37 °C for 1 h after which the scFv solutions were poured off. The plates were washed by incubating for 2 min in PBST ( PBS containing 1% Tween) three times followed by incubating for 2 min in PBS three times, all at room temperature.

To each well of both the TGFβ-1-coated and the uncoated plate, 50 µl of a 1 in 200 dilution of the anti-myc tag murine antibody 9E10 (Munro, S. & Pelham, H.R.B. (1986)Cell 46, 291-300) in 3MPBS was added and the plates incubated at 37 °C stationary for 1 h. Each plate was washed as described above and 50 µl of a 1 in 5,000 dilution goat anti-mouse alkaline phosphatase conjugate (Pierce) in 3MPBS added and incubated stationary at 37 °C for 1 h. Plates were washed as described above followed by two rinses in 0.9% NaCl. Alkaline phosphatase activity was visualised using either the chromagenic substrate pNPP (Sigma) or the Ampak system (Dako). The absorbance signal generated by each clone was assessed by measuring the optical density at either 405 nm (pNPP) or 492 nm (Ampak) using a microtitre plate reader. Clones were chosen for further analysis if the ELISA signal generated on the TGFβ-1-coated plate was at least double that on the uncoated plate.

### iii. Specificity ELISA

Clones identified as binding TGFβ-1 rather an uncoated well, as described above, were further analysed for fine specificity. Specificity ELISA's were carried out using scFv either displayed on phage or in solution as described above, except that 5 ml of media in 50 ml Falcon tubes were inoculated with each clone and grown to generate the phage or soluble scFv used in the ELISA. Microtitre plate wells were coated with 50 µl of either 0.2 µg/ml TGFβ-1, 0.2 µg/ml TGFβ-2, 10 µg/ml bovine serum albumin (BSA) or PBS (the uncoated well). After preblocking both the phage (or soluble scFv) and the microtitre plates, 50 µl blocked phage (or soluble scFv) from each clone was added to a well coated with either TGFβ-1, TGFβ-2, BSA or an uncoated well. As above, alkaline phosphatse activity was visualised using either the chromagenic substrate pNPP (Sigma) or the Ampak system (Dako). Clones were considered to be specific for TGFβ-1 if the ELISA signal generated in the TGFβ-1 coated well was at least five-fold greater than the signal on either TGFβ-2, BSA or an uncoated well.

### iv. Specificity determination by BIACore™

The antibodies were also shown to be specific for TGFβ1 compared to TGFβ2 ( obtained from R&D Systems Abingdon) by relative binding to theBIACore™ sensor chips coated with the appropriate antigen. TGFβ1 and TGFβ2 were immobilised by amine coupling to Biosensor CM5 sensorchips (Pharmacia) according to the manufacturers instructions. Single chain Fv fragments (35µl; purified by immobilized metal affinity chromatography as described in example 4) were injected over the immobilized antigen at a flow rate of 5µl/min. The amount of TGFβ bound was assessed as the total increase in resonance units (RUs) over this period. For 31G9 scFv an increase of 1059RUs was found with a TGFβ1 chip and 72 RUs was found with a TGFβ2 chip. Thus binding is much stronger to TGFβ1 than TGFβ2.

### e. Sequencing of TGFb1-Specific ScFv Antibodies

The nucleotide sequence of the TGFβ-1 specific antibodies was determined by first using vector-specific primers to amplify the inserted DNA from each clone. Cells from an individual colony on a 2YTAG agar plate were used as the template for a polymerase chain reaction (PCR) amplification of the inserted DNA using the primers pUC19reverse and fdtetseq (Table 1). Amplification conditions consisted of 30 cycles of 94 °C for 1 min, 55 °C for 1 min and 72 °C for 2 min, followed by 10 min at 72 °C. The PCR products were purified using a PCR Clean-up Kit (Promega) in to a final volume of 50 µl H20. Between 2 and 5 µl of each insert preparation was used as the template for sequencing using the Taq Dye-terminator cycle sequencing system (Applied Biosystems). The primers mycseq10 and PCR-L-Link were used to sequence the light chain of each clone and PCR-H-Link and pUC19reverse to sequence the heavy chain (Table 1)

### f. Sequence and Source of the Initial TGFβ-1-Specific ScFv Antibodies

Four different TGFβ-1 specific antibodies were isolated from the selections using the four libraries described above. Each clone name, its origin and its heavy and light chain germline is given below. The complete sequence of the VH domain genes of clones 1-B2 and 31-G9 are given in Figure 1(a) together with the VL domain gene from scFv 31-G9.

| CLONE | LIBRARY SOURCE | VH GERMLINE | VL ISOTYPE |
|---|---|---|---|
| 1-B2 | PBL | VH3 DP49 | VKappa |
| 1A-E5 | Synthetic VH | VH3 DP53 | VLambda |
| 1A-H6 | Tonsil | VH3 DP50 | VLambda |
| 31-G9 | large scFv | VH3 DP49 | VLambda |

Thus these initial isolates were obtained from libraries derived from different sources-both natural V genes of unimmunised humans and synthetic libraries from cloned germline V genes together with synthetic CDRs.

### 2. Affinity Maturation of the Initial TGFβ-1-Specific ScFv Antibodies

### a. Light Chain Shuffling of the TGFβ-1-Specific ScFv Antibody 1-B2

### i. Construction of Repertoires

The heavy chain of clone 1-B2 was recombined with the complete repertoire of light chains derived from the PBL and large (tonsil-derived) scFv repertoires. The 1-B2 heavy chain was amplified by PCR using the primers HuJh4-5For (Table 1) and pUC19reverse. Amplification conditions consisted of 30 cycles of 94 °C for 1 min, 55 °C for 1 min and 72 °C for 1min, followed by 10 min at 72 °C. The PCR product was separated through a 1% agarose-TAE gel, the band representing the amplified VH excised, and eluted from the agarose gel using the Geneclean Kit (Bio 101).

The PBL and tonsil light chains were amplified by PCR using the primers fdtetseq and a mix of RL1, 2 & 3 (Table 1). Amplification conditions consisted of 30 cycles of 94 °C for 1 min, 55 °C for 1 min and 72 °C for 1min, followed by 10 min at 72 °C. The PCR product was separated through a 1% agarose-TAE gel, the band representing the amplified VL excised, and eluted from the agarose gel using the Geneclean Kit (Bio 101).

Approximately 50 ng amplified 1-B2 heavy chain and 50 ng of either amplified PBL-derived or amplified tonsil-derived light chains were combined and precipitated with sodium acetate and ethanol using 25 µg glycogen as a carrier. The precipitated DNA was pelleted by centrifugation at 13,000 rpm in a microfuge, air dried and resuspended in 26 µl H20. This was used in an assembly amplification after the addition of reaction buffer to 1X, dNTP's to 200 nM and 5 units Taq polymerase. Amplification conditions consisted of 20 cycles of 94 °C for 1 min, 60 °C for 1 min and 72 °C for 1min 30 s, followed by 10 min at 72 °C. 10 µl of each assembly was used as the template in a 'pull-through' amplification with the primers fdtetseq and pUC19reverse. Amplification conditions consisted of 25 cycles of 94 °C for 1 min, 60 °C for 1 min and 72 °C for 1min 30 s, followed by 10 min at 72 °C.

The pull-through amplification product was separated through 1% agarose-TAE and the band representing the pull-through VH-VL excised and eluted using the Geneclean Kit. This was digested with the restriction endonucleases Sfi I and Not I (NEB) and ligated (Amersham ligation system) into the phagemid vector pCantab 6, previously digested with Sfi 1 and Not I. The ligation product was used to transform electrocompetent TG1 cells, plated out on 2YTAG plates and incubated overnight at 30 °C. Approximately 1 x 10⁵ individual clones were generated from the light chain-shuffle of the 1-B2 heavy chain with the PBL-derived light chains and approximately 1 x 10⁶ for the shuffle with the tonsil-derived light chains.

### ii. Selection of Light Chain Shuffle Repertoires

The two light chain-shuffle repertoires were selected for TGFβ-1-specific antibodies. Phagemid particles were recovered from each repertoire as described earlier for the initial libraries. Recovered phage were preblocked for 1 h in a final volume of 100 µl 3MPBS. Approximately 10¹¹ tu phage were used in the first round selection and between 10⁹ and 10¹⁰ for subsequent selections. For the first round selections, biotinylated TGFβ1 to a final concentration of 100 nM was added to the preblocked phage and incubated stationary at 37°C for 1h.

For each selection, 100 µl Dynabeads suspension (Dynal) was separated on a magnet and the beads recovered and preblocked for 2 h in 1 ml 3MPBS. The beads were recovered on a magnet and resuspended in the phagemid/biotinylated TGFβ-1 mixture and incubated at room temperature for 15 min while being turned end-over-end. The beads were captured on a magnet and washed four times with PBST followed by three washes in PBS. After each wash, the beads were captured on a magnet and resuspended in the next wash. Finally, half of the beads were resuspended in 10 µl 50 mM DTT (the other half of the beads stored at 4 °C as a back-up) and incubated at room temperature for 5 min. The whole bead suspension was then used to infect 5 ml logarithmically-growing TG1 cells. This was incubated at 37 °C, stationary for 15 min then with moderate shaking for 45 min, plated on 2YTAG plates and incubated overnight at 30 °C.

Colonies were scraped off the plates into 10 ml of 2YT broth and 15 % % (v/v) glycerol added for storage at -70 °C. A 250 µl aliqout of each plate scrape was used to inoculate 2YTAG and phagemid particles rescued as described earlier. For each repertoire, three rounds of selection using biotinylated TGFβ-1 was performed, essentially identical to the first round selection described above. All selections were at 100 nM TGFβ-1 except for the third round selection of the tonsil-derived light chain repertoire where the concentration of biotinylated TGFβ-1 in the selection was reduced to 50 nM.

### iii. Identification of TGFβ-1-Specific ScFv Antibodies from Light Chain Shuffle Repertoires

ScFv antibodies specific to TGFβ-1 were identified by both phage and soluble ELISA, and sequenced, as described earlier. Three new TGFβ-1-specific scFv antibodies were identified, two with PBL-derived light chains and one with a tonsil-derived light chain. All three had the 1B2 heavy chain sequence (DP49), described earlier. The sequences are summarised below and the complete sequence of each VL domain gene is given in figure 1(b).

| CLONE | VL SOURCE | VH GERMLINE | VL ISOTYPE |
|---|---|---|---|
| 7-A3 | PBL | DP49 (1B2) | VKappa |
| 10-A6 | PBL | DP49 (1B2) | VLambda |
| 14-A1 | Tonsil | DP49 (1B2) | VLambda |

Thus the VH domain 1B2 derived from the PBL library can be combined with VL domains derived from both PBL and tonsil libraries.

### b. CDR3 'Spiking' of the TGFβ-1-Specific ScFv Antibody 1B2

### i. Construction of 'spiked' repertoire

An 84 mer mutagenic oligonucleotide primer, 1B2 mutVHCDR3, was first synthesized (see Table 1). This primer was 'spiked' at 10%; i.e. at each nucleotide position there is a 10% probability that a non-parental nucleotide will be incorporated. The 1-B2 heavy chain was amplified by PCR using the primers pUC19reverse and 1B2 mutVHCDR3. Amplification conditions consisted of 30 cycles of 94 °C for 1 min, 55 °C for 1 min and 72 °C for 1min, followed by 10 min at 72 °C. The PCR product was separated through a 1% agarose-TAE gel, the band representing the amplified VH excised, and eluted from the agarose gel using the Geneclean Kit (Bio 101).

The parental 1B2 light chain was amplified by PCR using the primers fdtetseq and RL3 (Table 1). Amplification conditions consisted of 30 cycles of 94 °C for 1 min, 55 °C for 1 min and 72 °C for 1min, followed by 10 min at 72 °C. The PCR product was separated through a 1% agarose-TAE gel, the band representing the amplified VL excised, and eluted from the agarose gel using the Geneclean Kit (Bio 101).

Approximately 50 ng amplified 'spiked' 1-B2 heavy chain and 50 ng of amplified parental 1B2 light chain were combined and precipitated with sodium acetate and ethanol using 25 µg glycogen as a carrier. The precipitated DNA was pelleted by centrifugation at 13,000 rpm in a microfuge, air dried and resuspended in 26 µl H20. This was used in an assembly amplification after the addition of reaction buffer to 1X, dNTP's to 200 nM and 5 units Taq polymerase. Amplification conditions consisted of 25 cycles of 94 °C for 1 min, 65 °C for 4 min. Five µl of each assembly was used as the template in a 'pull-through' amplification with the primers fdtetseq and pUC19reverse. Amplification conditions consisted of 30 cycles of 94 °C for 1 min, 55 °C for 2 min and 72 °C for 1min, followed by 10 min at 72 °C.

The pull-through amplification product was separated through 1% agarose-TAE and the band representing the pull-through 'spiked' VH -VL excised and eluted using the Geneclean Kit. This was digested with the restriction endonucleases Sfi I and Not I (NEB) and ligated (Amersham ligation system) into the phagemid vector pCantab 6, previously digested with Sfi I and Not I. The ligation product was used to transform electrocompetent TG1 cells, plated out on 2YTAG plates and incubated overnight at 30 °C. Approximately 4 x 10⁶ individual clones were generated from this VH CDR3 'spiking' of the 1-B2 VH CDR3.

### ii. Selection of 1B2 CDR3 Spike Repertoire

The repertoire was selected for new TGFβ-1-specific scFv antibody by one round of panning on 1 µg/ml TGFβ-1 followed by two rounds of selection with biotinylated TGFβ-1 at 50 nM using methods as described earlier.

### iii. Identification of TGFβ-1-Specific ScFv Antibodies from the 1B2 CDR3 Spike Repertoire

ScFv antibodies specific to TGFβ-1 were identified by both phage and soluble and phage ELISA, and sequenced, as described earlier. Clone 27C1 was isolated from the spiked repertoire. It is virtually identical to clone 1B2 but with three differences in the heavy chain CDR3. The complete sequence of clone 27C1 is given in figure 1 (c). The 27C1 VH domain was combined with the 10A6 VL domain in the construction of the whole antibody 27C1/10A6 IgG4 (example 2). The properties of this antibody are described in more detail in examples 2 to 6. In addition to 27C1, a large number of other antibodies were isolated with up to 7 of the 14 amino acids differing in CDR3 of the VH domain (Figure 3). These had a similar preference for binding TGFβ1 compared to TGFβ2.

### 3. Identification and Characterisation of Antibodies to Human TGFβ-2 by Selection of Naive and Synthetic Phage Antibody Repertoires

### a. Induction of phage antibody libraries

Two different phage antibody repertoires were selected for antibodies to TGFβ-2. The VH synthetic (Nissim et al., 1994) and tonsil (constructed as described earlier) repertoires were each treated as described for TGFβ-1 to rescue phagemid particles.

### b. Panning of phage antibody library on TGFβ-2

Phage induced from the two repertoires were each separately panned on TGFβ-2 as described earlier for TGFβ-1 but using 0.5 µg/ml TGFβ-2 as the coating antigen.

### c. Identification and Sequencing of TGFβ-2-Specific ScFv Antibodies

Individual colonies from the third and fourth round selections were screened by both phage and soluble ELISA as described earlier for TGFβ-1 but using flexible microtitre plates coated with TGFβ-2 at 0.2 µg/ml rather than TGFβ-1. Clones were chosen for further analysis if the ELISA signal generated on the TGFβ-2-coated plate was at least double that on the uncoated plate. For the specificity ELISA, as described earlier for TGFβ-1, clones were considered to be specific for TGFβ-2 if the ELISA signal generated in the TGFβ-2 coated well was at least five-fold greater than the signal on either TGFβ-1, BSA or an uncoated well.

### d. Sequence and Source of the Initial TGFB-2-Specific ScFv Antibodies

Four different TGFβ-2 specific antibodies were isolated from the selections using the two libraries described above. Each clone name, its origin and its heavy and light chain germline is given below. The complete sequence of the VH domain genes of 2A-H11 and 2A-A9 are given in Figure 2 (a).

| CLONE | LIBRARY SOURCE | VH GERMLINE | VL ISOTYPE |
|---|---|---|---|
| 1-G2 | Tonsil | | |
| 1-N6 | Tonsil | DP49 | |
| 2A-H11 | Synthetic VH | DP50 | VLambda |
| 2A-A9 | Synthetic | DP46 | VLambda |
| Gold-11 | Large scFv | | VLambda |

Thus human antibodies binding to human TGFβ2 have been isolated from different sources-, both natural Vgenes of unimmunised humans and synthetic libraries from cloned germline V genes together with synthetic CDRs.

### 4. Light Chain Shuffling of the TGFβ-2-Specific ScFv Antibodies 2A-H11 and 2A-A9

### a. Construction of Repertoires

The heavy chain of clones 2A-H11 and 2A-A9 were recombined wich the complete repertoire of light chains derived from the PBL and large (tonsil-derived) scFv repertoires as described earlier for the TGFβ-1-specific scFv antibody 1-B2. Both repertoires generated from the recombination with the PBL light chain repertoire were approximately 1 x 10⁵, those generated from the recombination with the tonsil light chain repertoire were approximately 1 x 10⁶.

### b. Selection of Light Chain Shuffle Repertoires

The light chain-shuffle repertoires were selected for TGFβ-2-specific antibodies using biotinylated TGFβ-2, as described earlier for the selection of the TGFβ-1 light chain shuffle repertoires. For all of the first and second round selections, a concentration of 100 nM biotinylated TGFβ-2 was used. For the third round selection of the PBL-derived light chain shuffle repertoire, biotinylated TGFβ-2 was used at concentrations of 100 nM and 1 nM. For the third round selection of the tonsil-derived light chain shuffle repertoire, biotinylated TGFβ-2 was used at a concentration of 50 nM.

### c. Identification of TGFβ-2-Specific ScFv Antibodies from Light Chain Shuffle Repertoires

ScFv antibodies specific to TGFβ-2 were identified by both phage and soluble ELISA, and sequenced, as described earlier. Five new TGFβ-2-specific scFv antibodies were identified. The sequences are summarised below and the complete sequence of each clone given in Figure 2 (b).

| CLONE | VL SOURCE | VH GERMLINE | VL ISOTYPE |
|---|---|---|---|
| 6-H1 | PBL | DP50 (2A-H11) | VKappa |
| 6-A5 | PBL | DP50 (2A-H11) | VLambda |
| 6-B1 | PBL | DP50 (2A-H11) | VLambda |
| 11-E6 | PBL | DP46 (2A-A9) | VKappa |
| 14-F12 | Tonsil | DP46 (2A-A9) | VLambda |

### d. Specificity determination by ELISA

Clones identified as binding TGFβ-2 rather an uncoated well, as described above, were further analysed for fine specificity. Specificity ELISA's were carried out using scFv either displayed on phage or in solution as described above, except that 5 ml of media in 50 ml Falcon tubes were inoculated with each clone and grown to generate the phage or soluble scFv used in the ELISA. Microtitre plate wells were coated with 50 µl of either 0.2 µg/ml TGFβ-1, 0.2 µg/ml TGFβ-2, 10 µg/ml bovine serum albumin (BSA) or PBS (the uncoated well). After preblocking both the phage (or soluble scFv) and the microtitre plates, 50 µl blocked phage (or soluble scFv) from each clone was added to a well coated with either TGFβ-1, TGFβ-2, BSA or an unccated well. As above, alkaline phosphatse activity was visualised using either the chromagenic substrate pNPP (Sigma) or the Ampak system (Dako). Clones were considered to be specific for TGFβ-2 if the ELISA signal generated in the TGFβ-2 coated well was at least five-fold greater than the signal on either TGFβ-1, BSA or an uncoated well. Cross-reactivity with unrelated antigens was determined more extensively for anti-TGFβ2 antibody in whole antibody format, see example 2. The cross-reactivity of 6B1 IgG4 and 6A5 IgG4 with TGFβ1 and TGFβ3 (obtained from R&D Systems, Abingdon ) is also shown to be very low.

### e. Specificity determination by BIACore™

The antibodies were also shown to be specific for TGFβ2 compared to TGFβ1 by relative binding to theBIACore sensor chips coated with the appropriate antigen. TGFβ1 and TGFβ2 were immobilised by amine coupling to Biosensor CM5 sensorchips (Pharmacia) according to the manufacturers instructions. Single chain Fv fragments (35µl; purified by immobilized metal affinity chromatography) were injected over the immobilized antigen at a flow rate of 5µl/min. The amount of TGFβ bound was assessed as the total increase in resonance units (RUs) over this period. For the single chain Fv fragments 6H1, 6A5 and 14F12, these fragments gave a total of 686, 480 and 616 RUs respectively for the TGFβ1 coated sensor chip and 77, 71 and 115 RUs respectively for the TGFβ2 coated chip.

### 5. Building higher affinity anti TGFβ-1 biological neutralisers

### a. Recombining heavy chains derived from high affinity anti-TGFβ1 scFv with light chains derived from anti-TGFβ1 and anti-TGFβ2 scFv showing good properties

Antibodies derived by spiking CDR3 of the scFv antibody 1-B2 (section 2b) bind TGFβ-1 with high affinity. To improve the chance of obtaining high affinity neutralising antibodies it was decided to chain shuffle VHs derived from high affinity anti-TGFβ-1 scFv with VLs derived from scFv clones with promising properties and particularly with those capable of neutralising the activity of TGFβ-2 in vitro.

Heavy chains were amplified by PCR from the repertoire of CDR3 spiked 1-B2 clones after selection on TGFβ-1(section 2a.ii) using the primers pUC19reverse and PCR-H-Link (Table 1). Amplification conditions consisted of 30 cycles of 94 °C for 1 min, 55 °C for 1 min and 72 °C for 1min, followed by 10 min at 72 °C. The PCR product was separated through a 1% agarcse-TAE gel, the band representing the amplified VH excised, and eluted from the agarose gel using the Geneclean Kit (Bio 101).

Light chains were separately amplified by PCR from each of the anti TGFβ-1 specific neutralisers (7-A3, 10-A6 and 14-A1; section 2a.iii) and each of the anti TGFβ-2 specific neutralisers (6H1, 6A5, 6B1, 11E6 and 14F12; section 4c) using the primers fdtetseq1 and PCR-L-Link (Table 1). The same PCR conditions were used as described for theVH amplification. Each VL PCR product was then separately purified through a 1% agarose-TAE gel as described above. Purified products were finally mixed in approximately equimolar amounts (as estimated from an analytical agarose gel) to provide a VL 'pool'.

Approximately 50 ng amplified heavy chains and 50 ng of amplified pooled light chains were combined and precipitated with sodium acetate and ethanol using 25 ug glycogen as a carrier. The precipitated DNA was pelleted by centrifugation at 13,000 rpm in a microfuge, air dried and resuspended in 23 µl H20. This was used in an assembly amplification after the addition of reaction buffer, dNTP's to 200 nM and 5 units Taq polymerase. Amplification conditions consisted of 20 cycles of 94 °C for 1 min, 55 °C for 1 min and 72 °C for 2 mins, followed by 10 min at 72 °C. 5 µl of assembly was used as the template in a 50ul 'pull-through' amplification with the primers fdtetseq and pUC19reverse. Amplification conditions consisted of 30 cycles of 94 °C for 1 min, 55 °C for 1 min and 72 °C for 2mins, followed by 10 min at 72 °C.

The pull-through amplification product was separated through 1% agarose-TAE and the band representing the pull-through VH-VL excised and eluted using the Geneclean Kit. This was digested with the restriction endonucleases Sfi I and Not I (NEB) and ligated into the phagemid vector pCantab 6 (McCafferty et al. 1994 supra), previously digested with Sfi 1 and Not I, using the Amersham ligation system. The ligation product was used to transform electrocompetent TG1 cells, plated out on 2YTAG plates and incubated overnight at 30 °C. A repertoire of approximately 3 x 10⁶ individual clones was generated.

### b. Selection of chain shuffled repertoire

The chain shuffled repertoire was selected by a single round of panning on TGFβ-1 (1ug/ml), as previously described (section 1b).

### c. Identification of TGFβ-1 specific scFv antibodies

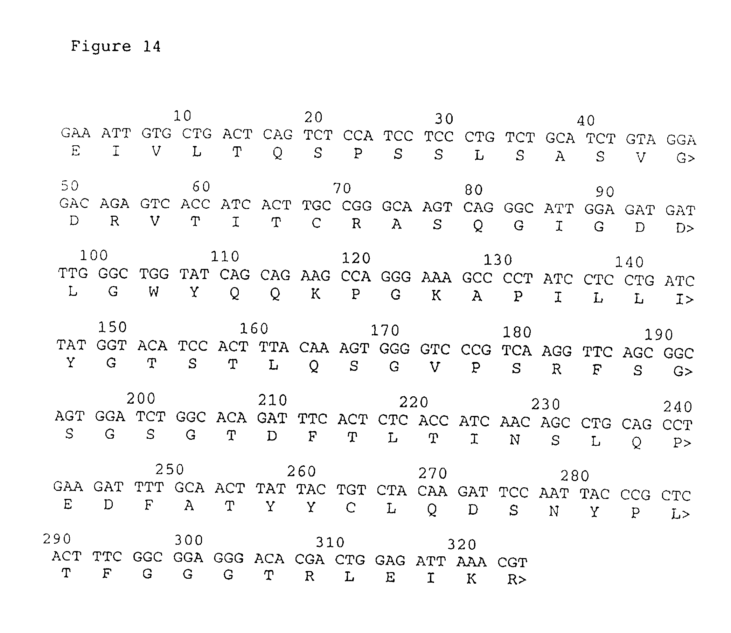

ScFv antibodies specific to TGFβ-1 were identified by phage ELISA and sequenced as described earlier (sections 1d.i and 1e). New TGFβ-1 specific scFv antibodies were identified. Five new high affinity clones were isolated - CS32 which comprises 31G9 VH and 7A3 VL; CS39 which comprises 31G9 VH and 6H1 VL; CS37 which comprises 31G9 VH and 11E6 VL with an Ile for Val substitution at residue 2 (VL sequence given in Figure 14); CS35 which comprises 31G9 heavy chain with substitutions of Glu for Gln at residue 1, Gln for Glu at residue 5 and 14F12 VL; and CS38 which comprises 31G9 VH with substitutions of Thr for Gln at residue 3, Glu for Gln at residue 5, Leu for Phe at residue 27, Ile for Asn at residue 56 and Arg for Gln at residue 105 and 6A5 VL.

### d. Off-rate determination for single chain Fv fragments binding to TGFβ1 and TGFβ2

The off-rates for binding to TGFβ1 or TGFβ2 of the single chain Fv fragments described in this example were determined as described by Karlsson et al (R. Karlsson et al, J. Immunol. Methods 145, 229-240, 1991). The results obtained are shown in Table 2, together with dissociation constants for those which have been determined. These results indicate that high affinity antibodies have been isolated.

### 6. Identification and Characterisation of an Antibody which Cross-reacts with both Human TGFβ-1 and TGFβ-2 but not TGFβ-3 by Selection of a Large ScFv Repertoire

### a. Panning of the Library and Identification of Binders

The large scFv library (described earlier) was induced, phagemid particles rescued and panned as described earlier with the following modifications. For the first round of panning, 10¹² tu library phage in 0.5 ml PBS were used (rather than the standard 2 ml), for the second round, 3.5 x 10⁹ phage in 0.5 ml PBS were used. The immuno tube was coated with 10 µg TGFβ-2 in 0.5 ml PBS for both the first and second round of selection. Individual colonies from the second selection were screened by ELISA using 0.2 µg/ml TGFβ-1. Clones binding TGFβ-1 were further screened on TGFβ-2, TGFβ-3, BSA and PBS. Clones were considered to be specific for both TGFβ-1 and TGFβ-2 if the ELISA signal generated in the TGFβ-1 and the TGFβ-2 coated wells were both at least five-fold greater than the signal on TGFβ-3, BSA and an uncoated well.

### c. Identification of a TGFβ-1/TGFβ-2 Cross-reactive ScFv Antibody

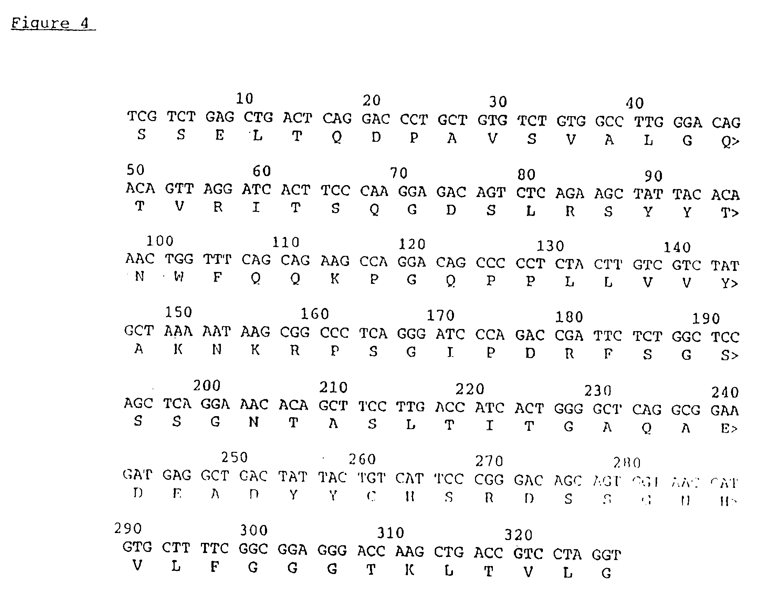

A single scFv antibody specific for both TGFβ-1 and TGFβ-2 was identified by both phage and soluble ELISA, and sequenced, as described earlier. The complete sequence of the VL domain of the antibody gene VT37 is given in figure 4. The dissociation constant of this single chain Fv antibody was estimated by analysis using BIACore™ to be 4nM for TGFβ1 and 7nM for TGFβ2. Cross-reactivity for TGFβ3 was also determined. Purified VT37scFv at 8.3µg/ml was passed over BIACore™ sensor chips coated with TGFβ1 (500RUs coated); TGFβ2 (450RUs coated) or TGFβ3 (5500RUs coated). The relative response for VT37 scFv binding was: TGFβ1 - 391RU bound; TGFβ2 - 261RU bound or TGFβ3 - 24RU bound. Thus this antibody binds strongly to TGFβ1 and TGFβ2 but binding to TGF β 3 is not detectable above background.

### EXAMPLE 2

### Construction of Cell Lines Expressing Whole Antibodies

For the construction of cell lines expressing IgG4 antibodies, variable domains were cloned into vectors expressing the human gamma 4 constant region for the VH domains or the human kappa or lambda constant regions for the VL domains.

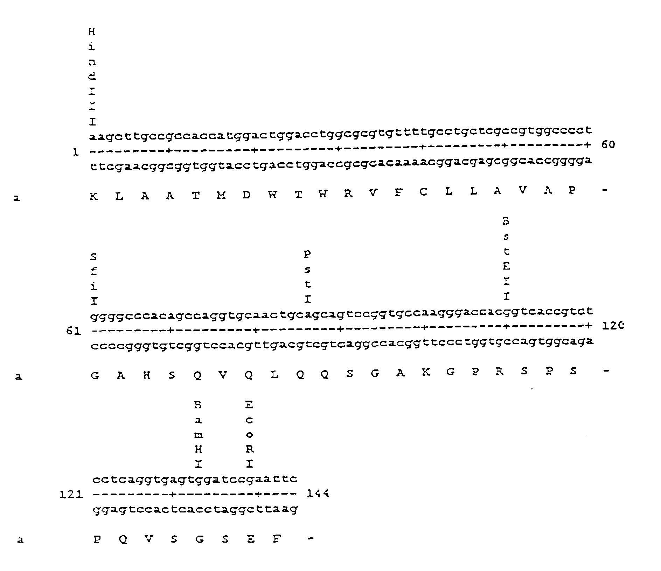

To construct the whole antibody, 27C1/10A6 IgG4 (specific for TGFβ₁), 27C1 VH DNA was prepared from the clone isolated above, in example 1. The VH gene was amplified by PCR using the oligonucleotides VH3BackSfiEu and VHJH6ForBam (Table 1) with cycles of 1 min at 94°C, 1 min at 55°C, 1.5 min at 72°C. Following digestion with SfiI and BamHI, the VH gene was cloned into the vector vhcassette2 (Figure 5) digested with SfiI and BamHI. Ligated DNA was transformed into E. coli TG1. Ampicillin resistant colonies were obtained and those containing the correct insert identified by DNA sequencing.

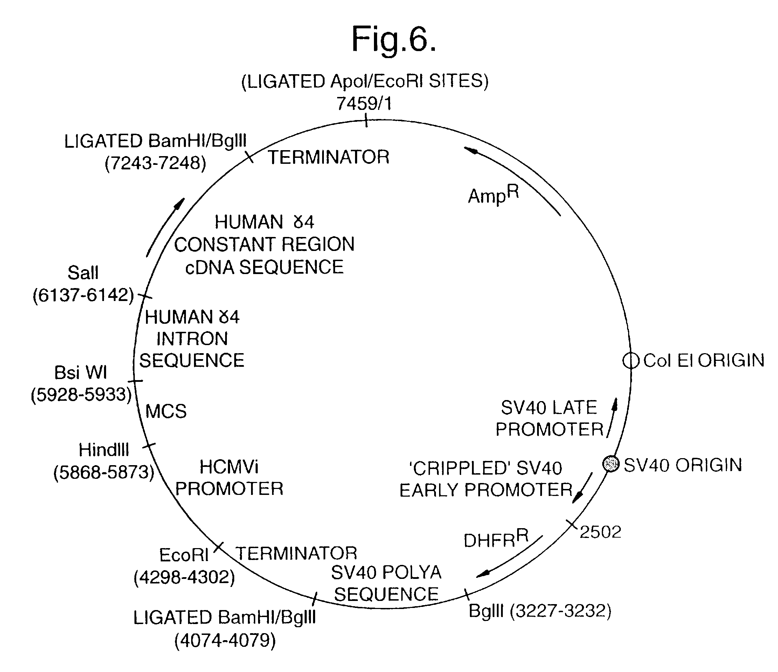

Plasmid DNA from these colonies was prepared and the DNA digested with HindIII and BamHI. The HindIII-BamHI restriction fragment was ligated into the human IgG4 heavy chain expression vector pG4D100 (Figure 6), which had been digested with HindIII and BamHI and the DNA transfected into E.*coli* TG1 by electroporation. The sequence of the VH gene insert was again verified by DNA sequencing.

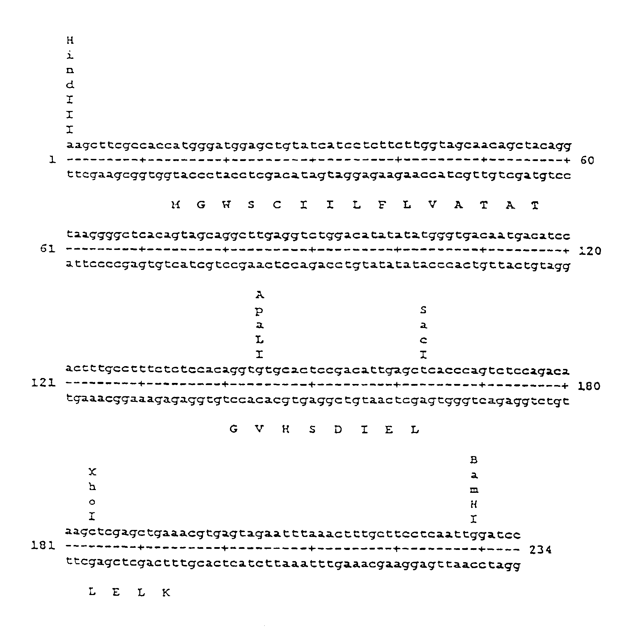

For the light chain, the VL gene of 10A6, isolated in example 1, was first mutagenized to remove its internal BamHI site using site directed mutagenesis (Amersham RPN1523) with the oligonucleotide DeltaBamHI (Table 1). The resulting VLDBamH1 gene was amplified by PCR using the oligonucleotides Vλ3/4BackEuApa and HuJλ2-3ForEuBam (Table 1). Following digestion of the amplified insert with ApaLI and BamHI, the VL gene was cloned into the vector vlcassetteCAT1 (Figure 7) digested with ApaLI and BamHI. Ligated DNA was transformed into E.coli TG1. Ampicillin resistant colonies were obtained and those containing the correct insert were identified by DNA sequencing.

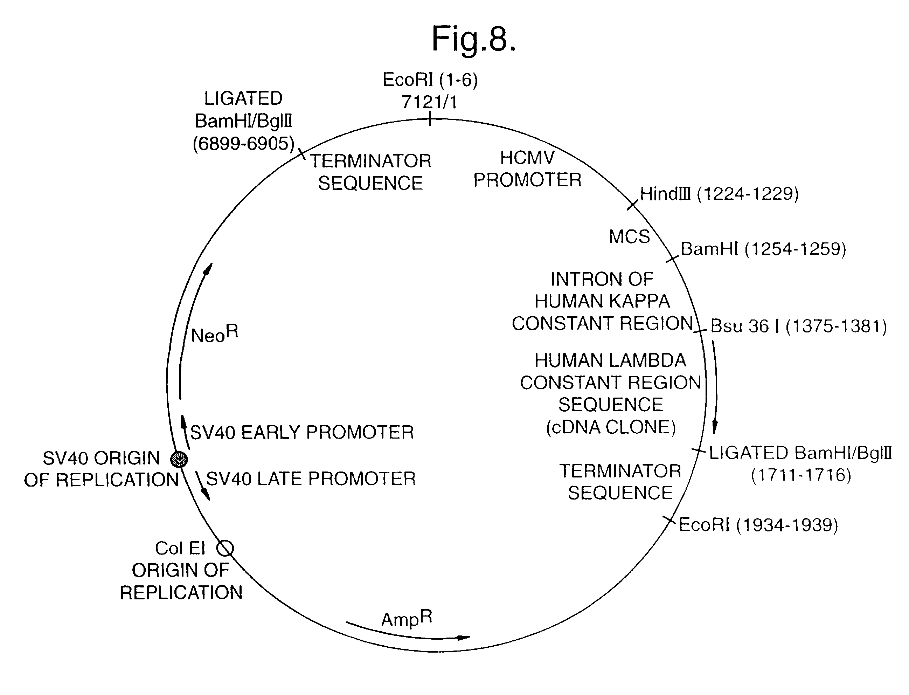

Plasmid DNA from these colonies was prepared and the DNA digested with Hind III and BamHI. The HindIII-BamHI restriction fragment containing the leader sequence and the VL domain was ligated into the human lambda light chain expression vector, pLN10 (Figure 8), which had been digested with HindIII and BamHI. Following electroporation, transformants in E.coli were checked by DNA sequencing.

Plasmid DNA was prepared from the pG4D100-27C1 clone and the pLN10-10A6 clone. This DNA was then co-transfected into DUKXB11 Chinese Hamster Ovary (CHO) cells by electroporation (290V; 960µF). The cells were then grown for 2 days in non-selective medium (alpha-MEM plus nucleosides). Cells were then transferred to a selective medium (alpha-MEM plus 1mg/ml G418 without nucleosides) and grown in 96 well plates. Colonies were then transferred to 24 well plates and samples assayed by sandwich ELISA for assembled human IgG4 antibody and by binding to TGFβ1 in ELISA (as in example 1). For the sandwich ELISA, goat anti-human IgG coated on to the ELISA plate and captured human IgG4 detected using goat antihuman lambda light chain alkaline phosphatase conjugate. High expressing cell lines were then derived by amplification of the inserted genes using selection in the presence of methotrexate (R.J. Kaufman Methods Enzymol. 185 537-566, 1990).

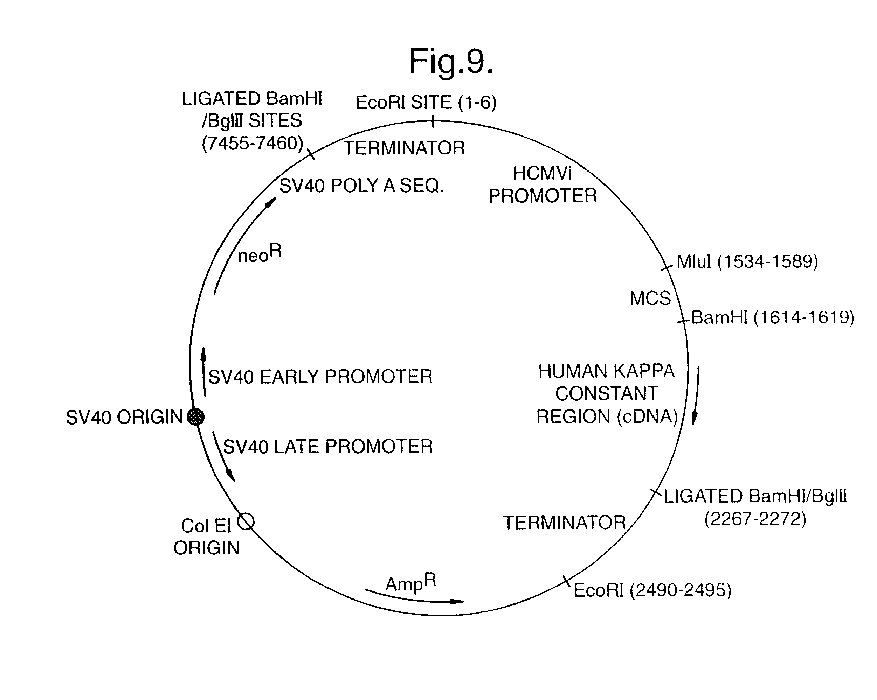

The whole antibody 6H1 IgG4 (specific for TGFβ2) was constructed in a similar way to the above construction of 27C1/10A6 IgG4. The 6H1 VH gene (example 2) was cloned into pG4D100 as for 27C1 above except that PCR amplification was performed with the oligonucleotides VH3BackSfiEu and VHJH1-2FORBam. The 6H1 VL gene (example 2) was subcloned into vlcassetteCAT1 as above except that PCR amplification was performed with the oligonucleotides Vk2BackEuApa and HuJk3FOREuBam. However, since the 6H1 VL is a kappa light chain the HindIII-BamHI fragment was subcloned into the human kappa light chain expression vector pKN100 (Figure 9) which had been digested with HindIII and BamHI. High expressing cell lines were then isolated as described above. Clones expressing antibody were identified from culture plates by sandwich ELISA for assembled human IgG4 antibody (detected using goat anti-human kappa light chain conjugate and by binding to TGFβ2 in ELISA (as in example 2).

To construct the whole antibodies 6A5 IgG4 and 6B1 IgG4, the same 6H1 VH construct in pG4D100 was used as for 6H1IgG4 since these antibodies all have the same VH gene. The 6B1 and 6A5 genes were each subcloned into vlcassetteCAT1 as above for the 10A6 light chain except that PCR amplification was performed with the nucleotides Vλ3backEuApa and HuJλ2-3ForEuBam. The HindIII-BamHI restriction fragment was then subcloned into pLN10 as above. Clones expressing antibody were identified from culture plates by sandwich ELISA for assembled human IgG4 antibody (detected using goat anti-human kappa light chain conjugate and by binding to TGFβ2 in ELISA (as in example 2).

### Properties of whole antibody constructs

### Purification of whole antibodies

Serum-free supernatant from CHO cells producing the relevant IgG was clarified by centrifugation at 8000 rpm (Beckman JS2-21) prior to purification. The supernatant was applied to a HiTrap Protein A Sepharose prepacked affinity column from Pharmacia, either 1 or 5ml size, with binding capacities of 25 or 120 mg respectively. Each IgG had a dedicated column to avoid any potential carry over of material from one purification to another. The column was equilibrated to phosphate buffered saline (PBS) with ten column volumes of 1xPBS prior to applying the supernatant. When all the supernatant had been applied to the column at a flow rate of 2-4 ml/minute, again, depending on the column size, the column was washed with ten column volumes of 1xPBS to remove any non-specifically bound material. Elution of the bound protein was achieved using 0.1M sodium acetate, adjusted to pH 3.3 with glacial acetic acid. The eluted material was collected in 8 fractions of 1.5 ml volume, and the amount of protein determined by measuring the absorbance at 280nm, and multiplying this value by 0.7 to get a value in mg/ml. This was then neutralised with 0.5ml of 1M Tris.HCl pH 9.0 per 1.5ml fraction, and the protein-containing fractions pooled and dialysed against 1x PBS to buffer exchange the IgG. The column was returned to neutral pH by running ten column volumes of 1xPBS through, and was stored in 20% ethanol as a preservative until required again.

A sample was then run on 10-15% SDS-PAGE (Phast system, Pharmacia) and silver stained. this allowed an assessment of the purity of the IgG preparation. This was usually found to be about 80-90%, with only a couple of other bands prominent on the stained gel.

### Binding specificity by ELISA

The IgG4 antibodies 6B1 and 6A5 were shown to bind TGFβ2 with very low cross-reactivity to TGFβ1 and TGFβ3 and no detectable cross-reactivity with a range of non-specific antigens: interleukin-1; human lymphotoxin (TNFb); human insulin; human serum albumin; single stranded DNA; oxazolone-bovine serum albumin; keyhole limpet haemocyanin; chicken egg white trypsin inhibitor; chymotrypsinogen; cytochrome c; glyceraldehyde phosphate dehydrogenase; ovalbumin; hen egg lysozyme; bovine serum albumin and tumour necrosis factor a - (TNF*a*) (Figure 13(a) and (b)). Likewise the antibodies 6B1, 6A5 and 6H1 IgG4 bound strongly to TGFβ2 coated on a BIACore™ sensor chip but not significantly to TGFβ1 or TGFβ3 coated chips.

### Binding properties of whole antibodies by BIACore™

The affinity constants of the above antibodies were determined by BIACore™, using the method of Karlsson et al. J. Immunol. Methods 145, 299-240, 1991 (supra) and found to be approximately 5nM for 27C1/10A6 IgG4 for TGFβ1 and 2nM for 6H1 IgG4 for TGFβ2. The antibody 27C1/10A6 IgG4 also shows some cross-reactivity with TGFβ2 coated onto Biosensor chips but the dissociation constant is approximately 10 fold or more higher for TGFβ2 compared to TGFβ1. There was no significant cross-reactivity with lysozyme coated onto a BIACore™ sensor chip.

Neutralisation and inhibition of radioreceptor binding by IgG4 antibodies to TGFβ1 and TGFβ 2 is described in examples 3 and 4.

### EXAMPLE 3

### Neutralisation by Antibodies of the Inhibitory Effect of TGF β1 and TGF β2 on Cell Proliferation

The neutralising activity of the antibodies described in examples 1 and 2 were tested in a modification of a bioassay for TGF β as described by Randall et al (1993) J. Immunol Methods 164, 61-67. This assay is based on the ability of TGF β₁ and TGF β₂ to inhibit the interleukin-5 induced proliferation of the erythroleukaemia cell line, TF1 and being able to reverse this inhibition with specific TGF β antibodies.

### Method

### Cells and maintenance

The human erythroleukaemia cell line TF1 was grown in RPMI 1640 medium supplemented with 5% foetal calf serum, penicillin/streptomycin and 2ng/ml rhGM-CSF in a humidified incubator containing 5% CO₂ at 37°C. Cultures were passaged when they reached a density of 2 X 10⁵/ml and diluted to a density of 5 x 10⁵/ml.

### Cytokines and Antibodies

rhGM-CSF and rhIL-5 were obtained from R&D systems, rhTGF β₂ was obtained AMS Biotechnology. Rabbit anti TGF β₂ antibody was from R&D Systems and Mouse anti-TGF β_{1,2,3} was from Genzyme. Other antibodies against TGF β₂ were as described in examples 1&2.

### Titration of Inhibition of Proliferation by TGF β₂.

Doubling dilutions of TGF β₂ (800pM - 25pM) for the construction of a dose response curve were prepared on a sterile microtitre plate in 100µl of RPMI 1640 medium containing 5% foetal calf serum and antibiotics. All dilutions were performed at least in quadruplicate. Additional wells containing 100µl of the above medium for reagent and cells only controls were also included.

TF1 cells were washed twice in serum free RPMI 1640 medium and resuspended in RPMI 1640 medium supplemented with 5% foetal calf serum, 100U/ml penicillin and 100µg/ml streptomycin and 4ng/ml rhIL-5 at a density of 2.5 x 10⁵/ml. Aliquots of 100µl were added to the previously prepared dilution series and the plate incubated for 48hr. in a humidified incubator containing 5% CO₂ at 37°C.

Cell proliferation was measured colourimetrically by addition of 40µl CellTiter96 substrate (Promega), returning the plate to the incubator for a further 4hr and finally determining the absorbance at 490nm. The percentage inhibition for each concentration of TGF β₂ as compared to cell only wells was then calculated.

### Assay for Neutralisation of TGF β₂ Inhibitory Activity by Anti-TGF β₂ Antibodies

Neutralisation of TGF β₂ was determined by making doubling dilutions in of each purified antibody in 100µl of medium as above. TGF β₂ was added to each antibody dilution to give a final concentration equivalent to that which gave 50% inhibition in the titration described above. Each dilution was prepared in quadruplicate. Additional wells were prepared for antibody only, cells only and reagent controls. Cell preparation and determination of cell proliferation was performed as described above.

### Results

TGF β₂ was shown to inhibit the proliferation of TF1 cells by 50% at a concentration of 50pM. This concentration was used for all neutralisation experiments.

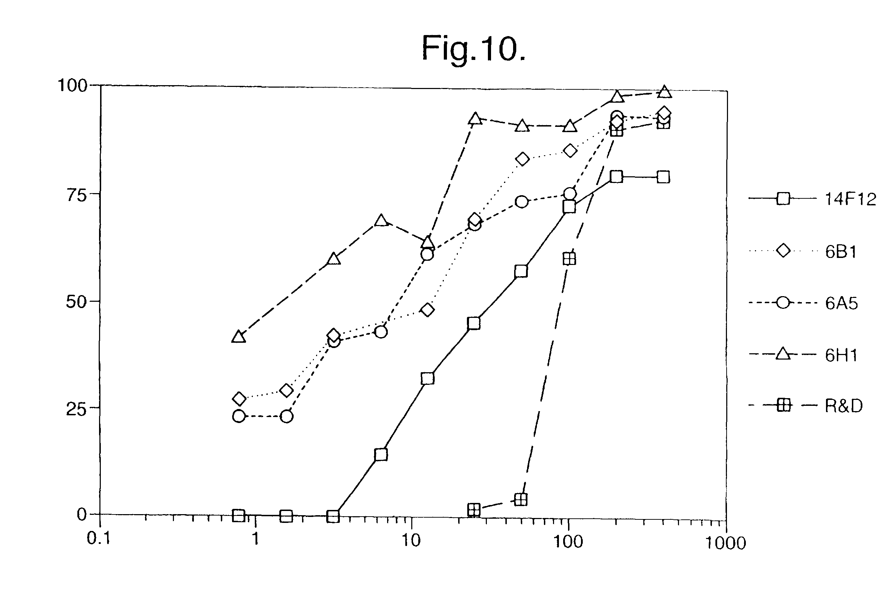

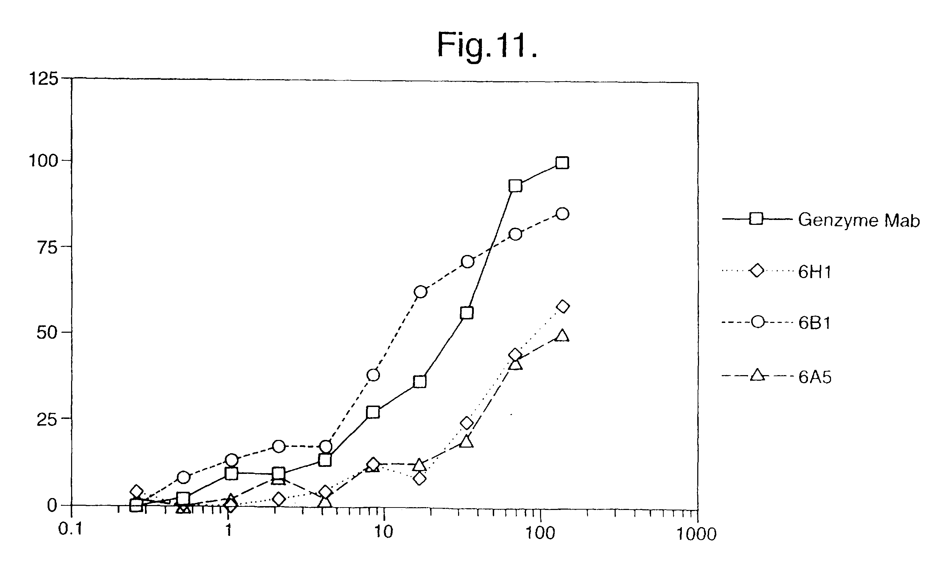

These assays showed that TGF β₂ activity was neutralised in a dose dependant manner for both scFv fragments (figure 10) and for whole IgG4 antibodies (figure 11). The concentration of antibody which gave 50% inhibition was determined from the graphs and is shown in table 4.

### EXAMPLE 4

### Inhibition by Antibodies of TGFβ Binding to Receptors Measured in A Radioreceptor Assay

Single chain Fv fragments and whole IgG4 antibodies from different clones were expressed and purified and their ability to inhibit binding of TGFβ to receptors measured in a radioreceptor assay.

### Purification of scFv

ScFvs containing a poly histidine tail are purified by immobilised metal affinity chromatography. The bacterial clone containing the appropriate plasmid is inoculated into 50 ml 2TY medium containing 2% glucose and 100 µg/ml ampicillin (2TYAG) and grown overnight at 30°C. The next day the culture is added to 500 ml prewarmed 2TYAG and grown at 30°C for 1 h. The cells are collected by centrifugation and added to 500 ml 2TY containing ampicillin and 1 mM IPTG and grown at 30°C for 4 h. The cells are then collected by centrifugation and are resuspended in 30 ml ice-cold 50 mM Tris HCl pH 8.0, 20% (w/v) sucrose, 1 mM EDTA. After 15 min end-to-end mixing at 4°C the mixture is centrifuged at 12 k rpm for 15 min at 4°C. The supernatant is removed and to it added ∼ 1ml NTA-agarose (Qiagen 30210) and mixed at 4°C for 30 min. The agarose beads are washed extensively with 50 mM sodium phosphate, 300 mM NaCl and loaded into a small column. After further washing with 50 mM sodium phosphate, 300 mM NaCl, 10 mM imidazole pH 7.4 scFv is eluted with 50 mM sodium phosphate, 300 mM NaCl, 250 mM imidazole pH 7.4. 0.5 ml fractions are collected and the protein containing fractions identified by measuring the A₂₈₀ₙₘ. Pooled fractions are concentrated and scFv further purified by gel filtration in PBS on a Superdex 75 column (Pharmacia).

### Purification of Whole Antibodies

Whole IgG4 antibodies were purified as described in Example 2.

### Radioreceptor Assay for TGF-β

Neutralisation of TGF-β activity is measured by the ability of the scFvs and IgGs to inhibit the binding of ¹²⁵-I labelled TGF-β to its receptors on A549 human lung carcinoma cells.

A549 cells (ATCC CCL 185) are grown in high glucose Dulbecco's modified Eagle's medium (Sigma D-6546) supplemented with 10% foetal calf serum (PAA), 2 mM glutamine (Sigma G-7513), penicillin/streptomycin (Sigma P-0781), MEM non-essential amino acids (Sigma M-7145).

Cells are seeded at 1-2 x 105 cells / ml / well into the wells of 24-well cluster plates and grown for 24 h in serum-free DMEM. Cell monlayers are washed twice with serum-free DMEM and 0.5 ml binding medium (DMEM/Hams F12 (Sigma D-6421) containing 0.1% (v/v) BSA added to each well.

Aliqouts of ¹²⁵I-TGF-β1 or -β2 (70-90 TBq/mmol; Amersham International) at 20 pM are preincubated with antibody in binding medium at room temperature for 1 h. Duplicate samples of 0.5 ml of TGF-β/antibody mixtures are then added to the cell monlayers and are incubated at 37°C for 1-2 h. Control wells contain TGF-β only. Unbound TGF-β is removed by washing 4 times with Hank's balanced salt solution containing 0.1% BSA. Cells are solubilised in 0.8 ml 25 mM Tris HCl pH 7.5, 10 % glycerol, 1 % Triton X-100 at room temperature for 20 min. The contents of each well are removed and ¹²⁵I measured in a gamma counter. The potency of each scFv or IgG is measured by the concentration of antibody combining sites necessary to inhibit binding of TGF-β by 50% (IC50; Table 5). Thus the IC50 values are below 10nM and in some cases below 1nM indicating very potent antibodies.

### EXAMPLE 5

### Prevention of Scar Formation by Antibodies Against TGF β1 and TGF β2 in the Injured Central Nervous System of the Rat

Logan *et al* (1994) Eur.3 Neuroscience 6,355-363 showed in a rat model of CNS injury, the ameliorating effect of a neutralising turkey antiserum directed against TGF β₁ on the deposition of fibrous scar tissue and the formation of a limiting glial membrane that borders the lesion. A study was set up to investigate the effects of neutralising engineered human antibodies directed against both TGF β₁ and TGF β₂ in the same rat model. The derivation of the antibodies used in this study is described in examples 1 and 2.

### Method

### Animals and surgery

Groups of five female Sprague-Dawley rats (250g) were anaesthetised with an i.p. injection. The anaesthetised rats had a stereotactically defined lesion made into the right occipital cortex (Logan *et al* 1992 Brain Res. 587, P216-227) and the lateral ventricle was surgically cannulated and exteriorised at the same time (Logan *et al* 1994 supra).

### Neutralisation of TGF β

Animals were intraventricularly injected daily with 5ul of purified anti TGF β antibodies (Table 3) diluted in a vehicle of artificial cerebrospinal fluid as described by Logan *et al* 1994 supra. Fourteen days post lesion all animals were perfusion fixed and 7mm polyester wax sections were processed for histochemical evaluation of the lesion site by immunofluorescent staining.

### Fluorescent immunohistochemistry and image analysis

Morphological changes within the wound site were followed by immunofluorescent staining with antibodies to fibronectin and laminin detected with anti-species FITC conjugates (Logan *et al* 1994 supra). These changes were semi-quantitatively assessed by image analysis using a Leitz confocal microscope linked to a Biorad MRC500 laser scanning system. Readings were taken at standard positions mid-way along the lesion.

### Results

### Effects of antibodies to TGF β at the site of CNS injury

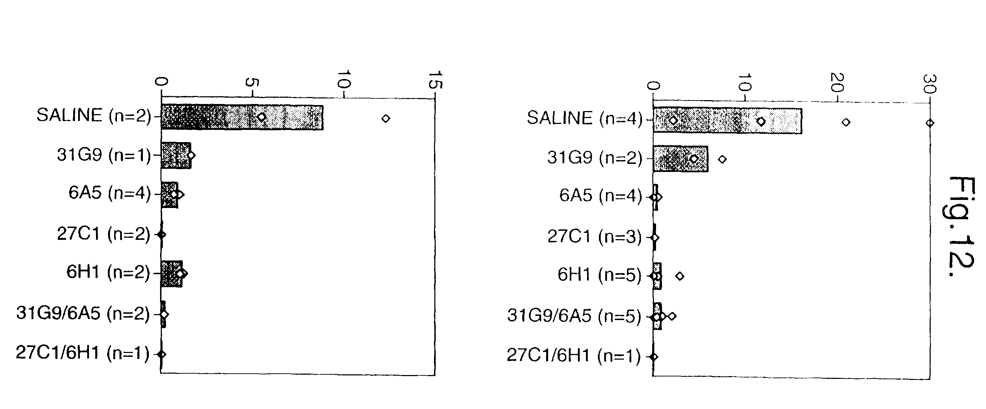

Quantitation of the specific relative fluorescence for each of the antibodies is shown in figure 12 a and b. Laminin is a measure of the formation of the glial limitans externa along the boundaries of the wound and together with fibronectin forms a matrix of fibrous tissue within the centre of the wound. Quantitation by image analysis of these two proteins allows the degree of scarring at the wound site to be determined.

Compared with the saline control (fig.12 a,b), There is a considerable decrease in fibronectin and laminin immuno-localisation in the wound in the anti-TGF β antibody treated brains. Thus this indicates that these engineered human antibodies directed against epitopes on TGF β₁ & TGF β₂ ameliorate the effects of injury to the CNS both separately and together. by preventing the deposition of the cellular matrix proteins fibronectin and laminin within the wound site. Previously Logan *et al* (1994 supra) had shown the effectiveness of a polyclonal turkey anti-sera directed against TGF β₁. This is the first report of any antibodies directed against TGF β₂ having been shown to be effective in this model.

### EXAMPLE 6

### Determination of Binding of 6B1 IgG4 to Active or Latent Form of TGF β₂

TGFβ₂ is synthesised and secreted exclusively as a biologically inactive or latent complex (Pircher *et al,* (1986) Biochem. Biophys Res. Commun. 158, 30-37). The latent complex consists of TGFβ₂ disulphide linked homodimer non-covalently associated with latency-associated peptide (LAP). Activation of TGFβ₂ occurs when it is released from it processed precursor. Active TGFβ₂ is capable of reversibly dissociating and reassociating with the LAP, which results in the turning on and off of its bio-activity respectively.

Cultured PC-3 adenocarcinoma cells (Ikeda *et al* (1987) Biochemistry 26, 2406-2410) have been shown to secrete almost exclusively latent TGFβ₂ providing a convenient source for determination of binding to the active or latent form of TGFβ₂ by the antibody 6B1 IgG4.

### Method

### Cell Culture

PC-3 prostatic adenocarcinoma cells were grown to confluence in supplemented with 10% FBS. The cells were washed 3x with PBS and cells cultured for a further 7 days in serum free Hams F12/DMEM supplemented with 1.4 x 10⁻⁵M tamoxifen (Brown *et al,* (1990) Growth Factors 3, 35-43). The medium was removed, clarified by centrifugation and divided into two 15ml aliquots. One aliquot was acidified for 15 min with 5M HCl by adding dropwise until the pH = 3.5 and then neutralised by the similar addition of 5M NaOH/lM HEPES pH7.4. This procedure activates the latent TGFβ2 quantitatively.

### Competition ELISA

Sixteen wells of an ELISA plate were coated overnight with 100µl 200ng/ml TGFβ₂ in PBS at 4°C. The plate was washed 3x with PBS tween and blocked at 37°C with 200µl of 3% Marvel in PBS.

The following samples were incubated at room temperature for 1 hour.
400µl Hams F12/DMEM (reagent blank)
400µl Hams F12/DMEM plus 4µg 6B1 IgG4 antibody (positive control)
400µl PC 3 acid activated conditioned media plus 4µg 6B1 IgG4 antibody (active TGFβ₂ sample)
400µl PC 3 untreated conditioned media plus 4µg 6B1 IgG4 antibody (latent TGFβ₂ sample)

The ELISA plate was emptied of blocking solution and 100µl of one of the above solutions added to sensitised wells in quadruplicate and incubated at room temperature for 2 hours. The plate was washed 3x with PBS/Tween and wells refilled with 100µl of goat anti-human IgG γ chain alkaline phosphatase conjugate diluted 1:5000 in 1% Marvel/PBS. After 1 hour the wells were washed 3x with PBS/Tween and bound antibody was revealed with *p*-NPP substrate by absorbance at 405 nm.

### Results

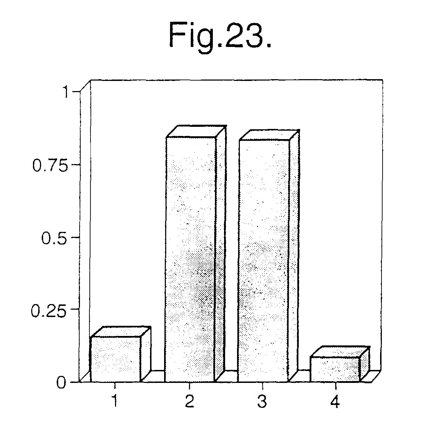

The results of this experiment are shown in Figure 23.

This result clearly shows that pre-incubation with activated TGFβ2 inhibits binding of 6B1 to TGFβ2 bound onto an ELISA plate, whereas the latent form does not. This proves that 6B1 IgG4 only binds to the active form of TGFβ2.

### EXAMPLE 7

### Neutralisation by antibodies directed against TGFβ2 of the inhibitory effect of TGFβ isoforms on cell proliferation

The neutralising activity of 6B1 IgG4, 6H1 IgG4 (purified as in example 2) and a mouse monoclonal antibody (Genzyme; J.R. Dasch et al., supra) was measured for each of the TGFβ isoforms, TGFβ1, TGFβ2 and TGFβ3 in the TF1 cell proliferation assay described in Example 3. The concentration of TGFβ isoform was 100pM in each assay.

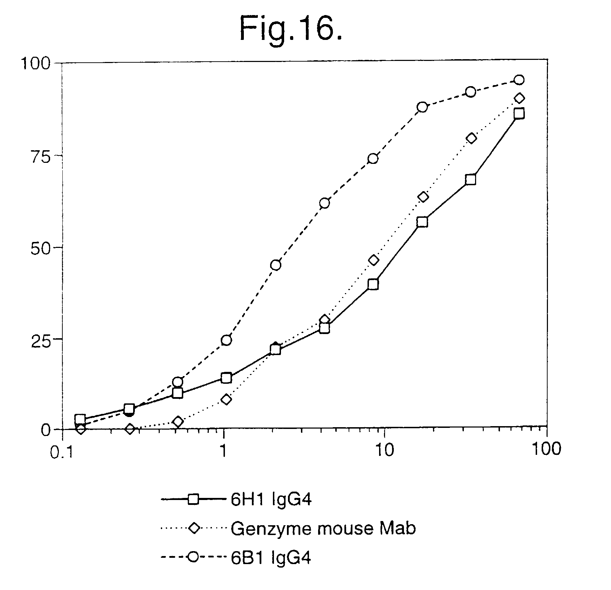

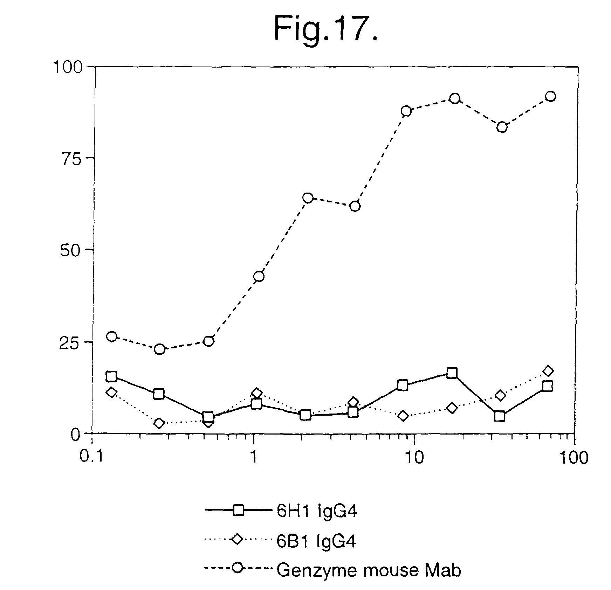

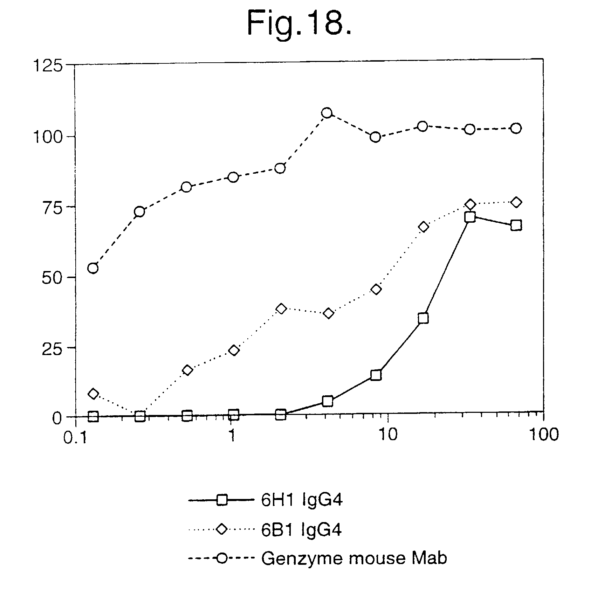

As shown in Figure 16, 6B1 IgG4 strongly neutralises TGFβ2 with an IC₅₀ of approximately 2nM (Table 6). This compares to 10nM for the mouse monoclonal from Genzyme and 12nM for 6H1 IgG4. Neither 6B1 IgG4 nor 6H1 IgG4 significantly neutralise TGFβ1 (Fig. 17). However, there is significant neutralisation of TGFβ3 by both 6B1 (IC₅₀ ca. 11nM) and 6H1 IgG4 ca. 20nM; Fig. 18). This is considerably less than the neutralisation potency of the Genzyme monoclonal (IC₅₀ ca. 0.1nM).

Both 6B1 IgG4 and 6H1 IgG4 are stronger neutralisers of TGFβ2 activity than of TGFgβ3 activity. The neutralisation of TGFβ3 activity is greater than would be predicted from the relative binding of these two isoforms by the antibodies (example 2) and the relative binding in a radioreceptor assay (example 8).

### EXAMPLE 8

### Inhibition by antibodies directed against TGFβ2 of binding of other TGFβ isoforms to receptors measured in a radioreceptor assay

The ability of 6B1 IgG4 to inhibit binding of TGFβ isoforms to receptors was measured in a radioreceptor assay as described in example 4.

6B1 IgG4 inhibited binding of ¹²⁵I-TGFβ2 with an IC₅₀ of 0.05nM. There was no significant inhibition of binding of ¹²⁵I-TGFβ1 whereas for ¹²⁵I-TGFβ3 6B1 IgG4 inhibited binding with an IC₅₀ of approximately 4nM (Table 6). This indicates the potency of 6B1 IgG4 in this assay and its selectivity for the neutralisation of TGFβ2 activity. Cross-reactivity with TGFβ3 in this assay is less than 2%.

Thus 6B1 IgG4 preferentially inhibits the binding of TGFβ2 to its receptors compared with binding of TGFβ3.

### EXAMPLE 9

### Assessment of TGFβ1 Antibodies for Therapeutic Use

The antibodies isolated in Example 1 were assessed for potential therapeutic value by *in vitro* measurements of the ability to inhibit TGFβ1 binding to its receptors and *in vitro* binding properties.

In Example 4 (Table 5) CS32 showed the strongest inhibition of the antibodies tested of the binding of ¹²⁵I-TGFβ1 to receptors on A549 cells. A further comparison was performed between CS32 and further antibodies (CS35, CS37 and CS38) that were isolated as described in the experiment in Example 1, section 5c. This showed that CS37 appeared to be the most potent of these antibodies in this assay with an IC₅₀ of approximately 8nM, compared with 40nM for CS32. The IC50 value for CS32 is higher than in the previous assay (Table 5) because the nature of the assay means that the absolute IC₅₀ value can vary with assay conditions.

The antibodies 1A-E5 and 1AH-6 (Example1, section 1f) and antibodies derived from them were much less potent than antibodies derived from 1B2 in neutralising TGFβ activity in this radioreceptor assay.

Thus CS37 was the most potent antibody candidate as assessed by inhibition of binding of ¹²⁵I-TGFβ1 to its receptor.

### Assessment of binding to TGFβ3 by anti-TGFβ1 antibodies

The antibodies 14A1 and 10A6 (Example 1, section 2 (a) (iii)) were shown to preferentially bind TGFβ1 over TGFβ2 and TGFβ3 using the same specificity ELISA as was described in Example 1, section 1 (d) (iii), except that microtitre plates were coated with 50µl of either 0.2µg/ml TGFβ1; 0.2µg/ml TGFβ2; 0.2 µg/ml TGFβ3; 10µg/ml bovine serum albumin (BSA) or PBS (the uncoated well). The clones were shown to be specific for TGFβ1 since the signal generated in the TGFβ1 coated well was at least five fold greater than the signal on TGFβ2 and TGFβ3.

Antibodies derived from the same 1B2 lineage as these antibodies, such as 27C1/10A6 IgG4 (which contains the same VL as 10A6 and the 27C1 VH was prepared by mutagenesis of CDR3 residues) should have the same cross-reactivity against TGFβ3.

### EXAMPLE 10

### Construction of a High Expressing Cell Line for 6B1 IgG4 using the Glutamine Synthase Selection Systemand Assessment in a Neutralisation Assay

### Construction of p6H1 VH gamma4

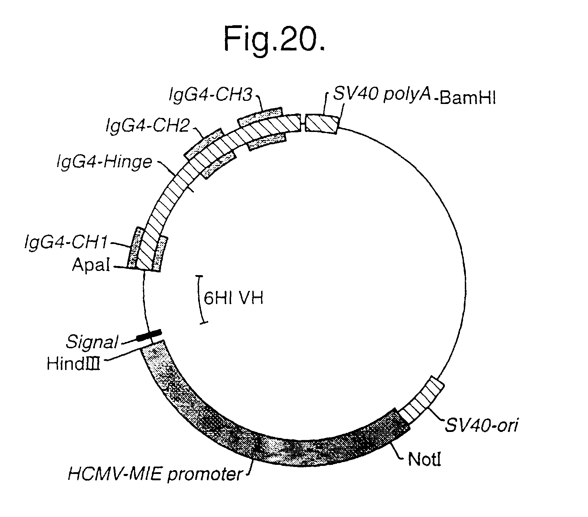

6B1 VH was amplified from 6H1 pG4D100 (Example 2) by PCR using oligonucleotides P16 and P17. This DNA was joined by PCR with a 158bp DNA fragment from M13VHPCR1 (R. Orlandi et al Proc. Natl. Acad. Sci. USA 86 3833-3837, 1989) containing a signal sequence, splice sites and an intron, using oligonucleotides P10 and P17. The PCR product was cut with HindIII ad ApaI and cloned into HindIII-Apal cut pGamma4 (Lonza Biologics plc). A plasmid with the correct insertion was identified and designated p6Hl VH gamma4 (see Figure 20). The VH gene and flanking regions were sequenced at this stage.

### Construction of 6B1ΔBam pLN10

The VL gene of 6B1 was amplified from the clone of 6B1 scFv in pCANTAB6 (Example 1) and subcloned into pUC119. The VL gene was then mutated by in vitro mutagenesis to remove an internal BamHI site, modifying the DNA sequence but not the protein sequence. In vitro mutagenesis was performed using the oligonucleotide LamDeltaBamHI (Table 1) using a kit from Amersham International plc. The mutated VL gene was amplified using the primers Vλ3backEuApa and HuJλ2-3ForEuBam and subcloned as an ApaLI-BamHI fragment into the vector vlcassetteCAT1. The VL gene was then cloned as a HindIII-BamHI fragment into the vector pLN10 (Figure 8) to generate the vector 6B1ΔBam pLN10.

### Construction of p6B1λ

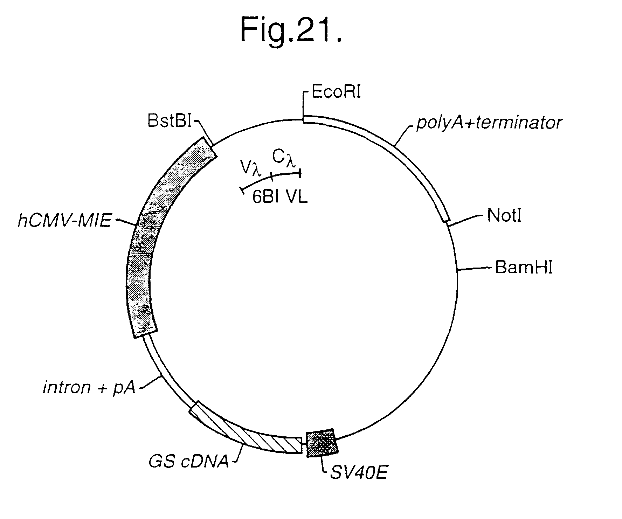

The 6B1 Vλ gene was amplified by PCR from p6B1ΔBampLN10 using oligonucleotides P22 and P26. The Cλ gene was amplified by PCR from pLN10-10A6 (Example 2) using oligonucleotides P25 and P19. The 2 DNAs were joined by overlapping PCR using the oligonucleotides P22 and P19 and the product cut with BstBI and EcoRI and cloned into BstBI-EcoRI cut pMR15.1 (Lonza Biologics plc). A plasmid with the correct insertion was identified and designated p6B1λ (Figure 21).

### Construction of final expression vector p6B1gamma4gs

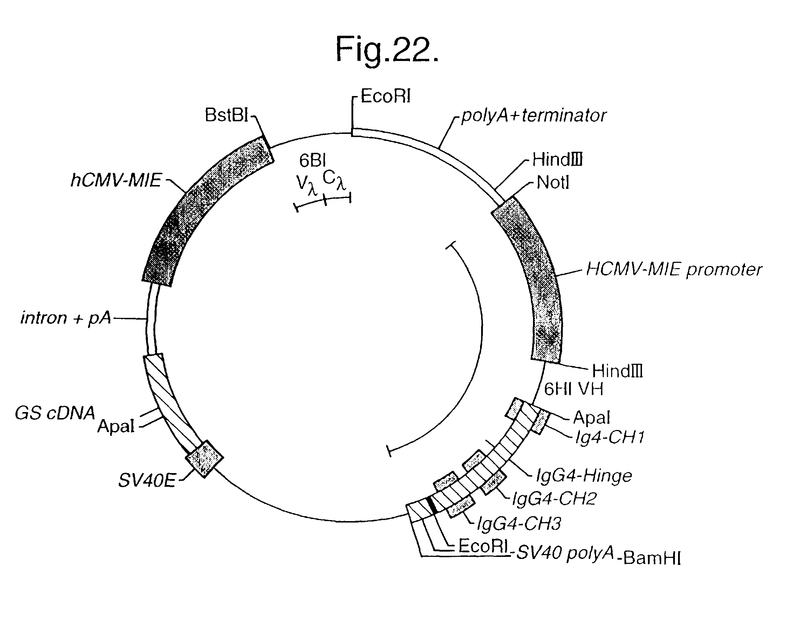

p6H1 VHgamma4 and p6B1λ were digested with BamHI and NotI, fragments were purified and ligated together. A plasmid of the desired configuration was identified from transformants and designated p6B1gamma4gs (Figure 22).

### Transfection of NS0 with p6B1 gamma4gs

Stable transfectants secreting 6B1 IgG4 were selected by introducing into NS0 myeloma cells p6B1 which includes the glutamine synthetase (gs) gene which allows growth in glutamine-free (G-) medium (C.R. Bebbington et al Bio/Technology 10 169-175, 1992). 40µg p6B1 gamma4gs were linearised by digestion with PvuI. The DNA was electroporated into 1.5 x 10⁷ NS0 cells. Cells were then added to G+DMEM/10% FCS and 50µl aliquots distributed into 6 x 96-well plates and allowed to recover for 24h. The medium was then made selective by the addition of 150µl G-DMEM/10%FCS. Three weeks later gs⁺ transfectants were screened by ELISA for the ability to secrete human IgG4λ antibody. The highest producers were expanded and further analysed. From this analysis 5D8 was selected as the candidate production cell line. 5D8 was cloned once by limiting dilution to give the cell line 5D8-2A6.

### Assessment of 6B1 IgG4 derived from cell line 5D8-2A6 in the TF1 neutralisation assay

6B1 IgG4 was purified from the GS/NS0 cell line 5D8-2A6 grown in serum-free medium as described in Example 2. The 6B1 IgG4 antibody was assayed in the TF1 neutralisation assay as described in Example 3. An IC₅₀ value of 1.8nM was obtained in this assay. Subsequent assays of preparations of 6B1 IgG4 derived from the 5D8-2A6 cell line have indicated values of IC₅₀ in the range of 0.65 to 2nM. These are comparable to the values obtained for 6B1 IgG4 produced from CHO cells (Example 2) and compare favourably with that obtained for 6H1 IgG4 derived from a CHO cell line (IC₅₀ of 15nM). The values obtained for the IC₅₀ for 6B1 IgG4 and 6H1 IgG4 in this example are more reliable than those obtained in Example 3 and are shown in Table 4, because of improvements in the assay and in the expression and purification of the antibodies. The IC₅₀ value may however be expected to vary with the precise conditions of the assay.

Thus the 6B1 IgG4 provides potent neutralisation of TGFβ2 and is suitable for use as a therapeutic.

### EXAMPLE 11

### Determination of the Epitope on TGFβ2 for the Antibody 6B1 using a Peptide Phage Display Library

The antibody 6B1 was further characterised by epitope mapping. This was done by using a peptide phage display library to select peptide sequences that bind specifically to 6B1. These peptide sequences were then compared to the amino acid sequence of TGFβ2. Correlation between peptide sequences that bind to 6B1 and matching parts of the TGFβ2 amino acid sequence indicate an epitope of TGFβ2 to which 6B1 binds. An "epitope" is that part of the surface of an antigen to which a specific antibody binds.

In this example, the peptide library used was constructed as described by Fisch et al (I. Fisch et al (1996) Proc. Natl. Acad. Sci USA 93 7761-7766) to give a phage display library of 1 x 10¹³ independent clones. Phage displaying peptides that bind to the antibody 6B1 were selected from this library by panning. This was performed as described in Example 1.

Purified 6B1 IgG4 antibody at 10µg/ml in 4ml of PBS was coated onto a plastic tube (Nunc; maxisorp) by incubating overnight at 4°C. After washing and blocking with MPBS (see Example 1) an aliquot of the peptide library containing 5 x 10¹³ phage in 4ml 3%MPBS was added to the tube and incubated at room temperature for 1.5 hours. The tube was washed 10 times with PBST(0.1%), then 10 times with PBS. Bound phage particles were eluted from the tube by adding 4ml of 100mM triethylamine and incubating the tube stationary for 10 minutes at room temperature. The eluted phage were then added to a tube containing 2ml 1M-Tris.HCl (pH7.4) and 10ml 2YT broth. The phage were then added to 20ml of logarithmically growing E. coli TG1 cells and grown for 1 hour shaking at 100rpm at 37°C. The infected cells were then plated on 2YT agar medium with 15µg/ml tetracycline in 243mm x 243mm dishes (Nunc). Plates were incubated at 30°C for 18 hours. Colonies were scraped off the plates into 10 ml 2TY broth containing 15% (v/v) glycerol for storage at -70°C.

250µl of cells from the first round of selection was used to inoculate 500ml 2YT broth (containing 15µg/ml tetracycline) in a 2 litre conical flask and grown overnight, at 30°C with shaking at 280rpm. A 2ml aliquot of this culture was then taken and centrifuged to remove all cells. 1ml of this phage supernatant was the used for a second round of selection as described above. The pattern of phage growth and panning was repeated over a third and a fourth round of selection.

Individual colonies from the fourth round of selection were used to inoculate 100µl 2YT broth (containing 15µg/ml tetracycline) into individual wells of 96 well tissue culture plates and grown overnight with gentle shaking at 100rpm at 30°C. Glycerol was added to a final concentration of 15% (v/v) and these master plates were stored frozen at -70°C.

These clones were screened for clones that bound specifically to the antibody 6B1 in ELISA. Cells from the master plates were used to inoculate 96 well tissue culture plates containing 100µl 2YT broth (containing 15µg/ml tetracycline) per well and grown overnight with gentle shaking at 100rpm at 30°C. The plates were then centrifuged at 2000rpm. The 100µl phage supernatants from each well were recovered and each was mixed with 100µl of 4% skimmed milk powder in 2x PBS. 100µl of each of these was then assayed by phage ELISA. Purified 6B1 IgG4 antibody at 10µg/ml in PBS was coated onto flexible microtitre plates by incubating overnight at 4°C. Control plates coated with an irrelevant IgG4 antibody at 10µg/ml were also prepared. The ELISAs were performed as described in Example 1, and visualised with the chromagenic substrate pNPP (Sigma).

Approximately 20% of all the clones analysed bound to the 6B1 coated plate. None of the clones analysed bound to ELISA plates coated with the irrelevant antibody. Binding therefore appeared to be specific for the binding site of the antibody 6B1.

Clones which bound 6B1 were analysed by DNA sequencing as described by Fisch et al. A total of 31 different clones were sequenced. These were analysed for possible matches with the sequence of TGFβ2 using Mac vector software. Of these clones, 12 showed poor matching with the sequence of TGFβ2 and 10 had no similarity at all. However, there were 4 different clones (some of which had been selected more than once) which showed a reasonable match to a region of the TGFβ2 sequence between amino acid positions 56 to 69. Table 8 shows the amino acid sequence of the exon of each of these clones that appears to be responsible for binding to 6B1.

None of these clones exactly match the sequence of TGFβ2 nor is there a single clear consensus sequence between the peptide clones. Nevertheless, careful examination of the sequences reveals a match with residues 60 to 64 of TGFβ2 (Table 8). Lining up four clones with L at position 64 reveals 2 clones with R at position 60, 1 clone with V at position 61, 2 with L at position 62 and 3 with S at position 63. This provides the sequence RVLSL corresponding to residues 60 to 64 which form part of the alpha helix which forms the heel region of TGFβ2. An antibody recognising this structure would not be expected to make contact with every amino acid residue in the helix and so a peptide mimicking this sequence could have considerable sequence variation at positions that correspond to parts of the helix that do not make contact. The alpha helix recognised is believed to form part of the receptor binding region of TGFβ2 (D.L. Griffith et al.(1996) Proc. Natl. Acad. Sci. USA 93 878-883).

### EXAMPLE 12

### Determination by Immunohistochemistry of Binding of 6B1 IgG4 to TGFβ2 in Mammalian Tissue and Absence of Cross Reactivity

To detect TGFβ2 in formalin-fixed tissue sections that express the cytokine, the tissue section is generally treated with a protease, pronase E. This digestion step unmasks the antigen, possibly activating latent TGF β2 to give active TGF β2. 6B1 IgG4 detects only the active form of TGF β2 (Example 6).

Using 6B1 IgG4 and immunohistochemical methods the distribution of TGF β2 was determined in formalin fixed-paraffin wax embedded rat normal rat kidney, and experimentally lesioned rat brain tissue, following pronase E digestion.

The reactivity of 6B1 IgG4 in frozen cryostat sections of acetone post-fixed normal human tissue was also ascertained to determine whether there was any binding to other antigens in these tissues.

### Method

### Rat Tissue

Paraffin embedded rat tissues were de-waxed and rehydrated through an alcohol series. The sections were then treated with 0.1% pronase E for exactly 8 min and then washed in water. TGF β2 was detected in the sections using 6B1 IgG4 at 500ng/ml following the protocol provided with a Vectastain ABC (avidin-biotin-complex) kit from Vector Laboratories. On kidney sections, bound antibody was located with alkaline phosphatase and peroxidase was used on rat brain tissues.

### Human Tissue

The following human tissue samples were used: Adrenal, Aorta, Blood, Large intestine, Small intestine, Cerebrum, Kidney, Lymph Node, Liver, Lung, Spleen, Pancreas, Skeletal muscle, Cardiac Muscle, Thyroid, Nerve, Skin, Eye.

Cryostat sections and smears were fixed for 15 minutes in acetone before application of 6B1 IgG4 antibody labelled with FITC using Sigma Immunoprobe kit. The labelled antibody was incubated for 18hr at 4°C, then detected using an indirect alkaline phosphatase method (detection with anti-FITC antibody followed with anti-species enzyme conjugated antibody). In instances where endogenous alkaline phosphatase activity could not be suppressed a peroxidase detection method was used. No pronase digestion was used in this case, therefore this procedure would detect only antigens with which the antibody cross-reacts.

### Results

### Rat Tissue

Rat kidneys displayed positive staining in tubules present on both the apical and the basolateral side, demonstrating the presence of TGF β2 in the tissues.

Injured rat brain at 5 days post injury showed positive staining of neurones, astrocytes and macrophages which was absent in normal brain. This indicates that the TGF β2 is expressed in rat brain following injury.

### Human Tissue

No specific staining of any tissue was observed using fixed cryostat sections of the tissues listed above. Therefore 6B1 IgG4 does not cross-react with antigens in these tissues and when used therapeutically will bind only active TGF β2 in tissue sections detected by immunohistochemical methods.

### EXAMPLE 13

### Kinetic analysis of the binding of 6B1 single chain Fv and 6B1 IgG4 to TGFβ isoforms

Surface plasmon resonance (SPR) can be used to examine real-time interactions between an immobilised ligand and an analyte, and derive kinetic constants from this data. This was performed using the BIAcore 2000 system (Pharmacia Biosensor) with the antigen immobilised on a surface, and the antibody as analyte.

The system utilises the optical properties of surface plasmon resonance to detect alterations in protein concentration within a dextran matrix. Antigen is covalently bound to the dextran matrix at a set amount, and as solution containing antibody passes over the surface to which this is attached, antibody binds to the antigen, and there is a detectable change in the local protein concentration, and therefore an increase in the SPR signal. When the surface is washed with buffer, antibody dissociates from the antigen and there is then a reduction in the SPR signal, so the rate of association, and dissociation, and the amount of antibody bound to the antigen at a given time can all be measured. The changes in SPR signal are recorded as resonance units (RU), and are displayed with respect to time along the y-axis of a sensorgram.

The density of immobilised ligand on the surface of a BIACore chip is important when deriving kinetic data from the sensorgrams generated. It needs to be quite low, so that only a small amount of analyte antibody is needed for saturation of the chip surface. For simplicity, the density of a chip surface is quoted in RU's, and an ideal amount for a ligand such as TGFβ2 or TGFβ3 (25kDa) is 400-600 RU's relative to the baseline set during the immobilisation of the ligand to the surface. The actual amount of TGFβ that has to be added to get the correct density has to be determined by investigation, but is reproducible once the correct concentration has been found.

Immobilisation of the ligand to the dextran matrix of the chip surface is facilitated via amine groups, on lysine side chains in the protein, and carboxyl groups in the dextran matrix. The carboxyl groups in the dextran are activated with N-hydroxysuccinimide (NHS) and N-ethyl-N'-(3-diethylaminopropyl) carbodiimide (EDC) the antigen in acidic solution is then bound to the surface, and finally any unreacted carboxyl groups are blocked with ethanolamine.

The immobilisation of ligand is automated by the BIACore 2000 machine, and all steps are carried out in the autosampler or in the flowcell, on the dextran surface of the chip. The buffer used throughout the immobilisation procedure, and the analysis of samples is Hepes -buffered saline (HBS) with a surfactant (Pharmacia Biosensor). The chips (Pharmacia, CM5), have dextran coating on a thin layer of gold. NHS at 100mM and EDC at 400mM are mixed by the autosampler, and then a fixed volume is injected over the flowcell surface. This is followed by an injection of antigen in a suitable buffer. In the case of TGFβ, a surface of the correct density was given by using 25-30µg/ml solution of TGFβ2 (AMS) OR TGFβ3 (R & D systems) in 10mM acetate. After injection of the ligand, the chip is blocked using 1M ethanolamine. The total amount of TGFβ bound was assessed from the total increase in resonance units over this period.

To determine the kinetic parameters, a series of dilutions of the antibody samples was made in HBS from about 500µg/ml down to less than 1 µg/ml, usually through doubling dilutions. After the antibody has been injected over the antigen surface, the surface is washed with HBS, then regenerated by stripping off the bound antibody with a pulse of 100mM HCl. At the higher concentrations of antibody the antigen on the chip surface is saturated, and the off rate is determined on washing with buffer in the dissociation phase. For determination of the on-rate, lower concentrations of antibody are used, giving a linear binding phase in the sensorgram, allowing kₒₙ determination.

The set of dilutions were repeated on a separate preparation of the same antibody.

To manipulate the sensorgrams to obtain kinetic constants kₒₙ and k_{off}, the BIAevaluation software package is used. For each binding curve used in the calculations, care was taken that the conditions were appropriate for the determination of kinetic constants.

6B1 IgG4 was purified from the GS/NS0 cell line of Example 10 as in Example 2. 6B1 single chain Fv was expressed intracellularly in *E. coli,* refolded *in vitro* (using the methodology of WO94/18227), and purified to give a homogeneous product. The values of kₒₙ and k_{off} were determined for 6B1 IgG4 for binding to both TGFβ2 and TGFβ3, and for the single-chain Fv 6B1 for binding to TGFβ2. The dissociation constant was calculated by dividing k_{off} by kₒₙ. The values for these kinetic parameters are shown in Table 7.

Thus, 6B1 scFv and 6B1 IgG4 show very low dissociation constants of 2.3nM and 0.89nM respectively for TGFβ2, and there is 9% cross-reactivity with TGFβ3 (as judged by the ratio of dissociation constants of 6B1 IgG4 for TGFβ3 and TGFβ2). For comparison, in earlier studies, where the standard errors were greater and the values less precise, the Kd values for TGFβ2 were determined to be 0.7nM for 6A5 scFv (Table 2) and 2nM for 6H1 IgG4 (Example 2). The Kd values for all the antibodies directed against TGFβ2 which share the same 6H1 VH domain are low and below 10nM.

### EXAMPLE 14

### Binding of a Peptide Corresponding to Residues 56 to 69 of TGFβ2 to 6B1 IgG4

A peptide was synthesised corresponding to the amino acids of TGFβ2 surrounding the residues RVLSL, the epitope identified from the selection of phage from the peptide display library (Example 11).

The 17-mer peptide CGG-TQHSRVLSLYNTIN (TGFβ2₅₆₋₆₉; synthesised by Cambridge Research Biochemicals) contains residues 56 to 69 of TGFβ2 with RVLSL (residues 60 to 64) at its centre. The CGG N-terminal extension is a spacer with a cysteine residue to facilitate coupling of the peptide to carrier proteins. The peptide corresponding to residues 56 to 69 from TGFβ1 (TGFβ1₅₆₋₆₉; CGG-TQYSKVLSLYNQHN) was also synthesised. As a control, irrelevant peptide GPEASRPPKLHPG was used.

Two approaches were used to confirm that the epitope on TGFβ2 for 6B1 IgG4 comprised the amino acids RVLSL.
(i) Assessment of the ability of 6B1 IgG4 to bind to TGFβ2₅₆₋₆₉ and TGFβ1₅₆₋₆₉ coupled to BSA by ELISA
(ii) Assessment of the ability of peptides to bind to 6B1 IgG4 coated onto a BIACore sensor chip.

### (i) Assessment of the ability of 6B1 IgG4 to bind to TGFβ2₅₆₋₆₉ and TGFβ1₅₆₋₆₉ coupled to BSA by ELISA

The binding of 6B1 IgG4 to synthetic peptides TGFβ1₅₆₋₆₉ and TGFβ2₅₆₋₆₉ conjugated to BSA was assessed in an ELISA assay. This was compared with the binding of a control antibody 2G6 IgG4 which is an engineered antibody with a heavy chain containing a VH from an antibody directed against the hapten NIP combined with a light chain containing a VL from an antibody directed against lysozyme.

### Method

Two mg of each of the peptides TGFβ1₅₆₋₆₉ and TGFβ2₅₆₋₆₉ were conjugated to BSA using an Imject Activated Immunogen Conjugation kit (Pierce).

An immunosorp microtitre plate (Nunc) was coated overnight with 10ug/ml of the conjugated peptides in PBS (rows A-D TGFβ1₅₆₋₆₉, rows E-F TGFβ2₅₆₋₆₉) at 100µl/well. The wells were washed 3x with PBS-tween and the following additions made: Column 1 -100µl PBS in each well as reagent control; Column 2, rows A,B,E and F 200µl of 6B1 IgG4 10µg/ml; Column 2, rows C,D,G and H 200µl of 2G6 IgG4 10µg/ml.

100µl of PBS was put into all the remaining wells. To produce doubling dilutions of the antibodies, 100µl was removed from each well in column 2 and placed into the next well in column 3. The sample was mixed and 100µl removed and added to the next well in column 4. This procedure was repeated along the plate with the last 100µl being discarded. The plate was then incubated at 4°C for 18hr.

After 3x washes with PBS-tween the wells were refilled with 100ul of an alkaline phosphatase conjugate of goat F(ab')₂ fragment specific for the human IgG gamma chain diluted 1:1000 in PBS and incubated for a further lhr. After 3x further washes with PBS-tween bound antibody was revealed with p-NPP substrate for 20min.

### Results

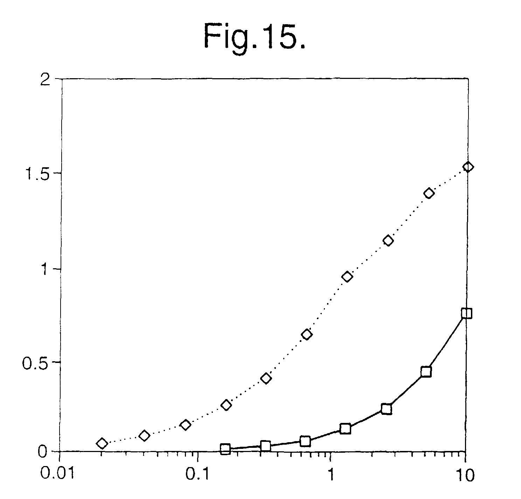

6B1 IgG4 was shown to bind to both conjugated peptides (Figure 15) but the ELISA signal obtained with TGFβ1₅₆₋₆₉ was much lower than that obtained with TGFβ2₅₆₋₆₉ at an equivalent concentration of 6B1 IgG4. An approximately 8 to 10 times higher concentration of 6B1 IgG4 was required to obtain an equivalent signal with TGFβ1₅₆₋₆₉ compared with TGFβ2₅₆₋₆₉. No signal was obtained with the control 2G6 IgG4 antibody with either peptide-BSA conjugate. 6B1 IgG4 therefore strongly binds TGFb256-69 and more weakly binds TGFβ1₅₆₋₆₉ coupled to BSA.

### (ii) Assessment of the ability of peptides to bind to 6B1 IgG4 coated onto a BIACore sensor chip.

The binding of 6B1 IgG4 to TGFβ2₅₆₋₆₉ was confirmed by binding the peptide to 6B1 IgG4 coated on to a BIACore sensor chip. The determination of binding properties by surface plasmon resonance using the Pharmacia BIACore 2000 was described in Example 13. The method of creating a BIACore sensor chip coated with 6B1 IgG4 was as for the method for coupling with TGFβ, described in Example 13, except that 5B1 IgG4 was coupled at 5µg/ml in 10mM acetate buffer, pH3.5. A surface of 5000RU was generated using 25µl of 6B1 IgG4.

Twenty µl of the the peptides were applied to the 6B1 surface at 1mg/ml with regeneration of the surface using an acid pulse to remove bound peptide between samples. The amount of binding was assessed by setting a baseline response of absolute RU prior to injection, and then subtracting this from the value at 20 seconds after the injection was complete to give a relative response in RU. This is taken to be the amount of binding to the 6B1 surface.

The binding obtained is shown in Table 9. There was a very low level of binding of the irrelevant peptide. TGFβ1₅₆₋₆₉ appeared to bind specifically at a low level to 6B1 IgG4. However, the TGFβ2₅₆₋₆₉ peptide bound to 6B1 IgG4 specifically and very much more strongly.

The low level of binding of 6B1 IgG4 to the TGFβ1 peptide in the ELISA and BIACore assays is not unexpected given that 10 of the 14 TGFβ amino acids are identical with the TGFβ2 peptide. Nevertheless, 6B1 IgG4 binds the TGFβ2₅₆₋₆₉ peptide very much more strongly than it binds the TGFβ1₅₆₋₆₉ peptide. The level of discrimination between these TGFβ1 and TGFβ2 peptides is very much lower however than is seen for the radioreceptor (Table 6) and neutralisation assays (Table 6 and Figures 16 and 17) with native isoforms. In these assays, 6B1 IgG4 strongly neutralises TGFβ2 but has little effect on TGFβ1 biological activity. This greater discrimination presumably reflects the context of the residues of the peptides in the native isoforms.

### Conclusions

These results support the assignment of the epitope of 6B1 IgG4 on TGFβ2 to the aminoacids in the region of residues 60 to 64. The peptide used in this example, residues 56 to 69, corresponds to the amino acids of alpha helix H3 (M.P. Schlunegger & M.G. Grutter Nature 358 430-434, 1992). TGFβ2 forms a head-to-tail dimer with the alpha helix H3 (also referred to as the heel) of one subunit forming an interface with finger regions (including residues 24 to 37 and residues in the region of amino acids 91 to 95; also referred to as fingers 1 and 2) from the other subunit (S. Daopin et al Proteins: Structure, Function and Genetics 17 176-192, 1993). It has been proposed that the primary structural features which interact with the TGFβ2 receptor consist of amino acids at the C-terminal end of the alpha helix H3 from one chain together with residues of fingers 1 and 2 of the other chain (D.L. Griffith et al Proc. Natl. Acad. Sci. USA 93 878-883, 1996). The identification of an epitope for 6B1 IgG4 within the alpha helix H3 of TGFβ2 is consistent with 6B1 IgG4 preventing receptor binding and neutralising the biological activity of TGFβ2.

If the epitope for 6B1 IgG4 is three dimensional there may be other non-contiguous epitopes to which the antibody may bind.

There is earlier evidence that antibodies directed against this region of TGFβ2 may be specific for TGFβ2 and neutralise its activity. Flanders et al (Development 113 183-191 1991) showed that polyclonal antisera could be raised in rabbits against residues 50 to 75 of mature TGFβ2 and that these antibodies recognised TGFβ2 but not TGFβ1 in Western blots. In an earlier paper, K.C. Flanders et al (Biochemistry 27 739-746, 1988) showed that polyclonal antisera raised in rabbits against amino acids 50 to 75 of TGFβ1 could neutralise the biological activity of TGFβ1. The antibody we have isolated and characterised, 6B1 IgG4, is a human antibody directed against amino acids in this region which neutralises the biological activity of human TGFβ2. It is surprising that such a neutralising antibody against TGFβ2 can be isolated in humans (where immunisation with a peptide cannot be used for ethical reasons) directly from a phage display antibody repertoire.

The complete disclosure of WO97/13844, including its claims, is specifically incorporated herein.

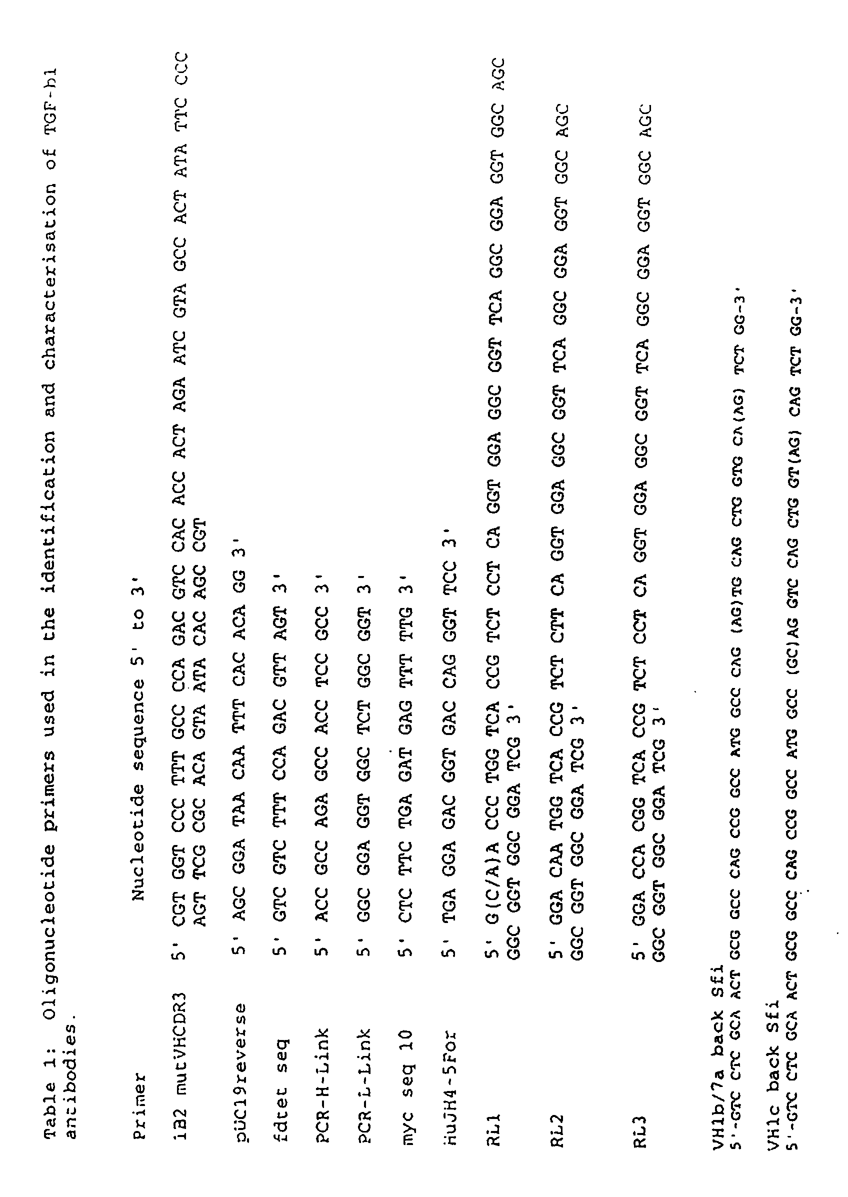

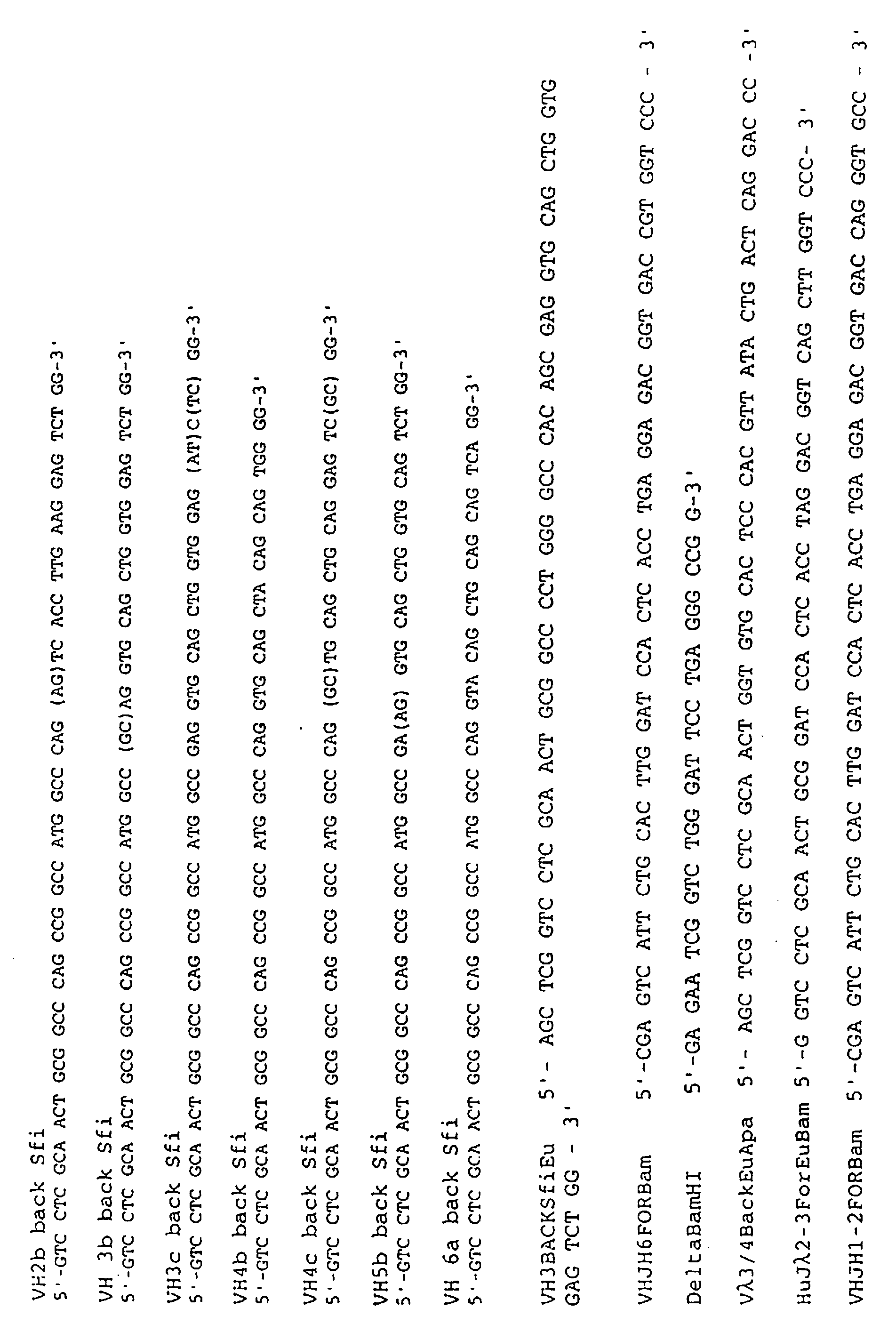

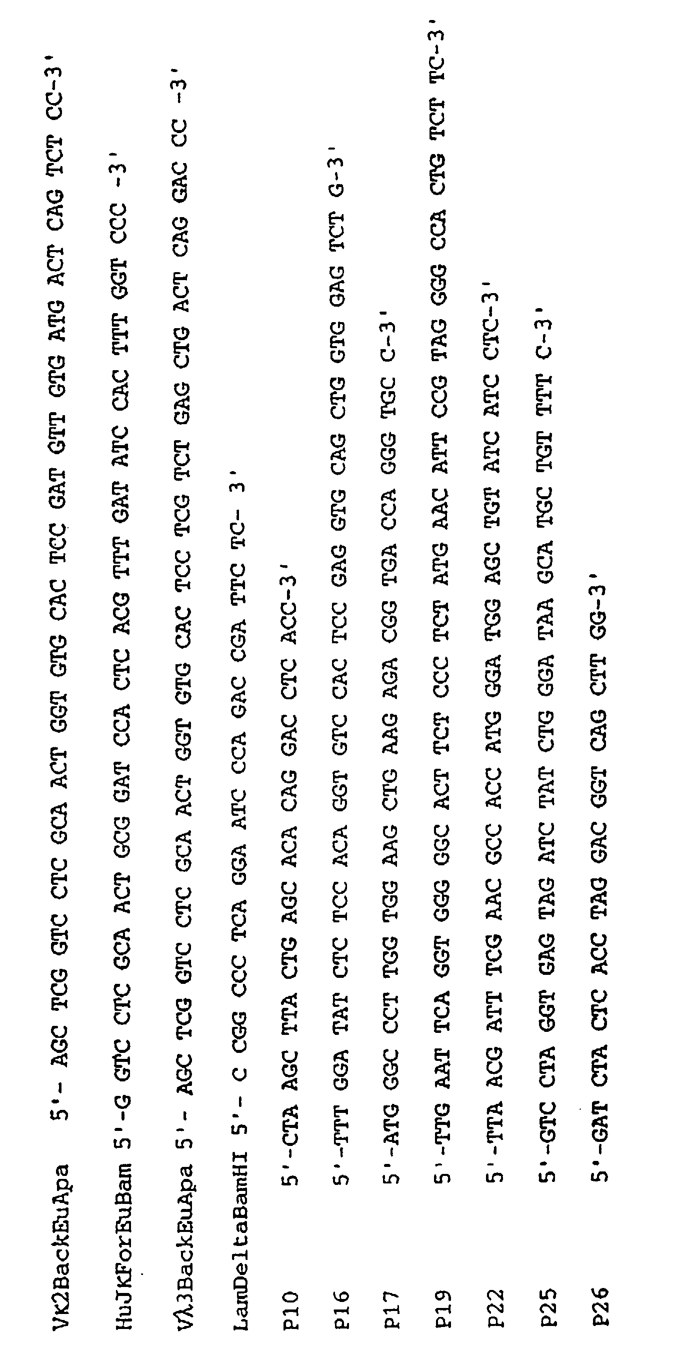

**Table 2**

| Properties of single chain Fv fragments for binding to TGFbetal or TGFbeta2 determined using BIACore | | |
|---|---|---|
| *Antibody* | *koff (s*^{*-1*}*)* | *K*_{*d*}*(nM)* |
| TGFbeta1 | | |
| 31G9 | 9.0 × 10⁻⁴ | 12 |
| CS32 | 1.2 × 10⁻³ | |
| CS39 | 1.7 × 10⁻³ | |

| TGFbeta2 | | |
|---|---|---|
| 6A5 | 1.4 × 10⁻⁴ | 0.7 |
| 6B1 | 6.0 × 10⁻⁴ | |
| 6H1 | 1.1 × 10⁻³ | |
| 14F12 | 2.1 × 10⁻³ | |

**Table 3**

| Daily dose levels for individual animals in each group | | | | |
|---|---|---|---|---|
| Group | Clone | Antibody format | Antigen | Dose |
| 1 | Saline | - | - | - |
| | Control | | | |
| 2 | 31G9 | scFv | TGF β₁ | 20ng |
| 3 | 6A5 | scFv | TGF β₂ | 20ng |
| 4 | 27C1/10A6 | IgG4 | TGF β₁ | 692ng |
| 5 | 6H1 | IgG4 | TGF β₂ | 1.76µg |
| 6 | 31G9 | scFv's | TGF β₁ | 20ng |
| | +6A5 | | TGF β₂ | " |
| 7 | 27C1/10A6 | IgG4's | TGF β₁ | 692ng |
| | +6H1 | | TGF β₂ | 1.76µg |

**Table 4**

| I.C.₅₀ values for antibodies in TF1 assay | | |
|---|---|---|
| Antibody | scFv (nM) | IgG4 (nM) |
| 6H1 | 1.5 | 100 |
| 6B1 | 15 | 11 |
| 6A5 | 8 | 150 |
| 14F12 | 90 | nd |
| nd = not determined | | |

**Table 5**

| IC₅₀ values for antibodies measured using a radioreceptor assay. | |
|---|---|
| **Anti-TGF-β**_{**1**} **antibody** | **IC**_{**50**}**, nM** |
| 7A3 scFv | >100 |
| 31G9 scFv | 30 |
| CS32 scFv | 4.5 |
| CS39 scFv | ∼60 |
| 27C1/10A6 IgG | 9 |
| VT37 scFv | ∼100 |

| **Anti-TGF-β**_{**2**} **antibody** | **IC**_{**50**}**, nM** |
|---|---|
| 6A5 scFv | 1.5 |
| 6A5 IgG | ∼6 |
| 6B1 scFv | 0.3 |
| 6B1 IgG | 0.6 |
| 6H1 scFv | 0.22 |
| 6H1 IgG | ∼10 |
| 11E6 IgG | 1.6 |
| 14F12 scFv | 3 |
| VT37 scFv | 2 |

**Table 6**

| Potency of neutralisation of TGFbeta isoforms | | |
|---|---|---|
| *TF1 cell proliferation assay IC*_{*50*} *(nM IgG)* | | |
| | 6B1 IgG4 | Genzyme |
| TGFbeta1 | >100 | 1.5 |
| TGFbeta2 | 2 | 10 |
| TGFbeta3 | 11 | 0.1 |

| *A549 cell radioreceptor assay IC*_{*50*} *(nM IgG)* | | |
|---|---|---|
| | 6B1 IgG4 | Genzyme |
| TGFbeta1 | >400 | 0.55 |
| TGFbeta2 | 0.05 | 0.5 |
| TGFbeta3 | 4 | 0.03 |

**Table 7**

| Kinetic parameters of 6B1 IgG4 and 6B1 single chain Fv | | | | |
|---|---|---|---|---|
| antibody format | antigen | k_{off} s⁻¹ | kₒₙ M⁻¹s⁻¹ | dissociation constant K_{d} nM |
| 6B1 scFv | TGFβ2 | 6.68 × 10⁻⁴ | 2.87 × 10⁵ | 2.32 |
| 6B1 IgG4 | TGFβ2 | 3.36× 10⁻⁴ | 3.84 × 10⁵ | 0.89 |
| 6B1 IgG4 | TGFβ3 | 4.5 × 10⁻⁴ | 4.5 × 10⁴ | 10.0 |

**Table 8**

| Peptide sequences from phage binding to 6B1 IgG4 | |
|---|---|
| This table shows the amino acid sequence of 4 phage peptide display clones that show a match with the sequence of TGFbeta2. These clones have been lined up below the relevant part of the sequence of TGFbeta2, which is shown from amino acid positions 56 to 77. | |
| TGFbeta2 | TQHSRVLSLYNTINPEASASPC |
| Clone 1 | RQLSLQQRMH |
| Clone 2 | DPMDMVLKLC |
| Clone 3 | WSEFMRQSSL |
| Clone 3 | VESTSLQFRG |

**Table 9**

| Binding of peptides from TGFbeta to 6B1 IgG4 immobilised on a BIACore chip | | |
|---|---|---|
| peptide | concentration of peptide, µM | amount of binding to 6B1 IgG4 surface, RU |
| TGFβ2₅₆₋₆₉ | 537 | 1012.8 |
| TGFβ1₅₆₋₆₉ | 524 | 190.7 |
| irrelevant peptide | 745 | 60.9 |

## Claims

1. A specific binding member comprising a human antibody antigen binding domain specific for human TGF-β which binds the human TGFβ isoform TGFβ1 preferentially over TGFβ3, and which neutralises TGFβ1, the human antibody antigen binding domain comprising the VH domain 31G9 VH of which the amino acid sequence is shown in Figure 1(a)(ii) and/or the VL domain CS37 VL of which the amino acid sequence is shown in Figure 14.

2. A specific binding member according to claim 1 comprising said CS37 VL domain.

3. A specific binding member according to claim 2 comprising said 31G9 VH domain.

4. A specific binding member comprising a human antibody antigen binding domain which competes for binding to TGF-β1 with a specific binding member according to claim 3, which binds TGFβ1 with a dissociation constant that is at least five fold lower than its dissociation constant for TGFβ3 and which neutralises TGFβ1.

5. A specific binding member according to claim 1 or claim 4 comprising a VL domain which is an amino acid sequence variant of the VL domain CS37 VL by way of substitution of one amino acid in the sequence shown in Figure 14.

6. A specific binding member according to claim 1 which binds the peptide TQYSKVLSLYNQHN.

7. A specific binding member according to any preceding claim comprising a single-chain Fv antibody molecule.

8. A specific binding member according to any of claims 1 to 6 which comprises one or more amino acids in addition to those forming said human antibody antigen binding domain.

9. A specific binding member according to claim 8 comprising an antibody constant region.

10. A specific binding member according to claim 9 which comprises a whole antibody.

11. A specific binding member according to claim 9 or 10 wherein said antibody constant region is IgG4 isotype.

12. A method comprising causing or allowing binding *in vitro* of a specific binding member according to any preceding claim to TGF-β1 isoform of human TGF-β.

13. A method according to claim 12 wherein said binding of the specific binding member neutralises said isoform.

14. A specific binding member according to any one of claims 1 to 11 for use in a method of treating an individual to counteract effects of TGF-β which are deleterious to the individual.

15. A specific binding member according to claim 14 wherein said effects are fibrosis promoting effects.

16. A specific binding member according to claim 15 wherein said individual has a condition selected from the group consisting of glomerulonephritis, neural scarring, dermal scarring, ocular scarring, lung fibrosis, arterial injury, proliferative retinopathy, retinal detachment, adult respiratory distress syndrome, liver cirrhosis, post myocardial infarcation, post angioplasty restenosis, keloid scarring, scleroderma, vascular disorders, cataract, and glaucoma.

17. A specific binding member according to claim 16 wherein said condition is neural scarring or glomerulonephritis.

18. A specific binding member according to claim 14 wherein said effects contribute to an immune or inflammatory disease condition.

19. A specific binding member according to claim 18 wherein said condition is selected from the group consisting of rheumatoid arthritis, macrophage deficiency disease and macrophage pathogen infection.

20. Use of a specific binding member according to any of claims 1 to 11 in the manufacture of a medicament for treating an individual to counteract effects of TGF-β which are deleterious to the individual.

21. Use according to claim 20 wherein said effects are fibrosis promoting effects.

22. Use according to claim 21 wherein said individual has a condition selected from the group consisting of glomerulonephritis, neural scarring, dermal scarring, ocular scarring, lung fibrosis, arterial injury, proliferative retinopathy, retinal detachment, adult respiratory distress syndrome, liver cirrhosis, post myocardial infarction, post angioplasty restenosis, keloid scarring, scleroderma, vascular disorders, cataract, and glaucoma.

23. Use according to claim 22 wherein said condition is neural scarring or glomerulonephritis.

24. Use according to claim 23 wherein said effects contribute to an immune or inflammatory disease condition.

25. Use according to claim 24 wherein said condition is selected from the group consisting of rheumatoid arthritis, macrophage deficiency disease and macrophage pathogen infection.

26. Nucleic acid encoding a specific binding member according to any of claims 1 to 11.

27. Nucleic acid according to claim 26 which is part of an expression vector.

28. A method which comprises use of nucleic acid according to claim 26 or claim 27 in an expression system for production of a specific binding member according to any of claims 1 to 11.

29. A host cell containing nucleic acid according to claim 26 or claim 27.

30. A host cell according to claim 29 which is capable of producing said specific binding member under appropriate culture conditions.

31. A method of producing a specific binding member according to any of claims 1 to 11 comprising culturing a host cell according to claim 30 under appropriate conditions for production of said specific binding member.

32. A method according to claim 31 wherein following said production said specific binding member is isolated from the cell culture.

33. A method according to claim 32 wherein following said isolation the specific binding member is used in formulation of a composition comprising at least one additional component.

34. A method according to claim 33 wherein said composition is a pharmaceutical composition comprising a pharmaceutically acceptable excipient.

35. A pharmaceutical composition comprising a specific binding member according to any of claims 1 to 11 and a pharmaceutically acceptable excipient.

36. A method for obtaining an antibody antigen binding domain with the properties of being specific for human TGF-β, binding the human TGF-β isoform TGF-β1 preferentially over TGF-β3, and neutralising TGF-β1, the method comprising providing by way of addition , deletion, substitution or insertion of one or more amino acids in the amino acid sequence shown in Figure 1 (a) (ii) a VH domain which is an amino acid sequence variant of the VH domain 31G9 VH, and/or providing by way of addition, deletion, substitution or insertion of one or more amino acids in the amino acid sequence shown in Figure 14 a VL domain which is an amino sequence variant of the VL domain CS37 VL, combining the VH domain and/or VL domains thus provided with one or more VL or VH domains respectively to provide one or more VH/VLcombinations, and testing the VH/VL combination or combinations for said properties to identify an antibody antigen binding domain with said properties.

37. A method according to claim 36 wherein said antibody antigen binding domain identified as having said properties is produced as an isolated single-chain Fv antibody molecule.

38. A method according to claim 36 wherein said antibody antigen binding domain identified as having said properties is produced in a polypeptide comprising one or more amino acids in addition to those forming the antibody antigen binding domain.

39. A method according to claim 38 wherein said polypeptide comprises an antibody constant region.

40. A method according to claim 39 wherein said polypeptide comprises a whole antibody.

41. A method according to claim 39 or 40 wherein said antibody constant region is IgG4 isotype.

42. A method according any one of claims 37 to 41 wherein said antibody antigen binding domain identified as having said properties is produced by means of expression from encoding nucleic acid.

43. A method according to any one of claims 37 to 42 wherein said antibody antigen binding domain identified as having said properties is formulated in a composition comprising at least one additional component.

44. A method according to claim 43 wherein said composition comprises a pharmaceutically acceptable excipient.

## Patentansprüche

1. Spezifisches Bindeelement, umfassend eine für Human-TGFβ spezifische Human-Antikörper-Antigen-Bindungsdomäne, welche die Isoform TGFβ1 von Human-TGFβ gegenüber der Isoform TGFβ3 bevorzugt bindet und TGFβ1 neutralisiert, wobei die Human-Antikörper-Antigen-Bindungsdomäne die VH-Domäne 31G9 VH, deren Aminosäuresequenz in Fig. 1(A) (ii) dargestellt ist, und/oder die VL-Domäne CS37 VL, deren Aminosäuresequenz in Fig. 14 dargestellt ist, umfasst.

2. Spezifisches Bindeelement nach Anspruch 1, umfassend die Domäne CS37 VL.

3. Spezifisches Bindeelement nach Anspruch 2, umfassend die Domäne 31G9 VH.

4. Spezifisches Bindeelement, das eine Human-Antikörper-Antigen-Bindungsdomäne, die mit einem spezifischen Bindeelement nach Anspruch 3 um Bindung an TGFβ1 konkurriert, umfasst und TGFβ1 mit einer gegenüber seiner Dissoziationskonstante für TGFβ3 um einen Faktor 5 niedrigeren Dissoziationskonstante bindet und TGFβ1 neutralisiert.

5. Spezifisches Bindeelement nach Anspruch 1 oder 4, umfassend eine VL-Domäne, die eine durch Substitution einer Aminosäure in der in Fig. 14 dargestellten Sequenz erzeugte Aminosäuresequenz-Variante der VL-Domäne CS37 VL ist.

6. Spezifisches Bindeelement nach Anspruch 1, welches das Peptid TQYSKVLSLYNQHN bindet.

7. Spezifisches Bindeelement nach einem der vorangegangenen Ansprüche, umfassend ein einkettiges Fv-Antikörpermolekül.

8. Spezifisches Bindeelement nach einem der Ansprüche 1 bis 6, das außer den die Human-Antikörper-Antigen-Bindungsdomäne bildenden Aminosäuren eine oder mehrere weitere Aminosäuren umfasst.

9. Spezifisches Bindeelement nach Anspruch 8, umfassend eine konstante Antikörper-Region.

10. Spezifisches Bindeelement nach Anspruch 9, das einen vollständigen Antikörper umfasst.

11. Spezifisches Bindeelement nach Anspruch 9 oder 10, worin die konstante Antikörper-Region der Isotyp IgG4 ist.

12. Verfahren, umfassend das Hervorrufen oder Ermöglichen der in vitro-Bindung eines spezifischen Bindeelements nach einem der vorangegangenen Ansprüche an die Isoform TGFβ1 von Human-TGFβ.

13. Verfahren nach Anspruch 12, worin die Bindung des spezifischen Bindeelements die Isoform neutralisiert.

14. Spezifisches Bindeelement nach einem der Ansprüche 1 bis 11 zur Verwendung in einem Verfahren zur Behandlung eines Patienten, um für den Patienten schädlichen Effekten von TGFβ entgegenzuwirken.

15. Spezifisches Bindeelement nach Anspruch 14, worin die Effekte Fibrose fördernde Effekte sind.

16. Spezifisches Bindeelement nach Anspruch 15, worin der Patient ein aus der aus Glomerulonephritis, Neuronenvernarbung, Hautvernarbung, Augenvernarbung, Lungenfibrose, Arterienschädigung, proliferativer Retinopathie, Netzhautablösung, Respiratory-Distress-Syndrom bei Erwachsenen, Leberzirrhose, Postmyokardinfarkt, Postangioplastierestenose, Keloidvernarbung, Sklerodermie, Gefäßstörungen, Katarakt und Glaukom bestehenden Gruppe ausgewähltes Leiden aufweist.

17. Spezifisches Bindeelement nach Anspruch 16, worin das Leiden Neuronenvernarbung oder Glomerulonephritis ist.

18. Spezifisches Bindeelement nach Anspruch 14, worin die Effekte zu einer Immun- oder Entzündungserkrankung beitragen.

19. Spezifisches Bindeelement nach Anspruch 18, worin die Erkrankung aus der aus Arthritis rheumatica, Makrophagenmangelerscheinungen und pathogenen Makrophageninfektionen bestehenden Gruppe ausgewählt ist.

20. Verwendung eines spezifischen Bindeelements nach einem der Ansprüche 1 bis 11 zur Herstellung eines Medikaments zur Behandlung eines Patienten, um für den Patienten schädlichen Effekten von TGFβ entgegenzuwirken.

21. Verwendung nach Anspruch 20, worin die Effekte Fibrose fördernde Effekte sind.

22. Verwendung nach Anspruch 21, worin der Patient ein aus der aus Glomerulonephritis, Neuronenvernarbung, Hautvernarbung, Augenvernarbung, Lungenfibrose, Arterienschädigung, proliferativer Retinopathie, Netzhautablösung, Respiratory-Distress-Syndrom bei Erwachsenen, Leberzirrhose, Postmyokardinfarkt, Postangioplastierestenose, Keloidvernarbung, Sklerodermie, Gefäßstörungen, Katarakt und Glaukom bestehenden Gruppe ausgewähltes Leiden aufweist.

23. Verwendung nach Anspruch 22, worin das Leiden Neuronenvernarbung oder Glomerulonephritis ist.

24. Verwendung nach Anspruch 23, worin die Effekte zu einer Immun- oder Entzündungserkrankung beitragen.

25. Verwendung nach Anspruch 24, worin die Erkrankung aus der aus Arthritis rheumatica, Makrophagenmangelerscheinungen und pathogenen Makrophageninfektionen bestehenden Gruppe ausgewählt ist.

26. Für ein spezifisches Bindeelement nach einem der Ansprüche 1 bis 11 kodierende Nukleinsäure.

27. Nukleinsäure nach Anspruch 26, die Teil eines Expressionsvektors ist.

28. Verfahren, umfassend die Verwendung von Nukleinsäure nach Anspruch 26 oder 27 in einem Expressionssystem zur Erzeugung eines spezifischen Bindeelements nach einem der Ansprüche 1 bis 11.

29. Wirtszelle, die Nukleinsäure nach Anspruch 26 oder 27 enthält.

30. Wirtszelle nach Anspruch 29, die in der Lage ist, unter geeigneten Kulturbedingungen das spezifische Bindeelement zu produzieren.

31. Verfahren zur Herstellung eines spezifischen Bindeelements nach einem der Ansprüche 1 bis 11, umfassend das Kultivieren einer Wirtszelle nach Anspruch 30 unter geeigneten Bedingungen, um das spezifische Bindeelement zu produzieren.

32. Verfahren nach Anspruch 31, worin das spezifische Bindeelement nach der Produktion aus der Zellkutur isoliert wird.

33. Verfahren nach Anspruch 32, worin das spezifische Bindeelement nach der Isolierung zur Formulierung einer Zusammensetzung verwendet wird, die zumindest eine weitere Komponente umfasst.

34. Verfahren nach Anspruch 33, worin die Zusammensetzung eine pharmazeutische Zusammensetzung ist, die einen pharmazeutisch annehmbaren Exzipienten umfasst.

35. Pharmazeutische Zusammensetzung, umfassend ein spezifisches Bindeelement nach einem der Ansprüche 1 bis 11 und einen pharmazeutisch annehmbaren Exzipienten.

36. Verfahren zum Erhalten einer Antikörper-Antigen-Bindungsdomäne mit den folgenden Eigenschaften: Spezifität für Human-TGFβ, bevorzugte Bindung der Isoform TGFβ1 von Human-TGFβ gegenüber TGFβ3 sowie Neutralisierung von TGFβ1, wobei das Verfahren Folgendes umfasst: Bereitstellung einer VH-Domäne, die eine Aminosäuresequenz-Variante der VH-Domäne 31G9 VH ist, durch Addition, Deletion, Substitution oder Insertion einer oder mehrerer Aminosäuren in der in Fig. 1(a) (ii) dargestellten Aminosäuresequenz und/oder Bereitstellung einer VL-Domäne, die eine Aminosäuresequenz-Variante der VL-Domäne CS37 VL ist, durch Addition, Deletion, Substitution oder Insertion einer oder mehrerer Aminosäuren in der in Fig. 14 dargestellten Aminosäuresequenz, Kombination der so bereitgestellten VH-Domäne und/oder VL-Domäne mit einer oder mehreren VL- bzw. VH-Domänen, um eine oder mehrere VH/VL-Kombinationen bereitzustellen, sowie Testen der VH/VL-Kombination(en) auf die genannten Eigenschaften, um eine Antikörper-Antigen-Bindungsdomäne mit diesen Eigenschaften zu identifizieren.

37. Verfahren nach Anspruch 36, worin die mit diesen Eigenschaften identifizierte Antikörper-Antigen-Bindungsdomäne als isoliertes einkettiges Fv-Antikörpermolekül erzeugt wird.

38. Verfahren nach Anspruch 36, worin die mit diesen Eigenschaften identifizierte Antikörper-Antigen-Bindungsdomäne in einem Polypeptid erzeugt wird, das außer den die Human-Antikörper-Antigen-Bindungsdomäne bildenden Aminosäuren eine oder mehrere weitere Aminosäuren umfasst.

39. Verfahren nach Anspruch 38, worin das Polypeptid eine konstante Antikörper-Region umfasst.

40. Verfahren nach Anspruch 39, worin das Polypeptid einen vollständigen Antikörper umfasst.

41. Verfahren nach Anspruch 39 oder 40, worin die konstante Antikörper-Region der Isotyp IgG4 ist.

42. Verfahren nach einem der Ansprüche 37 bis 41, worin die mit diesen Eigenschaften identifizierte Antikörper-Antigen-Bindungsdomäne mittels Expression aus dafür kodierender Nukleinsäure produziert wird.

43. Verfahren nach einem der Ansprüche 37 bis 42, worin die mit diesen Eigenschaften identifizierte Antikörper-Antigen-Bindungsdomäne zu einer Zusammensetzung formuliert wird, die zumindest eine weitere Komponente umfasst.

44. Verfahren nach Anspruch 43, worin die Zusammensetzung einen pharmazeutisch annehmbaren Exzipienten umfasst.

## Revendications

1. Un membre de liaison spécifique comprenant un domaine de liaison antigène anticorps humain spécifique du TGF-β humain qui se lie au TGF β1 isoforme du TGFβ humain de préférence à TGFβ3, et qui neutralise le TGFβ1, le domaine de liaison antigène anticorps humain comprenant le domaine 31G9 VH dont la séquence d'acides aminés est montrée en Figure 1 (a) (ii) et/ou le domaine CS37 VL dont la séquence d'acides aminés est montrée en Figure 14.

2. Un membre spécifique de liaison selon la revendication 1 comprenant ledit domaine CS37 VL.

3. Un membre spécifique de liaison selon la revendication 2 comprenant ledit domaine 31G9 VH.

4. Un membre spécifique de liaison comprenant un domaine de liaison antigène anticorps humain qui est en compétition pour lier au TGF-β1 avec un membre spécifique de liaison selon la revendication 3, qui lie le TGFβ1 avec une constante de dissociation qui est au moins cinq fois moindre que sa constante de dissociation pour TGFβ3 et qui neutralise TGFβ1.

5. Un membre spécifique de liaison selon la revendication 1 ou la revendication 4 comprenant un domaine VL qui est une variante d'une séquence d'acide aminé du domaine CS37 VL par le moyen de la substitution d'un acide aminé de la séquence montrée en Figure 14.

6. Un membre spécifique de liaison selon la revendication 1 qui lie le peptide TQYSKVLSLYNQHN.

7. Un membre spécifique de liaison selon l'une quelconque des revendications précédentes comprenant une molécule d'anticorps à chaîne simple Fv.

8. Un membre spécifique de liaison selon l'une quelconque des revendications 1 à 6 comprenant un ou plusieurs acides aminés en addition de ceux formant ledit domaine de liaison antigène anticorps humain.

9. Un membre spécifique de liaison selon la revendication 8 comprenant une région à anticorps constante.

10. Un membre spécifique de liaison selon la revendication 9 qui comprend un anticorps complet.

11. Un membre spécifique de liaison selon la revendication 9 ou 10 où ladite région à anticorps constante est isotype de IgG4.

12. Une méthode comprenant la provocation ou permettant la liaison in vitro d'un membre spécifique de liaison selon l'une quelconque des revendications précédentes à l'isoforme TGF-β1 du TGF-β humain.

13. Une méthode selon la revendication 12 où ladite liaison du membre spécifique de liaison neutralise ladite isoforme.

14. Un membre spécifique de liaison selon l'une quelconque des revendications 1 à 11 pour utilisation dans une méthode de traitement d'un individu pour s'opposer aux effets du TGF-β qui sont délétères sur les individus.

15. Un membre spécifique de liaison selon la revendication 14 où lesdits effets sont des effets promoteurs de fibrose.

16. Un membre spécifique de liaison selon la revendication 15 où ledit individu a une pathologie sélectionnée dans le groupe constitué par la glomérulonéphrite, la cicatrisation neutre, la cicatrisation dermique, la cicatrisation oculaire, la fibrose pulmonaire, les lésions artérielles, la rétinopathie proliférante, le décollement de la rétine, le syndrome de souffrance respiratoire de l'adulte, la cirrhose du foie, le post-infarctus du myocarde, la post-resténose d'angioplastie, la cicatrisation chéloïde, la sclérodermie, les troubles vasculaires, la cataracte, et le glaucome.

17. Un membre spécifique de liaison selon la revendication 16 où ladite pathologie est la cicatrisation neutre ou la glomérulonéphrite.

18. Un membre spécifique de liaison selon la revendication 14 où lesdits effets contribuent à une pathologie de maladie immunitaire ou inflammatoire.

19. Un membre spécifique de liaison selon la revendication 18 où ladite pathologie est sélectionnée dans le groupe constitué par l'arthrite rhumatoide, la maladie de déficience des macrophages et l'infection pathogène des macrophages.

20. Utilisation d'un membre spécifique de liaison selon l'une quelconque des revendications 1 à 11 pour la fabrication d'un médicament pour traiter un individu pour s'opposer aux effets du TGF-β qui sont délétères sur les individus.

21. Utilisation selon la revendication 20 où lesdits effets sont des effets promoteurs de fibrose.

22. Utilisation selon la revendication 21 où lesdits individus ont une pathologie sélectionnée dans le groupe constitué par la glomérulonéphrite, la cicatrisation neutre, la cicatrisation dermique, la cicatrisation oculaire, la fibrose pulmonaire, les lésions artérielles, la rétinopathie proliférante, le décollement de la rétine, le syndrome de souffrance respiratoire de l'adulte, la cirrhose du foie, le post-infarctus du myocarde, la post-resténose d'angioplastie, la cicatrisation chéloide, la sclérodermie, les troubles vasculaires, la cataracte, et le glaucome.

23. Utilisation selon la revendication 22 où ladite pathologie est la cicatrisation neutre ou la glomérulonéphrite.

24. Utilisation selon la revendication 23 où lesdits effets contribuent à une pathologie de maladie immunitaire ou inflammatoire.

25. Utilisation selon la revendication 24 où ladite pathologie est sélectionnée dans le groupe constitué par l'arthrite rhumatoïde, la maladie de déficience des macrophages et l'infection pathogène des macrophages.

26. Un acide nucléique encodant un membre de liaison spécifique selon l'une quelconque des revendications 1 à 11.

27. Un acide nucléique selon la revendication 26 qui fait partie d'un vecteur d'expression.

28. Une méthode qui comprend l'utilisation d'un acide nucléique selon la revendication 26 ou la revendication 27 dans un système d'expression pour la production d'un membre de liaison spécifique selon l'une quelconque des revendications 1à 11.

29. Une cellule hôte contenant un acide nucléique selon la revendication 26 ou la revendication 27.

30. Une cellule hôte selon la revendication 29 qui est capable de produire ledit membre de liaison spécifique dans des conditions de culture appropriées.

31. Une méthode pour produire un membre de liaison spécifique selon l'une quelconque des revendications 1 à 11 comprenant la culture d'une cellule hôte selon la revendication 30 dans des conditions appropriées pour la production dudit membre de liaison spécifique.

32. Une méthode selon la revendication 31 où en suivant ladite production, ledit membre de liaison est isolé de la culture cellulaire.

33. Une méthode selon la revendication 32 où en suivant ledit isolement, le membre spécifique de liaison est utilisé en formulation d'une composition comprenant au moins un composant additionnel.

34. Une méthode selon la revendication 33 où ladite composition est une composition pharmaceutique comprenant un excipient pharmaceutiquement acceptable.

35. Une composition pharmaceutique comprenant un membre de liaison spécifique selon l'une quelconque des revendications 1 à 11 et un excipient pharmaceutiquement acceptable.

36. Une méthode pour obtenir un domaine de liaison antigène anticorps avec les propriétés d'être spécifique du TGF-β humain, de se lier au TGF β1 isoforme du TGFβ de préférence à TGF-β3, et qui neutralise le TGFβ1, la méthode comprenant l'apport par le moyen de l'addition, de la suppression, de la substitution ou de l'insertion d'un ou de plusieurs acides aminés dans la séquence d'acides aminés montrée en Figure 1 (a) (ii) d'un domaine VH qui est une variante de la séquence d'acides aminés du domaine 31G9 VH, et/ou comprenant l'apport par le moyen de l'addition, de la suppression, de la substitution ou de l'insertion d'un ou de plusieurs acides aminés dans la séquence d'acides aminés montrée en Figure 14 d'un domaine VL qui est une variante de la séquence d'acides aminés du domaine CS37 VL, combinant le domaine VH et/ou le domaine VL , ainsi fourni avec un ou plusieurs domaines VL ou VH respectivement pour fournir une ou plusieurs combinaisons VL/VH, et tester la combinaison ou les combinaisons VL/VH pour lesdites propriétés pour identifier un domaine de liaison antigène anticorps avec lesdites propriétés.

37. Une méthode selon la revendication 36 où ledit domaine de liaison antigène anticorps identifié comme ayant lesdites propriétés est produit sous la forme d'une molécule d'anticorps à chaîne simple Fv isolée.

38. Une méthode selon la revendication 36 où ledit domaine de liaison antigène anticorps identifié comme ayant lesdites propriétés est produit dans un polypeptide comprenant un ou plusieurs acides aminés en addition de ceux formant le domaine de liaison antigène anticorps.

39. Une méthode selon la revendication 38 où ledit polypeptide comprend une région à anticorps constante.

40. Une méthode selon la revendication 39 où ledit polypeptide comprend un anticorps complet.

41. Une méthode selon la revendication 39 ou 40 où ladite région à anticorps constante est isotype d'IgG4.

42. Une méthode selon l'une quelconque des revendications 37 à 41 où ledit domaine de liaison antigène anticorps identifié comme ayant lesdites propriétés est produit par le moyen de l'expression d'un acide nucléique encodant.

43. Une méthode selon l'une quelconque des revendications 37 à 42 où ledit domaine de liaison antigène anticorps identifié comme ayant lesdites propriétés est formulé dans une composition comprenant au moins un composant additionnel.

44. Une méthode selon la revendication 43 où ladite composition comprend un excipient pharmaceutiquement acceptable.
